# EUROPEAN PATENT SPECIFICATION

(11) **EP 2 539 470 B1**
(45) Date of publication and mention of the grant of the patent: **22.02.2017**
(21) Application number: 11748087.1
(22) Date of filing: 24.02.2011
(51) Int. Cl.: C12Q 1/68, G01N 33/53

(54) **METHODS FOR AUTOIMMUNE DISEASE DIAGNOSIS, PROGNOSIS, AND TREATMENT**
DIAGNOSE-, PROGNOSE- UND BEHANDLUNGSVERFAHREN FÜR AUTOIMMUNERKRANKUNGEN
PROCÉDÉS POUR LE DIAGNOSTIC, LE PRONOSTIC ET LE TRAITEMENT DE MALADIES AUTO-IMMUNES

(30) Priority: 24.02.2010 US 307829 P
(43) Date of publication of application: 02.01.2013
(73) Proprietor: The Board of Trustees of the Leland Stanford Junior University, Palo Alto, CA 94306 (US)
(72) Inventor: HALE, Matthew, B., Stanford, CA 94305 (US); PTACEK, Jason, Stanford, CA 94305 (US); NOLAN, Garry, P., Stanford, CA 94305 (US)
(74) Representative: Harrison, Susan Joan
(86) International application number: PCT/US2011/026117
(87) International publication number: WO 2011/106558

(56) References cited:
- WO-A1-2010/135608
- WO-A1-2011/031803
- WO-A2-2009/134944
- US-A1- 2008 182 262
- US-A1- 2008 182 262
- US-A1- 2008 274 118
- US-A1- 2009 148 462
- US-A1- 2010 009 364

## Description

### BACKGROUND OF THE INVENTION

Autoimmune diseases result from the recognition of an organism's own tissues or organs as foreign antigens, which are subsequently attacked by the immune system. This class of disorders is highly varied, both between and within different kinds of autoimmune diseases, which complicates diagnosis and effective treatment. The causes of autoimmune diseases are also poorly understood, which results in courses of treatment that focus primarily on the symptoms. Further complicating this reactionary approach to treatment are diagnostic measures that are either highly subjective or generally poor correlatives of disease activity, and which currently fail to efficiently stratify patient sub-populations by likelihood of drug responsiveness.

For example, Systemic Lupus Erythematosus (SLE) is a debilitating autoimmune disease that can damage multiple organs, induce chronic renal failure, and lead to severe morbidity and mortality. A characteristic feature of SLE is the presence of anti-nuclear autoantibodies that form immune complexes with cellular debris and cause end-organ damage. Current treatment regimens are limited to non-specific immune suppression and management of inflammatory symptoms. Complicating treatment is the fact that SLE disease activity can be highly variable, with periods of increased disease activity (flare) and periods of temporary remission common and lasting weeks, months, or even years. Many of the therapeutic options available have life-threatening off-target effects and should only be used when absolutely necessary and only at the minimally effective dose. Unfortunately, as with other autoimmune diseases, existing methods for determining disease activity are highly subjective assessment of gross symptoms which are representative of cumulative damage and thus reflect past immune activity rather than providing a direct measurement of current immune activity. As a result, such methods are poor predictors of changes in disease state, which makes it difficult to effectively tailor a patient's therapeutic regimen, especially as regards avoidance of future flares.

Rheumatoid arthritis (RA) is a chronic, inflammatory autoimmune disease that causes the immune system to attack the joints. It is a disabling and painful inflammatory condition, which can lead to substantial loss of mobility due to pain and joint destruction. The disease is also systemic in that it often also affects many extra-articular tissues throughout the body including the skin, blood vessels, heart, lungs, and muscles. Rheumatoid arthritis can be difficult to diagnose. Symptoms differ from person to person and can be more severe in some people than in others. Within the same person, the full range of symptoms may develop over time, and only a few symptoms may be present in the early stages. Also, symptoms can be similar to those of other types of arthritis and joint conditions, and it may take some time for other conditions to be ruled out. Additionally, there is currently no single reliable test for the disease. One common test used to help diagnose RA is for rheumatoid factor, an antibody that is present eventually in the blood of most people with the disease. Not all people with RA test positive for rheumatoid factor, however, especially early in the disease. Also, some people test positive for rheumatoid factor, yet never develop the disease. Treatment options currently available include disease-modifying anti-rheumatic drugs, B-cell depleting antibodies, cytokine depleting biologics, and disruptors of costimulatory signals. However, each treatment proves efficacious in only a subset of patients, are there is presently no efficient means of predicting which drugs will be most effective for which patients. Trial and error to identify appropriate therapeutic regimens can be costly, both physically and financially.

WO2009/134944 discloses cell-based methods of determining the health status of individuals.

Thus, there is a need for improved measures for the diagnosis, prognosis, treatment, management, and therapeutic development for autoimmune disorders.

### SUMMARY OF THE INVENTION

The present invention is defined by the appended claims. Any subject-matter identified in the application as "invention", "embodiment", "aspect", etc., which exceeds the scope of the invention as represented by the claims does not form part the claimed invention but only serves as background information to better understand the invention.

In one aspect, the invention provides a method of classification, diagnosis, prognosis, theranosis, and/or prediction of an outcome of an autoimmune disease in a subject. In some embodiments, the method comprises: (a) exposing a cell from the subject to at least one modulator; (b) determining an activation level of at least one activatable element; and, (c) classifying, diagnosing, prognosing, theranosing, and/or predicting an outcome of an autoimmune disease in the subject based on the activation level of the at least one activatable element.

In some embodiments, the method comprises: (a) exposing a cell from the subject to at least one therapeutic agent, wherein the therapeutic agent is used to treat an immune system disorder; (b) determining the activation level of at least one activatable element; and, (c) classifying, diagnosing, prognosing, theranosing, and/or predicting an outcome of the autoimmune disease in the subject based on the activation level of the at least one activatable element.

In a further aspect, the invention provides a method of identifying therapeutic targets for the treatment of autoimmune disease in a subject. In some embodiments, the method comprises: (a) exposing a cell from the subject to at least one modulator; (b) determining an activation level of at least one activatable element; and, (c) identifying one or more therapeutic targets for the treatment of the autoimmune disease in the subject based on the activation level of the at least one activatable element.

Each of the above aspects can have a number of further embodiments. In some embodiments, the autoimmune disease is rheumatoid arthritis or systemic lupus erythematosus. In some embodiments, the cell is a hematopoietic cell. In some embodiments, the at least one modulator is IL-1, IL-2, IL-4, IL-6, IL-7, IL-10, IL-12, IL-15, IL-21, IL-27, GM-CSF, G-CSF, IFNα, IFNγ, a T cell receptor cross-linking antibody, a B cell receptor cross-linking antibody, or a combination thereof. In some embodiments, the cell is exposed to two or more modulators, simultaneously or sequentially. In some embodiments, the at least one activatable element is Stat1, Stat3, Stat5, Stat6, Lck, Lyn, Zap70, Syk, PLCγ2, p38, PI3K, or a combination thereof. In some embodiments, the activation level of two or more activatable elements is determined simultaneously or sequentially.

In some embodiments, the methods further comprise characterizing at least one pathway in the cell by determining the activation level of the at least one activatable element. In some embodiments, the methods further comprise determining a functional state of at least one pathway in the cell, wherein the functional state is based on the activation level of the at least one activatable element.

In some embodiments, the methods further comprise determining the presence or absence of one or more cell surface markers, intracellular markers, or a combination thereof. In some embodiments, the cell surface markers, intracellular markers, or combination thereof are independently selected from the group consisting of proteins, carbohydrates, lipids, nucleic acids, and metabolites. In some embodiments, determining the presence or absence of one or more cell surface markers or intracellular markers comprises determining the presence or absence of an epitope of the cell surface markers or intracellular markers. In some embodiments, the classification, diagnosis, prognosis, theranosis, and/or prediction of outcome of the autoimmune disease in the subject is based on both the activation level of the at least one activatable element and the presence or absence of the one or more cell surface markers, intracellular markers, or combination thereof.

In some embodiments, the activation level is determined by the binding of a binding element that is specific to a particular activation state of a particular activatable element. In some embodiments, the binding element comprises an antibody, recombinant protein, or fluorescent dye. In some embodiments, the step of determining the activation level comprises the use of flow cytometry, immunofluorescence, confocal microscopy, immunohistochemistry, immunoelectronmicroscopy, nucleic acid amplification, gene array, protein array, mass spectrometry, patch clamp, 2-dimensional gel electrophoresis, differential display gel electrophoresis, microsphere-based multiplex protein assays, ELISA, or label-free cellular assays to determine the activation level of one or more intracellular activatable element in single cells.

In some embodiments, the prediction of an outcome comprises the prediction of an increase in activity of the autoimmune disease. In some embodiments, the determination of the activation level of the one or more activatable elements guides the treatment of the subject.

In some embodiments, a cell is exposed to two or more therapeutic agents simultaneously or sequentially. In some embodiments, the methods comprise exposing a cell to at least one therapeutic agent and at least one additional modulator, simultaneously or sequentially, and characterizing at least one pathway by determining the activation level of at least one activatable element within the pathway. In some embodiments, the at least one therapeutic agent is selected from the group consisting of steroids, such as corticosteroids; cytokine depleting biologics, such as anti-TNFα, anti-IL1β, and anti-IL6; TNF-blocking agents, such as Infliximab, Adalimumab, and Etanercept; anti-interleukin agents, such as Anakinra, AMG1 08, Iguratimod, and Actemra; anti-B cell agents, such as Rituximab and Epratuzumab; anti-costimulatory molecule agents, such as Abatacept and Belimumab; tolerogenic agents (synthetic molecules directed to B cell surface DNA receptors), such as LJP 394 and TV-4710; anti-complement protein agents, such as Eculizumab; inhibitors of T cell signaling molecules, such as CP690550; inhibitors of cell migration, such as antagonist of chemokine receptors (Maraviroc, INCB3284); disease-modifying anti-rheumatic drugs, such as lefiunomide and methotrexate; sulfasalazine; hydroxychloroquine; azathioprine; cyclosporine; minocycline; D-penicillamine; equivalents thereof; and combinations thereof.

In some embodiments, the invention provides methods of classification, diagnosis, prognosis, theranosis, and/or prediction of an outcome of an autoimmune disease in a subject, the method comprising: a) contacting a first cell from a first cell population from the subject with: (i) at least a first modulator or a fragment thereof, or (ii) a presence of no modulator; b) contacting a second cell from a second cell population from the individual with: (i) at least a second modulator or a fragment thereof, or (ii) a presence of no modulator; c) determining an activation level of at least one activatable element in the first cell and the second cell; and c) classifying, diagnosing, prognosing, theranosing, and/or predicting an outcome of the autoimmune disease in the subject based on the activation level of the at least one activatable element. In some embodiments, the methods further comprise creating a response panel for the subject comprising the determined activation levels of the activatable elements.

In some embodiments, the first cell population and the second cell population are immune cells. In some embodiments, the first cell from the first cell population is a B cell and the second cell from the second cell population is a T cell. In some embodiments, the B cell is contacted with a presence of no modulator. In some embodiments, the at least one activatable element in the B cell type is selected from the group consisting of: CD69, Stat3, Lck, p-Lck, pZap70/Syk, pI3K, Stat5, and phospho-Stat3.

In some embodiments, the T cell is a naive CD4 T cell. In some embodiments, the at least one activatable element in the naive CD4 T cell is p-Stat1 and the naive CD4 T cell is contacted with IL6.

In some embodiments, the methods further comprise contacting a third cell from a third cell population from the subject with: (i) at least a second modulator or a fragment thereof, or (ii) a presence of no modulator; and determining an activation level of at least one activatable element in the third cell. In some embodiments, the third cell is a CD8 memory /effector T cell.

In some embodiments, the at least one activatable element is p-Lck and the CD8 memory /effector T cell is contacted with a T cell receptor stimulation. In some embodiments, the first cell from the first cell population is a CD4 T cell and the second cell from the second cell population is a CD8 T cell. In some embodiments, the at least one activatable element in the CD4 T cell and/or CD8 T cell is selected from the group consisting of: p-p38, Lck, p-Lck, p-Stat5, and PLCγ2.

In some embodiments, the modulator is a cytokine. In some embodiments, the modulator is a STAT pathway modulator.

In some embodiments, the T cell is a regulatory T cell. In some embodiments, the at least one activatable element is p-Stat5 and the regulatory T cell is contacted with IL-21.

In some embodiments, the invention provides method of categorizing disease activity in an autoimmune disease, comprising: (a)determining an activation level of at least one activatable element in a in one or more cells in a plurality of immune cell populations in response to: (i) at least a modulator or a fragment thereof, or (ii) a presence of no modulator; and (b) categorizing the disease activity, based on the activation level of at least one activatable element in the one or more cells.

In some embodiments, the disease is rheumatoid arthritis, and the at least one activatable element is selected from the group consisting of CD69, p-Stat5, p-Stat1, p-Lck, p-p38, Lck, p-Stat1, p-Stat3, p-p38, PLCγ2, and p-PLCγ2.

In some embodiments, the at least one activatable element, the one or more cells, and the modulator or presence of no modulator is selected from Table 1, Table 2, Table 3 or Table 4.

In some embodiments, the disease is systemic lupus erythematosis.

In some embodiments, the plurality of immune cell populations is a T cell population and a B cell population.

In some embodiments, the activation level of p-Stat 3 is determined in a Naïve CD8 T cell population in response to IFNα, the activation of p-Stat6 is determined in a B cell population in response to a presence of no modulator, and the activation level of p-Stat6 is determined in a B cell population in response to IL-10.

In some embodiments, the activation level of p-Stat 3 is determined in a Naïve CD8 T cell population in response to IFNα, the activation of p-Stat1 is determined in a Naïve CD8 T cell population in response to a presence of no modulator, and the activation level of the activation of p-Stat1 is determined in a Naïve CD4 T cell population in response to IL-6.

In some embodiments, the invention provides methods of predicting treatment responsiveness of a subject with rheumatoid arthritis, comprising: a) contacting a B cell from a B cell population from the subject with: (i) at least a first modulator or a fragment thereof, or (ii) a presence of no modulator; b) contacting a CD8 T cell from a CD8 T cell population from the subject with : (i) at least a second modulator or a fragment thereof, or (ii) a presence of no modulator; c) determining an activation level of at least one activatable element in the B cell and the CD8 T cell; and d) predicting treatment responsiveness of the subject based on the activation level of the at least one activatable element.

In some embodiments, the activation level of an activatable element selected from the group consisting of CD69, Stat3, Lck, p-Lck, pZap70/Syk, pI3K, Stat5, and phospho-Stat3 is determined in the B cell. In some embodiments, the activation level of an activatable element selected from the group consisting of Lck, and p-PLCγ2 is determined in the CD8 T cell.

In some embodiments, the subject is being or is going to be treated with Orencia.

In some embodiments, the invention provides methods of predicting changes in disease activity in a subject with systemic lupus erythematosis, comprising: a) contacting a Naïve CD8 cell from a CD8 cell population from the subject with: (i) at least a first modulator or a fragment thereof, or (ii) a presence of no modulator; b) contacting a B cell from a B cell population from the subject with: (i) at least a first modulator or a fragment thereof, or (ii) a presence of no modulator; c) determining an activation level of at least one activatable element in the B cell and the Naïve CD8 T cell; and d) predicting changes in disease activity in the subject based on the activation level of the at least one activatable element.

In some embodiments, the activation level of p-Stat3 is determined in the B cell in response to a modulator selected from the group consisting IL4, IL6, and IFNγ. In some embodiments, the activation level of p-Stat5 is determined in the B cell in response to IFNα. In some embodiments, the activation level of p-Stat6 is determined in the B cell in response to IL10, or a presence of no modulator. In some embodiments, the activation level of p-Stat1 is determined in the Naïve CD8 T cell in response to a modulator selected from the group consisting of IL-10, IFNα, and IL-6. In some embodiments, the activation level of p-Stat6 is determined in the Naïve CD8 T cell in response to a modulator selected from the group consisting of IL-15, and IFNα. In some embodiments, the activation level ofp-Stat5 is determined in the Naïve CD8 T cell in response to IL-21.

In some embodiments, for any of the methods described herein the Area under the curve (AUC) value is higher than 0.7. In some embodiments, for any of the methods described herein the AUC value is higher than 0.8. In some embodiments, for any of the methods described herein the AUC value is higher than 0.9. In some embodiments, for any of the methods described herein the positive predictive value is higher than 70%. In some embodiments, for any of the methods described herein the negative predictive value is higher than 70%.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**Figure 1** shows a corners classifier for the stratification of patients on the basis of treatment with Abatacept
**Figure 2** shows a receiver-operator characteristic curve for the corners classifier of Fig. 1.

### DEFINITIONS

As used herein, the term "theranosis" refers to the use of results obtained from a diagnostic method to direct the selection of, maintenance of, or changes to a therapeutic regimen, including but not limited to the choice of one or more therapeutic agents, changes in dose level, changes in dose schedule, changes in mode of administration, and changes in formulation. Diagnostic methods used to inform a theranosis can include any that provides information on the state of a disease, condition, or symptom.

The terms "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the amount of an agent that is sufficient to effect beneficial or desired results. The therapeutically effective amount will vary depending upon the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose will vary depending on the particular agent chosen, the dosing regimen to be followed, whether it is administered in combination with other compounds, timing of administration, the tissue to be imaged, and the physical delivery system in which it is carried.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

### DETAILED DESCRIPTION OF THE INVENTION

The present disclosure refers to the following documents for further information: Haskell et al, Cancer Treatment, 5th Ed., W.B. Saunders and Co., 2001; Alberts et al., The Molecular Biology of the Cell, 4th Ed., Garland Science, 2002; Vogelstein and Kinzler, The Genetic Basis of Human Cancer, 2d Ed., McGraw Hill, 2002; Michael, Biochemical Pathways, John Wiley and Sons, 1999; Weinberg, The Biology of Cancer, 2007; Immunobiology, Janeway et al. 7th Ed., Garland, and Leroith and Bondy, Growth Factors and Cytokines in Health and Disease, A Multi Volume Treatise, Volumes 1A and 1B, Growth Factors, 1996.

Patents and applications that are also of interest include: U.S. Patent Nos. 7,381,535, 7,393,656, 7,695,924 and 7,695,926 and U.S. Patent Application Nos. 10/193,462; 11/655,785; 11/655,789; 11/655,821; 11/338,957, 12/877,998; 12/784,478; 12/730,170; 12/703,741; 12/687,873; 12/617,438; 12/606,869; 12/713,165; 12/293,081; 12/581,536; 12/776,349; 12/538,643; 12/501,274; 61/079,537 ; 12/501,295; 12/688, 851; 12/471,158 ; 12/910,769; 12/460,029 ; 12/432,239; 12/432,720; and 12/229,476. Many of these references disclose single cell network profiling (SCNP). Some commercial reagents, protocols, software and instruments that are useful in some embodiments of the present invention are available at the Becton Dickinson Website http://www.bdbiosciences.com/features/products/, and the Beckman Coulter website, http://www.beckmancoulter.com/Default.asp?bhfv=7. Relevant articles include High-content single-cell drug screening with phosphospecific flow cytometry, Krutzik et al., Nature Chemical Biology 23: 132-42, 2007; Irish et al., FLt3 ligand Y591 duplication and Bcl-2 over expression are detected in acute myeloid leukemia cells with high levels of phosphorylated wild-type p53, Blood 109: 2589-96 2007; Irish et al. Mapping normal and cancer cell signaling networks: towards single-cell proteomics, Nature Rev. Cancer, 6: 146-55 2006; Irish et al., Single cell profiling of potentiated phospho-protein networks in cancer cells, Cell, Vol. 118, 1-20 July 23, 2004; Schulz, K. R., et al., Single-cell phospho-protein analysis by flow cytometry, Curr Protoc Immunol, Chapter 8: Units 8.17.1-20, 2007; Krutzik, P.O., et al., Coordinate analysis of murine immune cell surface markers and intracellular phosphoproteins by flow cytometry, J Immunol. 2005 1754: 2357-65; Krutzik, P.O., et al., Characterization of the murine immunological signaling network with phosphospecific flow cytometry, J Immunol. 175: 2366-73, 2005; Stelzer et al. Use of Multiparameter Flow Cytometry and Immunophenotyping for the Diagnosis and Classification of Acute Myeloid Leukemia, Immunophenotyping, Wiley, 2000; and Krutzik, P.O. and Nolan, G. P., Intracellular phospho-protein staining techniques for flow cytometry: monitoring single cell signaling events, Cytometry A.55:61-70, 2005; Hanahan D. ,Weinberg, The Hallmarks of Cancer, Cell 100:57-70, 2000; Krutzik et al, High content single cell drug screening with phosphospecific flow cytometry, Nat Chem Biol. 4:132-42, 2008. Guiding principles of statistical analysis can be found in Begg CB. (1987). Biases in the assessment of diagnostic tests. Stat in Med. 6, 411-423.; Bossuyt, P.M., et al. (2003) Towards complete and accurate reporting of studies of diagnostic accuracy: the STARD initiative. Clinical Chemistry 49, 1-6 (also in Ann. Intern. Med., BMJ and Radiology in 2003).; CDRH, FDA. (2003). Statistical Guidance on Reporting Results from Studies Evaluating Diagnostic Tests: Draft Guidance (March, 2003).; Pepe MS. (2003). The Statistical Evaluation of Medical Tests for Classification and Prediction. Oxford Press.; Zhou X-H, Obuchowski NA, McClish DK. (2002). Statistical Methods in Diagnostic Medicine. Wiley.

Multiparametric analyses of cells provide an approach for the simultaneous determination of the activation states of a plurality of cellular components. The activation status of the plurality of cellular components can be measured after exposure of cells to extracellular modulators and in so doing allows the signaling capacity of signaling networks to be determined when compared to the activation status of those networks in the absence of such modulators. The induced activation status of a protein rather than the frequently measured basal phosphorylation state of a protein has been shown in several studies to be more informative, as it takes into account (and reveals) signaling deregulation that is the consequence of numerous cytogenetic, epigenetic and molecular changes characteristic of cells associated with a disease state. For example, multiparameter flow cytometry at the single cell level measures the activation status of multiple intracellular signaling proteins as well as assigns activation states of these molecules to the varied cell sub-sets within complex primary cell populations.

Protein phosphorylation is a critical post translational process in controlling many cell functions such as migration, apoptosis, proliferation and differentiation. Site specific phosphorylation of proteins can be detected, for example, by incubating cells with fluorochrome-conjugated phospho-specific antibodies using flow cytometry. Recently, phospo-flow cytometry has emerged as a powerful tool to stratify cancer patients according to their risk of relapse using objective measures.

In one aspect, the present invention provides methods for the classification, diagnosis, prognosis, theranosis, and/or prediction of an outcome of an autoimmune disease in a subject. In one embodiment, the method comprises (a) exposing a cell from the subject to at least one modulator; (b) determining an activation level of at least one activatable element; and, (c) classifying, diagnosing, prognosing, theranosing, and/or predicting an outcome of said autoimmune disease in said subject based on said activation level of said at least one activatable element.

In some embodiments, the invention provides for methods for the classification, diagnosis, prognosis, theranosis, and/or prediction of an outcome of an autoimmune disease in a subject by determining an activation of at least one activatable element in one or more cells in a plurality of discrete cell populations. In some embodiments, one or more of the cell populations is exposed to the presence of one or more modulators, or the presence of no modulator. A cell population, as used herein, refers to a population of cells in which the majority of cells is of a same cell type or has a same characteristic. In some embodiments, the characterization of different discrete cell populations in a condition (e.g. rheumatoid arthritis) shows disruptions in cellular networks that are reflective of mechanisms that may be involved in the condition. In some embodiments, the disruption in these networks can be revealed by exposing a plurality of discrete cell populations to one or more modulators that mimic one or more environmental cues.

For many years, research into several conditions (e.g. autoimmune disease) has focused on attempts to identify a causative cell population comprised of cells of a single cell type. However, several discrete cell populations or the interactions between several cell populations may contribute to the pathology of a condition. For example, in the case of an immune cell, the immune cell may possess a dysregulated signaling pathway resulting in a defective (e.g., overactive) response to an environmental cue, or the defective production of a signaling factor (e.g., a cytokine). Without intending to be limited to any theory, several different cell types participate as part of the immune system, including B cells, T cells, macrophages, neutrophils, basophils and eosinophils. Each of these cell types has a distinct role in the immune system, and communicates with other immune cells using secreted factors called cytokines, including interleukins, TNF, and the interferons. Macrophages phagocytose foreign bodies and are antigen-presenting cells, using cytokines to stimulate specific antigen dependent responses by B and T cells and non-specific responses by other cell types. T cells secrete a variety of factors to coordinate and stimulate immune responses to specific antigen, such as the role of helper T cells in B cell activation in response to antigen. The proliferation and activation of eosinophils, neutrophils and basophils respond to cytokines as well. Cytokine communication is often local, within a tissue or between cells in close proximity. Each of the cytokines is secreted by one set of cells and provokes a response in another target set of cells, often including the cell that secretes the cytokine.

In a condition like rheumatoid arthritis (RA) contributions made by interactions between dendritic cells, T cells and other immune cells, and local production of cytokines and chemokines may contribute to the pathogenesis of RA. These cells further interact with local cells (e.g. synoviocytes). In response to local inflammation and production of proinflammatory cytokines, after unknown event dendritic cells, T cells and other immune cells are attracted to the synovium in response to local production of cytokines and chemokines. In some patients with rheumatoid arthritis, chronic inflammation leads to the destruction of the cartilage, bone, and ligaments, causing deformity of the joints. Damage to the joints can occur early in the disease and be progressive.

Thus, the successful diagnosis of a condition and use of therapies may require knowledge of the activation state data of different discrete cell populations that may play a role in the pathogenesis of a condition (e.g. autoimmune disease). The determination of the activation state data of different discrete cell populations that might interact directly or indirectly in a network serves as an indicator of the state of the network. In addition, it provides directionality to the interactions among the different discrete cell populations in the network. It also provides information across the cell populations participating in the network. As a result, the determination of activation state data of different discrete cell populations may serve as a better indicator of a condition than the analysis of a single cell population.

An autoimmune disease, as pertains to the present invention, is a disease or disorder arising from and directed against an individual's own tissues or a co-segregate or manifestation thereof or resulting condition therefrom. Examples of autoimmune diseases or disorders include, but are not limited to: arthritis, including rheumatoid arthritis, acute arthritis, chronic rheumatoid arthritis, gout or gouty arthritis, acute gouty arthritis, acute immunological arthritis, chronic inflammatory arthritis, degenerative arthritis, type II collagen-induced arthritis, infectious arthritis, Lyme arthritis, proliferative arthritis, psoriatic arthritis, Still's disease, vertebral arthritis, juvenile-onset rheumatoid arthritis, osteoarthritis, arthritis chronica progrediente, arthritis deformans, polyarthritis chronica primaria, reactive arthritis, and ankylosing spondylitis; inflammatory hyperproliferative skin diseases; psoriasis, such as plaque psoriasis, gutatte psoriasis, pustular psoriasis, and psoriasis of the nails; atopy, including atopic diseases such as hay fever and Job's syndrome; dermatitis, including contact dermatitis, chronic contact dermatitis, exfoliative dermatitis, allergic dermatitis, allergic contact dermatitis, dermatitis herpetiformis, nummular dermatitis, seborrheic dermatitis, non-specific dermatitis, primary irritant contact dermatitis, and atopic dermatitis; x-linked hyper IgM syndrome; allergic intraocular inflammatory diseases; urticaria, such as chronic allergic urticaria, chronic idiopathic urticaria, and chronic autoimmune urticaria; myositis; polymyositis/dermatomyositis; juvenile dermatomyositis; toxic epidermal necrolysis; scleroderma, including systemic scleroderma; sclerosis, such as systemic sclerosis, multiple sclerosis (MS), spino-optical MS, primary progressive MS (PPMS), relapsing remitting MS (RRMS), progressive systemic sclerosis, atherosclerosis, arteriosclerosis, sclerosis disseminata, and ataxic sclerosis; neuromyelitis optica (NMO); inflammatory bowel disease (IBD), including Crohn's disease, autoimmune-mediated gastrointestinal diseases, colitis, ulcerative colitis, colitis ulcerosa, microscopic colitis, collagenous colitis, colitis polyposa, necrotizing enterocolitis, transmural colitis, and autoimmune inflammatory bowel disease; bowel inflammation; pyoderma gangrenosum; erythema nodosum; primary sclerosing cholangitis; respiratory distress syndrome, including adult or acute respiratory distress syndrome (ARDS); meningitis; inflammation of all or part of the uvea; iritis; choroiditis; an autoimmune hematological disorder; rheumatoid spondylitis; rheumatoid synovitis; hereditary angioedema; cranial nerve damage, as in meningitis; herpes gestationis; pemphigoid gestationis; pruritis scroti; autoimmune premature ovarian failure; sudden hearing loss due to an autoimmune condition; IgE-mediated diseases, such as anaphylaxis and allergic and atopic rhinitis; encephalitis, such as Rasmussen's encephalitis and limbic and/or brainstem encephalitis; uveitis, such as anterior uveitis, acute anterior uveitis, granulomatous uveitis, nongranulomatous uveitis, phacoantigenic uveitis, posterior uveitis, or autoimmune uveitis; glomerulonephritis (GN) with and without nephrotic syndrome, such as chronic or acute glomerulonephritis, primary GN, immune-mediated GN, membranous GN (membranous nephropathy), idiopathic membranous GN or idiopathic membranous nephropathy, membrano- or membranous proliferative GN (MPGN), including Type I and Type II, and rapidly progressive GN; proliferative nephritis; autoimmune polyglandular endocrine failure; balanitis, including balanitis circumscripta plasmacellularis; balanoposthitis; erythema annulare centrifugum; erythema dyschromicum perstans; eythema multiform; granuloma annulare; lichen nitidus; lichen sclerosus et atrophicus; lichen simplex chronicus; lichen spinulosus; lichen planus; lamellar ichthyosis; epidermolytic hyperkeratosis; premalignant keratosis; pyoderma gangrenosum; allergic conditions and responses; allergic reaction; eczema, including allergic or atopic eczema, asteatotic eczema, dyshidrotic eczema, and vesicular palmoplantar eczema; asthma, such as asthma bronchiale, bronchial asthma, and auto-immune asthma; conditions involving infiltration of T cells and chronic inflammatory responses; immune reactions against foreign antigens such as fetal A-B-O blood groups during pregnancy; chronic pulmonary inflammatory disease; autoimmune myocarditis; leukocyte adhesion deficiency; lupus, including lupus nephritis, lupus cerebritis, pediatric lupus, non-renal lupus, extra-renal lupus, discoid lupus and discoid lupus erythematosus, alopecia lupus, systemic lupus erythematosus (SLE), cutaneous SLE, subacute cutaneous SLE, neonatal lupus syndrome (NLE), and lupus erythematosus disseminatus; juvenile onset (Type I) diabetes mellitus, including pediatric insulin-dependent diabetes mellitus (IDDM), adult onset diabetes mellitus (Type II diabetes), autoimmune diabetes, idiopathic diabetes insipidus, diabetic retinopathy, diabetic nephropathy, and diabetic large-artery disorder; immune responses associated with acute and delayed hypersensitivity mediated by cytokines and T-lymphocytes; tuberculosis; sarcoidosis; granulomatosis, including lymphomatoid granulomatosis; Wegener's granulomatosis; agranulocytosis; vasculitides, including vasculitis, large-vessel vasculitis, polymyalgia rheumatica and giant-cell (Takayasu's) arteritis, medium-vessel vasculitis, Kawasaki's disease, polyarteritis nodosa/periarteritis nodosa, microscopic polyarteritis, immunovasculitis, CNS vasculitis, cutaneous vasculitis, hypersensitivity vasculitis, necrotizing vasculitis, systemic necrotizing vasculitis, ANCA-associated vasculitis, Churg-Strauss vasculitis or syndrome (CSS), and ANCA-associated small-vessel vasculitis; temporal arteritis; aplastic anemia; autoimmune aplastic anemia; Coombs positive anemia; Diamond Blackfan anemia; hemolytic anemia or immune hemolytic anemia, including autoimmune hemolytic anemia (AIHA), pernicious anemia (anemia perniciosa); Addison's disease; pure red cell anemia or aplasia (PRCA); Factor VIII deficiency; hemophilia A; autoimmune neutropenia; pancytopenia; leukopenia; diseases involving leukocyte diapedesis; CNS inflammatory disorders; multiple organ injury syndrome, such as those secondary to septicemia, trauma or hemorrhage; antigen-antibody complex-mediated diseases; anti-glomerular basement membrane disease; anti-phospholipid antibody syndrome; allergic neuritis; Behçet's disease/syndrome; Castleman's syndrome; Goodpasture's syndrome; Reynaud's syndrome; Sjögren's syndrome; Stevens-Johnson syndrome; pemphigoid, such as pemphigoid bullous and skin pemphigoid, pemphigus, pemphigus vulgaris, pemphigus foliaceus, pemphigus mucus-membrane pemphigoid, and pemphigus erythematosus; autoimmune polyendocrinopathies; Reiter's disease or syndrome; thermal injury; preeclampsia; an immune complex disorder, such as immune complex nephritis, and antibody-mediated nephritis; polyneuropathies; chronic neuropathy, such as IgM polyneuropathies and IgM-mediated neuropathy; thrombocytopenia (as developed by myocardial infarction patients, for example), including thrombotic thrombocytopenic purpura (TTP), post-transfusion purpura (PTP), heparin-induced thrombocytopenia, autoimmune or immune-mediated thrombocytopenia, idiopathic thrombocytopenic purpura (ITP), and chronic or acute ITP; scleritis, such as idiopathic ceratoscleritis, and episcleritis; autoimmune disease of the testis and ovary including, autoimmune orchitis and oophoritis; primary hypothyroidism; hypoparathyroidism; autoimmune endocrine diseases, including thyroiditis, autoimmune thyroiditis, Hashimoto's disease, chronic thyroiditis (Hashimoto's thyroiditis), or subacute thyroiditis, autoimmune thyroid disease, idiopathic hypothyroidism, Grave's disease, polyglandular syndromes, autoimmune polyglandular syndromes, and polyglandular endocrinopathy syndromes; paraneoplastic syndromes, including neurologic paraneoplastic syndromes; Lambert-Eaton myasthenic syndrome or Eaton-Lambert syndrome; stiff-man or stiff-person syndrome; encephalomyelitis, such as allergic encephalomyelitis, encephalomyelitis allergica, and experimental allergic encephalomyelitis (EAE); myasthenia gravis, such as thymoma-associated myasthenia gravis; cerebellar degeneration; neuromyotonia; opsoclonus or opsoclonus myoclonus syndrome (OMS); sensory neuropathy; multifocal motor neuropathy; Sheehan's syndrome; hepatitis, including autoimmune hepatitis, chronic hepatitis, lupoid hepatitis, giant-cell hepatitis, chronic active hepatitis, and autoimmune chronic active hepatitis; lymphoid interstitial pneumonitis (LIP); bronchiolitis obliterans (non-transplant) vs NSIP; Guillain-Barre syndrome; Berger's disease (IgA nephropathy); idiopathic IgA nephropathy; linear IgA dermatosis; acute febrile neutrophilic dermatosis; subcorneal pustular dermatosis; transient acantholytic dermatosis; cirrhosis, such as primary biliary cirrhosis and pneumonocirrhosis; autoimmune enteropathy syndrome; Celiac or Coeliac disease; celiac sprue (gluten enteropathy); refractory sprue; idiopathic sprue; cryoglobulinemia; amylotrophic lateral sclerosis (ALS; Lou Gehrig's disease); coronary artery disease; autoimmune ear disease, such as autoimmune inner ear disease (AIED); autoimmune hearing loss; polychondritis, such as refractory or relapsed or relapsing polychondritis; pulmonary alveolar proteinosis; Cogan's syndrome/nonsyphilitic interstitial keratitis; Bell's palsy; Sweet's disease/syndrome; rosacea autoimmune; zoster-associated pain; amyloidosis; a non-cancerous lymphocytosis; a primary lymphocytosis, including monoclonal B cell lymphocytosis (e.g., benign monoclonal gammopathy and monoclonal gammopathy of undetermined significance, MGUS); peripheral neuropathy; channelopathies, such as epilepsy, migraine, arrhythmia, muscular disorders, deafness, blindness, periodic paralysis, and channelopathies of the CNS; autism; inflammatory myopathy; focal or segmental or focal segmental glomerulosclerosis (FSGS); endocrine opthalmopathy; uveoretinitis; chorioretinitis; autoimmune hepatological disorder; fibromyalgia; multiple endocrine failure; Schmidt's syndrome; adrenalitis; gastric atrophy; presenile dementia; demyelinating diseases, such as autoimmune demyelinating diseases and chronic inflammatory demyelinating polyneuropathy; Dressler's syndrome; alopecia areata; alopecia totalis; CREST syndrome (calcinosis, Raynaud's phenomenon, esophageal dysmotility, sclerodactyly, and telangiectasia); male and female autoimmune infertility (e.g., due to anti-spermatozoan antibodies); mixed connective tissue disease; Chagas' disease; rheumatic fever; recurrent abortion; farmer's lung; erythema multiforme; post-cardiotomy syndrome; Cushing's syndrome; bird-fancier's lung; allergic granulomatous angiitis; benign lymphocytic angiitis; Alport's syndrome; alveolitis, such as allergic alveolitis and fibrosing alveolitis; interstitial lung disease; transfusion reaction; leprosy; malaria; Samter's syndrome; Caplan's syndrome; endocarditis; endomyocardial fibrosis; diffuse interstitial pulmonary fibrosis; interstitial lung fibrosis; pulmonary fibrosis; idiopathic pulmonary fibrosis; cystic fibrosis; endophthalmitis; erythema elevatum et diutinum; erythroblastosis fetalis; eosinophilic faciitis; Shulman's syndrome; Felty's syndrome; flariasis; cyclitis, such as chronic cyclitis, heterochronic cyclitis, iridocyclitis (acute or chronic), or Fuch's cyclitis; Henoch-Schonlein purpura; sepsis; endotoxemia; pancreatitis; thyroxicosis; Evan's syndrome; autoimmune gonadal failure; Sydenham's chorea; post-streptococcal nephritis; thromboangitis ubiterans; thyrotoxicosis; tabes dorsalis; chorioiditis; giant-cell polymyalgia; chronic hypersensitivity pneumonitis; keratoconjunctivitis sicca; epidemic keratoconjunctivitis; idiopathic nephritic syndrome; minimal change nephropathy; benign familial and ischemia-reperfusion injury; transplant organ reperfusion; retinal autoimmunity; joint inflammation; bronchitis; chronic obstructive airway/pulmonary disease; silicosis; aphthae; aphthous stomatitis; arteriosclerotic disorders; aspermiogenese; autoimmune hemolysis; Boeck's disease; cryoglobulinemia; Dupuytren's contracture; endophthalmia phacoanaphylactica; enteritis allergica; erythema nodosum leprosum; idiopathic facial paralysis; febris rheumatica; Hamman-Rich's disease; sensoneural hearing loss; haemoglobinuria paroxysmatica; hypogonadism; ileitis regionalis; leucopenia; mononucleosis infectiosa; traverse myelitis; primary idiopathic myxedema; nephrosis; ophthalmia symphatica; orchitis granulomatosa; pancreatitis; polyradiculitis acuta; pyoderma gangrenosum; Quervain's thyreoiditis; acquired spenic atrophy; non-malignant thymoma; vitiligo; toxic-shock syndrome; food poisoning; conditions involving infiltration of T cells; leukocyte-adhesion deficiency; immune responses associated with acute and delayed hypersensitivity mediated by cytokines and T-lymphocytes; diseases involving leukocyte diapedesis; multiple organ injury syndrome; antigen-antibody complex-mediated diseases; antiglomerular basement membrane disease; allergic neuritis; autoimmune polyendocrinopathies; oophoritis; primary myxedema; autoimmune atrophic gastritis; sympathetic ophthalmia; rheumatic diseases; mixed connective tissue disease; nephrotic syndrome; insulitis; polyendocrine failure; autoimmune polyglandular syndrome type I; adult-onset idiopathic hypoparathyroidism (AOIH); cardiomyopathy such as dilated cardiomyopathy; epidermolisis bullosa acquisita (EBA); hemochromatosis; myocarditis; nephrotic syndrome; primary sclerosing cholangitis; purulent or nonpurulent sinusitis; acute or chronic sinusitis; ethmoid, frontal, maxillary, or sphenoid sinusitis; an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils; anaphylaxis; seronegative spondyloarthritides; polyendocrine autoimmune disease; sclerosing cholangitis; chronic mucocutaneous candidiasis; Bruton's syndrome; transient hypogammaglobulinemia of infancy; Wiskott-Aldrich syndrome; ataxia telangiectasia syndrome; angiectasis; autoimmune disorders associated with collagen disease, rheumatism, neurological disease, lymphadenitis, reduction in blood pressure response, vascular dysfunction, tissue injury, cardiovascular ischemia, hyperalgesia, renal ischemia, cerebral ischemia, and disease accompanying vascularization; allergic hypersensitivity disorders; glomerulonephritides; reperfusion injury; ischemic re-perfusion disorder; reperfusion injury of myocardial or other tissues; lymphomatous tracheobronchitis; inflammatory dermatoses; dermatoses with acute inflammatory components; multiple organ failure; bullous diseases; renal cortical necrosis; acute purulent meningitis or other central nervous system inflammatory disorders; ocular and orbital inflammatory disorders; granulocyte transfusion-associated syndromes; cytokine-induced toxicity; narcolepsy; acute serious inflammation; chronic intractable inflammation; pyelitis; endarterial hyperplasia; peptic ulcer; valvulitis; and endometriosis.

In some embodiments, the method provides for a diagnosis of an autoimmune disease. In other embodiments, the method provides for the classification of an autoimmune disease. Classification can include, but is not limited to, a determination of disease subtype, disease stage, disease activity level, and responsiveness to treatment. In other embodiments, a prediction is made as to the likelihood of a possible disease-related outcome. In one embodiment, the prediction relates to the future course of autoimmune disease activity. The future course of predicted activity can be a change, as in an increase or decrease in disease activity, or the future course of activity may be predicted to remain substantially similar to the activity level at the time of analysis. In some embodiments, predictions as to the future onset of a significant change in disease activity are made 1, 2, 3, 4, 5, 6, 7, 8, 9, 10, 11, 12, or more weeks in advance of the change in activity. In some embodiments, predictions as to the future onset of a significant change in disease activity are made 1, 2, 3, 4, 5, 6, 7, 8, 9, 10, 11, or more months in advance of the change in activity.

In some embodiments, a prediction is made as to the likelihood of responsiveness to one or more treatments. Treatments about which a prediction can be made can include a treatment the subject has not yet received, or changes in treatments with which a subject is being treated. Changes can include, but are not limited to changes in dose, dosing schedule, route of administration, and/or combination with additional treatments. In some embodiments, a prediction guides the selection of or changes in treatment received by a subject.

In some embodiments, the methods for classification, diagnosis, prognosis, theranosis, and/or prediction of outcome of an autoimmune disease in a subject are performed for at least two time points for a single subject. A subject can be subjected to the methods of the present invention at least 1, 2, 3, 4, 5, 6, 7, 8, 9, 10, 15, 20, 25, 30, 35, 40, 50, 100, 150, 200 times or more. The methods of the present invention can be repeated on a subject at intervals of less than, about, or more than 1, 2, 3, 4, 5, 6, 7, 8, 9, 10, 12, 14, 16, 18, 20, 22, or 24 hours; less than, about, or more than 1, 2, 3, 4, 5, 6, 7, 8, 9, 10, 12, 14 days; less than, about, or more than 1, 2, 3, 4, 5, 6, 7, 8, 9, 10, 12, or 14 weeks; less than, about, or more than 1, 2, 3, 4, 5, 6, 7, 8, 9, 10, 12, or 14 months; and less than, about, or more than 1, 2, 3, 4, 5, 6, 7, 8, 9, 10, 12, 14, or more years; each repetition representing a "time point" for the subject. In some embodiments, the methods of the present invention are used to monitor a subject for less than, about, or more than 1, 2, 3, 4, 5, 6, 7, 8, 9, 10, 12, 14 days; less than, about, or more than 1, 2, 3, 4, 5, 6, 7, 8, 9, 10, 12, or 14 weeks; less than, about, or more than 1, 2, 3, 4, 5, 6, 7, 8, 9, 10, 12, or 14 months; and less than, about, or more than 1, 2, 3, 4, 5, 6, 7, 8, 9, 10, 12, 14, or more years, including for the life of the subject. In some embodiments, two or more time points for the subject are compared. In some embodiments, a subject is undergoing treatment with a therapeutic agent. In some embodiments, a determination of the efficacy of the therapeutic agent with which the subject is being treated is made based on the comparison of two or more time points.

In some embodiments, a modulator to which a cell from a subject is exposed is a therapeutic agent, wherein the therapeutic agent is one used to treat an autoimmune disorder. Therapeutic agents used to treat an autoimmune disorder can include any designed for, used in, shown to have a beneficial effect in, or possibly having a beneficial effect in the treatment of an autoimmune disease. Non-limiting classes of therapeutic agents used to treat an autoimmune disorder include biological or chemical compounds such as a simple or complex organic or inorganic molecules, peptides, peptide mimetics, proteins, small molecules, aptamers, nucleic acid molecules, and antibodies. Examples of therapeutic agents commonly used to treat autoimmune disorders include, but are not limited to: steroids, such as corticosteroids; cytokine depleting biologics, such as anti-TNFα, anti-IL1β, and anti-IL6; TNF-blocking agents, such as Infliximab, Adalimumab, and Etanercept; anti-interleukin agents, such as Anakinra, AMG1 08, Iguratimod, and Actemra; anti-B cell agents, such as Rituximab and Epratuzumab; anti-costimulatory molecule agents, such as Abatacept and Belimumab; tolerogenic agents (synthetic molecules directed to B cell surface DNA receptors), such as LJP 394 and TV-4710; anti-complement protein agents, such as Eculizumab; inhibitors of T cell signaling molecules, such as CP690550; inhibitors of cell migration, such as antagonist of chemokine receptors (Maraviroc, INCB3284); disease-modifying anti-rheumatic drugs, such as lefiunomide and methotrexate; sulfasalazine; hydroxychloroquine; azathioprine; cyclosporine; minocycline; D-penicillamine; equivalents thereof; and combinations thereof. In some embodiments, a cell is exposed to two or more therapeutic agents simultaneously or in sequence. In further embodiments, a cell is exposed to one or more therapeutic agents and one or more additional modulator simultaneously or in sequence.

In another aspect, the invention provides a method for identifying therapeutic targets for the treatment of autoimmune disease in a subject. In one embodiment, the method comprises the steps of (a) exposing a cell from said subject to at least one modulator; (b) determining an activation level of at least one activatable element; and, (c) identifying one or more therapeutic targets for the treatment of said autoimmune disease in said subject based on said activation level of said at least one activatable element. In some embodiments, identifying one or more therapeutic targets involves identifying correlations between the level of or change in the level of an activatable element and one or more of: the presence, absence, stage, sub-type, activity level, and/or changes in activity level of an autoimmune disease. In some embodiments, correlations can indicate as possible therapeutic targets the activatable element, molecules associated with the activatable element, molecules that interact directly or indirectly with the activatable element, molecules regulating or regulated by the activatable element, and/or other activatable elements in the same or related pathways. In some embodiments, the therapeutic agents directed against the one or more therapeutic targets may act specifically on a single therapeutic target, specifically on two or more therapeutic targets, non-specifically on one or more therapeutic targets, on multiple therapeutic targets including one or more of the identified therapeutic targets, and/or a combination of therapeutic and non-therapeutic targets. Therapeutic targets can be targeted individually, in combination, simultaneously, and/or in sequence.

### Samples and Sampling

The methods involve analysis of one or more samples from an individual. An individual is any multicellular organism; in some embodiments, the individual is an animal, e.g., a mammal. In some embodiments, the individual is a human.

The sample can be any suitable type that allows for the analysis of one or more cells. Samples can be obtained once or multiple times from an individual. Multiple samples can be obtained from different locations in the individual (e.g., blood samples, bone marrow samples and/or lymph node samples), at different times from the individual (e.g., a series of samples taken to monitor response to treatment or to monitor for return of a pathological condition), or any combination thereof. These and other possible sampling combinations based on the sample type, location, and time of sampling allows for the detection of the presence of pre-pathological or pathological cells, the measurement of treatment response, and also the monitoring for disease.

When samples are obtained as a series, e.g., a series of blood samples obtained after treatment, the samples can be obtained at fixed intervals, at intervals determined by the status of the most recent sample or samples or by other characteristics of the individual, or some combination thereof. For example, samples can be obtained at intervals of approximately 1, 2, 3, or 4 weeks, at intervals of approximately 1, 2, 3, 4, 5, 6, 7, 8, 9, 10, or 11 months, at intervals of approximately 1, 2, 3, 4, 5, or more than 5 years, or some combination thereof. It will be appreciated that an interval may not be exact, according to an individual's availability for sampling and the availability of sampling facilities, thus approximate intervals corresponding to an intended interval scheme are encompassed by the invention. As an example, an individual who has undergone treatment for a cancer can be sampled (e.g., by blood draw) relatively frequently (e.g., every month or every three months) for the first six months to a year after treatment, then, if no abnormality is found, less frequently (e.g., at times between six months and a year) thereafter. If, however, any abnormalities or other circumstances are found in any of the intervening times, or during the sampling, sampling intervals can be modified.

Generally, the most easily obtained samples are fluid samples. Fluid samples include normal and pathologic bodily fluids and aspirates of those fluids. Fluid samples also comprise rinses of organs and cavities (lavage and perfusions). Bodily fluids include whole blood, bone marrow aspirate, synovial fluid, cerebrospinal fluid, saliva, sweat, tears, semen, sputum, mucus, menstrual blood, breast milk, urine, lymphatic fluid, amniotic fluid, placental fluid and effusions such as cardiac effusion, joint effusion, pleural effusion, and peritoneal cavity effusion (ascites). Rinses can be obtained from numerous organs, body cavities, passage ways, ducts and glands. Sites that can be rinsed include lungs (bronchial lavage), stomach (gastric lavage), gastrointestinal track (gastrointestinal lavage), colon (colonic lavage), vagina, bladder (bladder irrigation), breast duct (ductal lavage), oral, nasal, sinus cavities, and peritoneal cavity (peritoneal cavity perfusion). In some embodiments the sample or samples is blood.

Solid tissue samples can also be used, either alone or in conjunction with fluid samples. Solid samples can be derived from individuals by any method known in the art including surgical specimens, biopsies, and tissue scrapings, including cheek scrapings. Surgical specimens include samples obtained during exploratory, cosmetic, reconstructive, or therapeutic surgery. Biopsy specimens can be obtained through numerous methods including bite, brush, cone, core, cytological, aspiration, endoscopic, excisional, exploratory, fine needle aspiration, incisional, percutaneous, punch, stereotactic, and surface biopsy.

In some embodiments, the sample is a blood sample. In some embodiments, the sample is a bone marrow sample. In some embodiments, the sample is a lymph node sample. In some embodiments, the sample is cerebrospinal fluid. In some embodiments, combinations of one or more of a blood, bone marrow, cerebrospinal fluid, and lymph node sample are used.

One or more cells or cell types, or samples containing one or more cells or cell types, can be isolated from body samples. The cells can be separated from body samples by centrifugation, elutriation, density gradient separation, apheresis, affinity selection, panning, FACS, centrifugation with Hypaque, solid supports (magnetic beads, beads in columns, or other surfaces) with attached antibodies, etc. By using antibodies specific for markers identified with particular cell types, a relatively homogeneous population of cells can be obtained. Alternatively, a heterogeneous cell population can be used. Cells can also be separated by using filters. For example, whole blood can also be applied to filters that are engineered to contain pore sizes that select for the desired cell type or class. Rare pathogenic cells can be filtered out of diluted, whole blood following the lysis of red blood cells by using filters with pore sizes between 5 to 10 µm, as disclosed in U.S. Patent Application No. 09/790,673. Once a sample is obtained, it can be used directly, frozen, or maintained in appropriate culture medium for short periods of time. Methods to isolate one or more cells for use according to the methods of this invention are performed according to standard techniques and protocols well-established in the art. See also U.S.S. Nos. 61/048,886; 61/048,920; and 61/048,657. See also, the commercial products from companies such as BD and BCI as identified above. See also U.S. Patent Nos. 7,381,535 and 7,393,656. All of the above patents and applications are incorporated by reference as stated above.

In some embodiments, the cells are cultured post collection in a media suitable for revealing the activation level of an activatable element (e.g. RPMI, DMEM) in the presence, or absence, of serum such as fetal bovine serum, bovine serum, human serum, porcine serum, horse serum, or goat serum. When serum is present in the media it could be present at a level ranging from 0.0001 % to 30%.

### Modulators

In some embodiments, the methods utilize a modulator. A modulator can be an activator, a therapeutic agent, an inhibitor or a compound capable of impacting cellular signaling networks. Modulators can take the form of a wide variety of environmental cues and inputs. In some embodiments, a cell is exposed to a modulator. Exposing a cell to a modulator can include the introduction of modulator to a cell's surrounding environment, the change of a physical parameter of a cell's environment, and contacting a cell with a modulator. In some embodiments, the modulator is selected from the group comprising: growth factors, cytokines, adhesion molecules, drugs, therapeutic agents, hormones, small molecules, polynucleotides, antibodies, natural compounds, lactones, chemotherapeutic agents, immune modulators, carbohydrates, proteases, ions, reactive oxygen species, radiation, physical parameters such as heat, cold, UV radiation, peptides, and protein fragments, either alone or in the context of cells, cells themselves, viruses, and biological and non-biological complexes (e.g. beads, plates, viral envelopes, antigen presentation molecules such as major histocompatibility complex). Examples of modulators include but are not limited to IL-1, IL-2, IL-3, IL-4, IL-6, IL-7, IL-10, IL-12, IL-15, IL-21, IL-27, GM-CSF, G-CSF, IFNα, IFNγ, T cell receptor (TCR) cross-linking antibodies, B cell receptor (BCR) cross-linking antibodies SDF-1α, FLT-3L, IGF-1, M-CSF, SCF, PMA, Thapsigargin, H₂O₂, etoposide, AraC, daunorubicin, staurosporine, benzyloxycarbonyl-Val-Ala-Asp (OMe) fluoromethylketone (ZVAD), lenalidomide, EPO, azacitadine, decitabine, LPS, TNF-α, and CD40L. In some embodiments, the modulator is an activator. In some embodiments the modulator is an inhibitor. In some embodiments, the modulators include growth factors, cytokines, chemokines, phosphatase inhibitors, and pharmacological reagents. The response panel is composed of at least one of: IL-1, IL-2, IL-3, IL-4, IL-6, IL-7, IL-10, IL-12, IL-15, IL-21, IL-27, GM-CSF, G-CSF, IFNα, IFNγ, T cell receptor cross-linking antibodies, B cell receptor cross-linking antibodies SDF-1α, FLT-3L, IGF-1, M-CSF, SCF, PMA, Thapsigargin, H₂O₂, etoposide, AraC, daunorubicin, staurosporine, benzyloxycarbonyl-Val-Ala-Asp (OMe) fluoromethylketone (ZVAD), lenalidomide, EPO, azacitadine, decitabine, LPS, TNF-α, and CD40L. Examples of TCR crosslinking antibodies include, but are not limited to, anti-CD3 and anti CD28 antibodies. Examples of BCR crosslinking antibodies include, but are not limited to, anti-IgG, anti-IgM, anti-kappa, and anti-lambda antibodies.

Modulation can be performed in a variety of environments. In some embodiments, cells are exposed to a modulator immediately after collection. In some embodiments where there is a mixed population of cells, purification of cells is performed after modulation. In some embodiments, whole blood is collected to which a modulator is added. In some embodiments, cells are modulated after processing for single cells or purified fractions of single cells. As an illustrative example, whole blood can be collected and processed for an enriched fraction of lymphocytes that is then exposed to a modulator. Modulation can include exposing cells to more than one modulator. For instance, in some embodiments, cells are exposed to at least 2, 3, 4, 5, 6, 7, 8, 9, or 10 modulators. In some embodiments, cells are exposed to at least two modulators, wherein one modulator is an activator and one modulator is an inhibitor, at least one modulator is an inhibitor, or at least one modulator is an activator. In some embodiments, cells are exposed to two or more modulators simultaneously or in sequence. In some embodiments, the effect of one or more modulators is compared to untreated cells (cells not exposed to the one or more modulators) to characterize the level of response to modulation. See U.S. Patent Application 61/048,657 which is incorporated by reference.

In some embodiments, cells are cultured after collection in a suitable media before exposure to a modulator. In some embodiments, the media is a growth media. In some embodiments, the growth media is a complex media that can include serum. In some embodiments, the growth media comprises serum. In some embodiments, the serum is selected from the group consisting of fetal bovine serum, bovine serum, human serum, porcine serum, horse serum, and goat serum. In some embodiments, the serum level ranges from 0.0001% to 30 %. In some embodiments, the growth media is a chemically defined minimal media and is without serum. In some embodiments, cells are cultured in a differentiating media.

Modulators include chemical and biological entities, and physical or environmental stimuli. Modulators can act extracellularly or intracellularly. Chemical and biological modulators include growth factors, cytokines, neurotransmitters, adhesion molecules, hormones, small molecules, inorganic compounds, polynucleotides, antibodies, natural compounds, lectins, lactones, chemotherapeutic agents, biological response modifiers, carbohydrates, proteases and free radicals. Modulators include complex and undefined biologic compositions that can comprise cellular or botanical extracts, cellular or glandular secretions, physiologic fluids such as serum, amniotic fluid, or venom. Physical and environmental stimuli include electromagnetic, ultraviolet, infrared or particulate radiation, redox potential and pH, the presence or absences of nutrients, changes in temperature, changes in oxygen partial pressure, changes in ion concentrations and the application of oxidative stress. Modulators can be endogenous or exogenous and may produce different effects depending on the concentration and duration of exposure to the single cells or whether they are used in combination or sequentially with other modulators. Modulators can act directly on the activatable elements or indirectly through the interaction with one or more intermediary biomolecule. Indirect modulation includes alterations of gene expression wherein the expressed gene product is the activatable element or is a modulator of the activatable element.

In some embodiments, the modulator is an inhibitor. In some embodiments, the inhibitor is an inhibitor of a cellular factor or a plurality of factors that participates in a cellular pathway (e.g. signaling cascade) in the cell. In some embodiments, the inhibitor is a phosphatase inhibitor. Examples of phosphatase inhibitors include, but are not limited to H2O2, siRNA, miRNA, Cantharidin, (-)-p-Bromotetramisole, Microcystin LR, Sodium Orthovanadate, Sodium Pervanadate, Vanadyl sulfate, Sodium oxodiperoxo(1,10-phenanthroline)vanadate, bis(maltolato)oxovanadium(IV), Sodium Molybdate, Sodium Perm olybdate, Sodium Tartrate, Imidazole, Sodium Fluoride, β-Glycerophosphate, Sodium Pyrophosphate Decahydrate, Calyculin A, Discodermia calyx, bpV(phen), mpV(pic), DMHV, Cypermethrin, Dephostatin, Okadaic Acid, NIPP-1, N-(9,10-Dioxo-9,10-dihydro-phenanthren-2-yl)-2,2-dimethyl-propionamide, α-Bromo-4-hydroxyacetophenone, 4-Hydroxyphenacyl Br, α-Bromo-4-methoxyacetophenone, 4-Methoxyphenacyl Br, α-Bromo-4-(carboxymethoxy)acetophenone, 4-(Carboxymethoxy)phenacyl Br, and bis(4-Trifluoromethylsulfonamidophenyl)-1,4-diisopropylbenzene, phenylarsine oxide, Pyrrolidine Dithiocarbamate, and Aluminium fluoride.

### Activatable Elements

In some embodiments, the invention is directed to methods for determining the activation level of one or more activatable elements in a cell before and/or after treatment with one or more modulators. The activation of an activatable element in the cell upon treatment with one or more modulators can reveal operative pathways in a condition that can then be used, e.g., as an indicator to predict the course of the condition, to identify a risk group, to predict an increased risk of developing secondary complications or suffering harmful side effects, to choose a therapy for an individual, to predict response to a therapy for an individual, to determine the efficacy of a therapy in an individual, and to determine the prognosis for an individual.

In some embodiments, the activation level of an activatable element in a cell is determined by exposing the cell to at least 1, 2, 3, 4, 5, 6, 7, 8, 9, or 10 modulators. In some embodiments, the activation level of an activatable element in a cell is determined by exposing the cell to at least 1, 2, 3, 4, 5, 6, 7, 8, 9, or 10 modulators where at least one of the modulators is an inhibitor. In other embodiments, the activation level of an activatable element in a cell is determined by exposing the cell to at least 1, 2, 3, 4, 5, 6, 7, 8, 9, or 10 modulators where at least one of the modulators is an activator. In some embodiments, the activation level of an activatable element in a cell is determined by exposing the cell to an inhibitor and another modulator, where the modulator can be an inhibitor or an activator. In some embodiments, the activation level of an activatable element in a cell is determined by exposing the cell to an inhibitor and an activator. In some embodiments, the activation level of an activatable element in a cell is determined by exposing the cell to two or more modulators.

In some embodiments, a phenotypic profile of a population of cells is determined by measuring the activation level of an activatable element when the population of cells is exposed to a plurality of modulators in separate cultures. Non-limiting examples of modulators include IL-1, IL-2, IL-3, IL-4, IL-6, IL-7, IL-10, IL-12, IL-15, IL-21, IL-27, GM-CSF, G-CSF, IFNα, IFNγ, T cell receptor (TCR) cross-linking antibodies, B cell receptor (BCR) cross-linking antibodies SDF-1a, FLT-3L, IGF-1, M-CSF, SCF, PMA, Thapsigargin, H₂O₂, etoposide, AraC, daunorubicin, staurosporine, benzyloxycarbonyl-Val-Ala-Asp (OMe) fluoromethylketone (ZVAD), lenalidomide, EPO, azacitadine, decitabine, LPS, TNF-α, and CD40L.and/or a combination thereof. Examples of TCR crosslinking antibodies include, but are not limited to, anti-CD3 and anti CD28 antibodies. Examples of BCR crosslinking antibodies include, but are not limited to, anti-IgG, anti-IgM, anti-kappa, and anti-lambda antibodies. In some embodiments, the phenotypic profile of the population of cells is used to classify the population as described herein.

The methods and compositions of the invention can be employed to examine and profile the status of any activatable element in a cellular pathway, or collections of such activatable elements. Single or multiple distinct pathways can be profiled (sequentially or simultaneously), or subsets of activatable elements within a single pathway or across multiple pathways can be examined (sequentially or simultaneously).

As will be appreciated by those in the art, a wide variety of activation events can find use in the present invention. In general, the basic requirement is that the activation results in a change in an activatable element that is quantitatable by some indication (termed an "activation state indicator"), preferably by altered binding of a labeled binding element or by changes in detectable biological activities (e.g., the activated state has an enzymatic activity which can be measured and compared to a lack of activity in the non-activated state, or the cell cycle arrests at a certain point, resulting in a specific level of DNA accumulation).

The activation level of an individual activatable element represents a relative quantity of the activation element. The activation level can be represented by numeric values or partitioned into categorical groups associated with activation states such as high activation/low activation/no activation or an "on or off" state. As an illustrative example, and without intending to be limited to any mechanism or process, an individual phosphorylatable site on a protein can activate or deactivate the protein. Additionally, phosphorylation of an adapter protein may promote its interaction with other components/proteins of distinct cellular signaling pathways. The terms "on" and "off," when applied to an activatable element that is a part of a cellular constituent, are used herein to describe the state of the activatable element, and not the overall state of the cellular constituent of which it is a part. Typically, a cell possesses a plurality of a particular protein or other constituent with a particular activatable element and this plurality of proteins or constituents usually has some proteins or constituents whose individual activatable element is in the on state and other proteins or constituents whose individual activatable element is in the off state. Since the activation state of each activatable element is measured through the use of a binding element that recognizes a specific activation state, only those activatable elements in the specific activation state recognized by the binding element, representing some fraction of the total number of activatable elements, will be bound by the binding element to generate a measurable signal. The measurable signal corresponding to the summation of individual activatable elements of a particular type that are activated in a single cell is the "activation level" for that activatable element in that cell.

Activation levels for a particular activatable element can vary among individual cells so that when a plurality of cells is analyzed, the activation levels follow a distribution. The distribution can be a normal distribution, also known as a Gaussian distribution, or it can be of another type. Different populations of cells can have different distributions of activation levels that can then serve to distinguish between the populations.

In some embodiments, the basis for classifying cells is that the distribution of activation levels for one or more specific activatable elements will differ among different phenotypes. A certain activation level, or more typically a range of activation levels for one or more activatable elements seen in a cell or a population of cells, is indicative that that cell or population of cells belongs to a distinctive phenotype. Other measurements, such as cellular levels (e.g., expression levels) of biomolecules that may not contain activatable elements, can also be used to classify cells in addition to activation levels of activatable elements; it will be appreciated that these levels also will follow a distribution, similar to activatable elements. Thus, the activation level or levels of one or more activatable elements, optionally in conjunction with the level of one or more biomolecules that may or may not contain activatable elements, of a cell or a population of cells can be used to classify a cell or a population of cells into a class. Once the activation level of intracellular activatable elements of individual single cells is known they can be placed into one or more classes, e.g., a class that corresponds to a phenotype. A class encompasses a class of cells wherein every cell has the same or substantially the same known activation level, or range of activation levels, of one or more intracellular activatable elements. For example, if the activation levels of five intracellular activatable elements are analyzed, predefined classes of cells that encompass one or more of the intracellular activatable elements can be constructed based on the activation level, or ranges of the activation levels, of each of these five elements. It is understood that activation levels can exist as a distribution and that an activation level of a particular element used to classify a cell can be a particular point on the distribution but more typically can be a portion of the distribution.

In addition to activation levels of intracellular activatable elements, levels of intracellular or extracellular biomolecules, e.g., proteins, can be used alone or in combination with activation states of activatable elements to classify cells. Further, additional cellular elements, e.g., biomolecules or molecular complexes such as RNA, DNA, carbohydrates, metabolites, and the like, can be used in conjunction with activatable states or expression levels in the classification of cells encompassed here.

In some embodiments, other characteristics that affect the status of a cellular constituent can also be used to classify a cell. Examples include the translocation of biomolecules or changes in their turnover rates and the formation and disassociation of complexes of biomolecule. Such complexes can include multi-protein complexes, multi-lipid complexes, homo- or hetero-dimers or oligomers, and combinations thereof. Other characteristics include proteolytic cleavage, e.g. from exposure of a cell to an extracellular protease or from the intracellular proteolytic cleavage of a biomolecule.

Additional elements can also be used to classify a cell, such as the presence or absence of extracellular markers, surface markers, intracellular markers, nuclear antigens, enzymatic activity, protein expression and localization, cell cycle analysis, chromosomal analysis, cell volume, and morphological characteristics like granularity and size of nucleus or other distinguishing characteristics. Non-limiting examples of cell surface markers and intracellular markers include proteins, carbohydrates, lipids, nucleic acids, and metabolites. For example, B cells can be further subdivided based on the expression of cell surface markers such as CD19, CD20, CD22 or CD23. Other non-limiting examples of markers useful for the classification of cells include CD3, CD4, CD8, CD19, CD25, CD33, CD45RA, CD69, and Foxp3. Cells can be categorized for the presence, absence, high level, or low level of one or more markers. Markers can be used alone or in combination. For example, cells can be classified by using 1, 2, 3, 4, 5, 6, 7, 8, 9, 10, or more markers.

Alternatively, predefined classes of cells can be aggregated or grouped based upon shared characteristics that can include inclusion in one or more additional predefined classes or the presence of extracellular or intracellular markers, similar gene expression profile, nuclear antigens, enzymatic activity, protein expression and localization, cell cycle analysis, chromosomal analysis, cell volume, and morphological characteristics like granularity and size of nucleus or other distinguishing cellular characteristics.

In some embodiments, the physiological status of one or more cells is determined by examining and profiling the activation level of one or more activatable elements in a cellular pathway. In some embodiments, a cell is classified according to the activation level of a plurality of activatable elements. In some embodiments, a cell is classified according to the activation levels of a plurality of activatable elements. In some embodiments, 1, 2, 3, 4, 5, 6, 7, 8, 9, 10 or more activatable elements can be analyzed in a cell signaling pathway. In some embodiments, the activation levels of one or more activatable elements of a cell are correlated with a condition. In some embodiments, the condition is an autoimmune disease. In some embodiments, the cell is a hematopoietic cell. In further embodiments, an activation level that is found to be correlated with a condition is used as a classifying, diagnostic, prognostic, theranostic, or predictive indicator of the condition in a subject. In this way, new markers associated with a particular classification, diagnosis, prognosis, theranosis, or prediction are identified.

In some embodiments, the activation level of one or more activatable elements in single cells in the sample is determined. Cellular constituents that may include activatable elements include without limitation proteins, carbohydrates, lipids, nucleic acids, and metabolites. The activatable element can be a portion of the cellular constituent, for example, an amino acid residue in a protein that may undergo phosphorylation, or it can be the cellular constituent itself, for example, a protein that is activated by translocation, change in conformation (due to, e.g., change in pH or ion concentration), by proteolytic cleavage, degradation through ubiquitination and the like. Upon activation, a change occurs to the activatable element, such as covalent modification of the activatable element (e.g., binding of a molecule or group to the activatable element, such as phosphorylation) or a conformational change. Such changes generally contribute to changes in particular biological, biochemical, or physical properties of the cellular constituent that contains the activatable element. The state of the cellular constituent that contains the activatable element is determined to some degree, though not necessarily completely, by the state of a particular activatable element of the cellular constituent. For example, a protein may have multiple activatable elements, and the particular activation states of these elements may overall determine the activation state of the protein; the state of a single activatable element is not necessarily determinative. Additional factors, such as the binding of other proteins, pH, ion concentration, interaction with other cellular constituents, and the like, can also affect the state of the cellular constituent.

In some embodiments, the activation levels of a plurality of intracellular activatable elements in single cells are determined. In some embodiments, at least about 1, 2, 3, 4, 5, 6, 7, 8, 9, 10 or more than 10 intracellular activatable elements are determined.

Activation states of activatable elements can result from chemical additions or modifications of biomolecules and include biochemical processes such as glycosylation, phosphorylation, acetylation, methylation, biotinylation, glutamylation, glycylation, hydroxylation, isomerization, prenylation, myristoylation, lipoylation, phosphopantetheinylation, sulfation, ISGylation, nitrosylation, palmitoylation, SUMOylation, ubiquitination, neddylation, citrullination, amidation, and disulfide bond formation, disulfide bond reduction. Other possible chemical additions or modifications of biomolecules include the formation of protein carbonyls, direct modifications of protein side chains, such as o-tyrosine, chloro-, nitrotyrosine, and dityrosine, and protein adducts derived from reactions with carbohydrate and lipid derivatives. Other modifications can be non-covalent, such as binding of a ligand or binding of an allosteric modulator.

One example of a covalent modification is the substitution of a phosphate group for a hydroxyl group in the side chain of an amino acid (phosphorylation). A wide variety of proteins are known that recognize specific protein substrates and catalyze the phosphorylation of serine, threonine, or tyrosine residues on their protein substrates. Such proteins are generally termed "kinases." Substrate proteins that are capable of being phosphorylated are often referred to as phosphoproteins (after phosphorylation). Once phosphorylated, a substrate phosphoprotein may have its phosphorylated residue converted back to a hydroxylated residue by the action of a protein phosphatase that specifically recognizes the substrate protein. Protein phosphatases catalyze the replacement of phosphate groups by hydroxyl groups on serine, threonine, or tyrosine residues. Through the action of kinases and phosphatases a protein may be reversibly phosphorylated on a multiplicity of residues and its activity may be regulated thereby. Thus, the presence or absence of one or more phosphate groups in an activatable protein is a preferred readout in the present invention.

Another example of a covalent modification of an activatable protein is the acetylation of histones. Through the activity of various acetylases and deacetlylases the DNA binding function of histone proteins is tightly regulated. Furthermore, histone acetylation and histone deactelyation have been linked with malignant progression. See Nature, 429: 457-63, 2004.

Another form of activation involves cleavage of the activatable element. For example, one form of protein regulation involves proteolytic cleavage of a peptide bond. While random or misdirected proteolytic cleavage may be detrimental to the activity of a protein, many proteins are activated by the action of proteases that recognize and cleave specific peptide bonds. Many proteins derive from precursor proteins, or pro-proteins, which give rise to a mature isoform of the protein following proteolytic cleavage of specific peptide bonds. Many growth factors are synthesized and processed in this manner, with a mature isoform of the protein typically possessing a biological activity not exhibited by the precursor form. Many enzymes are also synthesized and processed in this manner, with a mature isoform of the protein typically being enzymatically active, and the precursor form of the protein being enzymatically inactive. This type of regulation is generally not reversible. Accordingly, to inhibit the activity of a proteolytically activated protein, mechanisms other than "reattachment" must be used. For example, many proteolytically activated proteins are relatively short-lived proteins, and their turnover effectively results in deactivation of the signal. Inhibitors can also be used. Among the enzymes that are proteolytically activated are serine and cysteine proteases, including cathepsins and caspases respectively. Many other proteolytically activated enzymes, known in the art as "zymogens," also find use in the instant invention as activatable elements.

In an alternative embodiment, the activation of the activatable element involves prenylation of the element. By "prenylation", and grammatical equivalents used herein, is meant the addition of any lipid group to the element. Common examples of prenylation include the addition of farnesyl groups, geranylgeranyl groups, myristoylation, and palmitoylation. In general these groups are attached via thioether linkages to the activatable element, although other attachments can be used.

In another embodiment, activation of the activatable element is detected as intermolecular clustering of the activatable element. By "clustering" or "multimerization", and grammatical equivalents used herein, is meant any reversible or irreversible association of one or more signal transduction elements. Clusters can be made up of 2, 3, 4, etc., elements. Clusters of two elements are termed dimers. Clusters of 3 or more elements are generally termed oligomers, with individual numbers of clusters having their own designation; for example, a cluster of 3 elements is a trimer, a cluster of 4 elements is a tetramer, etc. Clusters can be made up of identical elements or different elements. Clusters of identical elements are termed "homo" clusters, while clusters of different elements are termed "hetero" clusters. Accordingly, a cluster can be a homodimer, as is the case for the β2-adrenergic receptor. Alternatively, a cluster can be a heterodimer, as is the case for GABA B-R. In other embodiments, the cluster is a homotrimer, as in the case of TNFα, or a heterotrimer such as the one formed by membrane-bound and soluble CD95 to modulate apoptosis. In further embodiments the cluster is a homo-oligomer, as in the case of Thyrotropin releasing hormone receptor, or a hetero-oligomer, as in the case of TGFβ1.

In some embodiments, the activation or signaling potential of elements is mediated by clustering, irrespective of the actual mechanism by which the element's clustering is induced. For example, elements can be activated to cluster a) as membrane bound receptors by binding to ligands (ligands including both naturally occurring or synthetic ligands), b) as membrane bound receptors by binding to other surface molecules, or c) as intracellular (non-membrane bound) receptors binding to ligands.

In another embodiment, the activatable elements are membrane bound receptor elements that cluster upon ligand binding such as cell surface receptors. As used herein, "cell surface receptor" refers to molecules that occur on the surface of cells, interact with the extracellular environment, and transmit or transduce (through signals) the information regarding the environment intracellularly in a manner that can modulate cellular activity directly or indirectly, e.g., via intracellular second messenger activities or transcription from specific promoters, resulting in transcription of specific genes. One class of receptor elements includes membrane bound proteins, or complexes of proteins, which are activated to cluster upon ligand binding. As is known in the art, these receptor elements can have a variety of forms, but in general they comprise at least three domains. First, these receptors have a ligand-binding domain, which can be oriented either extracellularly or intracellularly, usually the former. Second, these receptors have a membrane-binding domain (usually a transmembrane domain), which can take the form of a seven pass transmembrane domain (discussed below in connection with G-protein-coupled receptors) or a lipid modification, such as myristylation, to one of the receptor's amino acids which allows for membrane association when the lipid inserts itself into the lipid bilayer. Finally, the receptor has a signaling domain, which is responsible for propagating the downstream effects of the receptor.

Examples of such receptor elements include hormone receptors, steroid receptors, cytokine receptors, such as IL1-α, IL-β, IL-2, IL-3, IL-4, IL-5, IL-6, IL-7, IL-8, IL-9, IL-10. IL-12, IL-15, IL-18, IL-21, CCR5, CCR7, CCR-1-10, CCL20, chemokine receptors, such as CXCR4, adhesion receptors and growth factor receptors, including, but not limited to, PDGF-R (platelet derived growth factor receptor), EGF-R (epidermal growth factor receptor), VEGF-R (vascular endothelial growth factor), uPAR (urokinase plasminogen activator receptor), ACHR (acetylcholine receptor), IgE-R (immunoglobulin E receptor), estrogen receptor, thyroid hormone receptor, integrin receptors (β1, β2, β3, β4, β5, β6, α1, α2, α3, α4, α5, α6), MAC-1 (β2 and cd11b), αVβ33, opioid receptors (mu and kappa), FC receptors, serotonin receptors (5-HT, 5-HT6, 5-HT7), β-adrenergic receptors, insulin receptor, leptin receptor, TNF receptor (tissue-necrosis factor), statin receptors, FAS receptor, BAFF receptor, FLT3 lignad receptor, GMCSF receptor, and fibronectin receptor.

In one embodiment the activatable element is a cytokine receptor. Cytokines are a family of soluble mediators of cell-to-cell communication that includes interleukins, interferons, and colony-stimulating factors. The characteristic features of cytokines lie in their pleiotropy and functional redundancy. Most of the cytokine receptors that constitute distinct superfamilies do not possess intrinsic protein tyrosine kinase domains, yet receptor stimulation usually invokes rapid tyrosine phosphorylation of intracellular proteins, including the receptors themselves. Many members of the cytokine receptor superfamily activate the Jak protein tyrosine kinase family, with resultant phosphorylation of the STAT family of transcription factors. IL-2, IL-4, IL-7 and Interferon γ have all been shown to activate Jak kinases (Frank et al. Proc. Natl. Acad. Sci. USA 92: 7779-7783, 1995); Scharfe et al. Blood 86:2077-2085, 1995); (Bacon et al. Proc. Natl. Acad. Sci. USA 92: 7307-7311, 1995); and (Sakatsume et al. J. Biol. Chem. 270: 17528-17534, 1995). Events downstream of Jak phosphorylation have also been elucidated. For example, exposure of T lymphocytes to IL-2 has been shown to lead to the phosphorylation of signal transducers and activators of transcription (STAT) proteins STAT1α, STAT1β, and STAT3, as well as of two STAT-related proteins, p94 and p95. The STAT proteins translocate to the nucleus and bind to a specific DNA sequence, thus suggesting a mechanism by which IL-2 may activate specific genes involved in immune cell function (Frank et al. supra). Jak3 is associated with the gamma chain of the IL-2, IL-4, and IL-7 cytokine receptors (Fujii et al. Proc. Natl. Acad. Sci. 92: 5482-5486, 1995) and (Musso et al. J. Exp. Med. 181: 1425-1431, 1995). The Jak kinases have been shown to be activated by numerous ligands that signal via cytokine receptors such as, growth hormone, erythropoietin and IL-6 (Kishimoto Stem cells Suppl. 12: 37-44, 1994). Preferred activatable elements are selected from the group of proteins including, but not limited to, Stat1, Stat3, Stat5, Stat6, Lck, Lyn, Zap70, Syk, PLCγ2, p38, PI3K, S6, Akt, Erk, CREB, andNF-KB.

In one embodiment, the activatable element is a member of the nerve growth factor receptor superfamily, such as the tumor necrosis factor alpha receptor. Tumor necrosis factor α (TNF-α or TNF-alpha) is a pleiotropic cytokine that is primarily produced by activated macrophages and lymphocytes but is also expressed in endothelial cells and other cell types. TNF-alpha is a major mediator of inflammatory, immunological, and pathophysiological reactions. (Grell, M., et al., Cell, 83:793-802, 1995). Two distinct forms of TNF exist, a 26 kDa membrane expressed form and the soluble 17 kDa cytokine which is derived from proteolytic cleavage of the 26 kDa form. The soluble TNF polypeptide is 157 amino acids long and is the primary biologically active molecule. TNF-alpha exerts its biological effects through interaction with high-affinity cell surface receptors. Two distinct membrane TNF-alpha receptors have been cloned and characterized. These are a 55 kDa species, designated p55 TNF-R and a 75 kDa species designated p75 TNF-R (Corcoran. A. E., et al., Eur. J. Biochem., 223: 831-840, 1994). The two TNF receptors exhibit 28% similarity at the amino acid level. This is confined to the extracellular domain and consists of four repeating cysteine-rich motifs, each of approximately 40 amino acids. Each motif contains four to six cysteines in conserved positions. Dayhoff analysis shows the greatest intersubunit similarity among the first three repeats in each receptor. This characteristic structure is shared with a number of other receptors and cell surface molecules, which comprise the TNF-R/nerve growth factor receptor superfamily (Corcoran. A. E., et al., Eur. J. Biochem., 223: 831-840, 1994).

In one embodiment, the activatable element is a receptor tyrosine kinase. The receptor tyrosine kinases can be divided into subgroups on the basis of structural similarities in their extracellular domains and the organization of the tyrosine kinase catalytic region in their cytoplasmic domains. Sub-groups I (epidermal growth factor (EGF) receptor-like), II (insulin receptor-like) and the EPH/ECK family contain cysteine-rich sequences (Hirai et al., (1987) Science 238:1717-1720 and Lindberg and Hunter, (1990) Mol. Cell. Biol. 10:6316-6324). The functional domains of the kinase region of these three classes of receptor tyrosine kinases are encoded as a contiguous sequence (Hanks et al. (1988) Science 241:42-52). Subgroups III (platelet-derived growth factor (PDGF) receptor-like) and IV (the fibro-blast growth factor (FGF) receptors) are characterized as having immunoglobulin (Ig)-like folds in their extracellular domains, as well as having their kinase domains divided in two parts by a variable stretch of unrelated amino acids (Yanden and Ullrich (1988), Ann. Rev. Biochem, 57,443-478; and Hanks et al. (1988) supra).

The family with the largest number of known members is the Eph family (with the first member of the family originally isolated from an erythropoietin producing hepatocellular carcinoma cell line). Since the description of the prototype, the Eph receptor (Hirai et al. (1987) Science 238:1717-1720), sequences have been reported for at least ten members of this family, not counting apparently orthologous receptors found in more than one species. Additional partial sequences, and the rate at which new members are still being reported, suggest the family is even larger (Maisonpierre et al. (1993) Oncogene 8:3277-3288; Andres et al. (1994) Oncogene 9:1461-1467; Henkemeyer et al. (1994) Oncogene 9:1001-1014; Ruiz et al. (1994) Mech. Dev. 46:87-100; Xu et al. (1994) Development 120:287-299; Zhou et al. (1994) J. Neurosci. Res. 37:129-143; and references in Tuzi and Gullick (1994) Br. J. Cancer 69:417-421). Remarkably, despite the large number of members in the Eph family, all of these molecules were identified as orphan receptors without known ligands.

As used herein, the terms "Eph receptor" or "Eph-type receptor" refer to a class of receptor tyrosine kinases, comprising at least eleven paralogous genes, though many more orthologs exist within this class, e.g. homologs from different species. Eph receptors, in general, are a discrete group of receptors related by homology and easily recognizable, e.g., they are typically characterized by an extracellular domain containing a characteristic spacing of cysteine residues near the N-terminus and two fibronectin type III repeats (Hirai et al. (1987) Science 238:1717-1720; Lindberg et al. (1990) Mol. Cell Biol. 10:6316-6324; Chan et al. (1991) Oncogene 6:1057-1061; Maisonpierre et al. (1993) Oncogene 8:3277-3288; Andres et al. (1994) Oncogene 9:1461-1467; Henkemeyer et al. (1994) Oncogene 9:1001-1014; Ruiz et al. (1994) Mech. Dev. 46:87-100; Xu et al. (1994) Development 120:287-299; Zhou et al. (1994) J. Neurosci. Res. 37:129-143; and references in Tuzi and Gullick (1994) Br. J. Cancer 69:417-421). Exemplary Eph receptors include the eph, elk, eck, sek, mek4, hek, hek2, eek, erk, tyro1, tyro4, tyro5, tyro6, tyrol11, cek4, cek5, cek6, cek7, cek8, cek9, cek10, bsk, rtk1, rtk2, rtk3, myk1, myk2, ehk1, ehk2, pagliaccio, htk, erk and nuk receptors.

In another embodiment the receptor element is a member of the hematopoietin receptor superfamily. Hematopoietin receptor superfamily is used herein to define single-pass transmembrane receptors, with a three-domain architecture: an extracellular domain that binds the activating ligand, a short transmembrane segment, and a domain residing in the cytoplasm. The extracellular domains of these receptors have low but significant homology within their extracellular ligand-binding domain comprising about 200-210 amino acids. The homologous region is characterized by four cysteine residues located in the N-terminal half of the region, and a Trp-Ser-X-Trp-Ser (WSXWS) motif located just outside the membrane-spanning domain. Further structural and functional details of these receptors are provided by Cosman, D. et al., (1990). The receptors of IL-1, IL-2, IL-3, IL-4, IL-5, IL-6, IL-7, prolactin, placental lactogen, growth hormone GM-CSF, G-CSF, M-CSF and erythropoietin have, for example, been identified as members of this receptor family.

In a further embodiment, the receptor element is an integrin other than Leukocyte Function Antigen-1 (LFA-1). Members of the integrin family of receptors function as heterodimers, composed of various α and β subunits, and mediate interactions between a cell's cytoskeleton and the extracellular matrix. (Reviewed in, Giancotti and Ruoslahti, Science 285, 13 Aug. 1999). Different combinations of the α and β subunits give rise to a wide range of ligand specificities, which may be increased further by the presence of cell-type-specific factors. Integrin clustering is know to activate a number of intracellular signals, such as RAS, MAP kinase, and phosphotidylinosital-3-kinase. In one embodiment the receptor element is a heterodimer (other than LFA-1) composed of a β integrin and an α integrin chosen from the following integrins; β1, β2, β3, β4, β5, β6, α1, α2, α3, α4, α5, and α6, or is MAC-1 (β2 and cd11b), or aVβ3.

In one embodiment the activatable element is an intracellular adhesion molecule (ICAM). ICAMs -1, -2, and -3 are cellular adhesion molecules belonging to the immunogloblin superfamily. Each of these receptors has a single membrane-spanning domain and all bind to β2 integrins via extracellular binding domains similar in structure to Ig-loops (Signal Transduction, Gomperts, et al., eds, Academic or government Press Publishers, 2002, Chapter 14, pp 318-319).

In another embodiment the activatable elements cluster for signaling by contact with other surface molecules. In contrast to the receptors discussed above, these elements cluster for signaling by contact with other surface molecules, and generally use molecules presented on the surface of a second cell as ligands. Receptors of this class are important in cell-cell interactions, such as mediating cell-to-cell adhesion and immunorecognition. Examples of such receptor elements are CD3 (T cell receptor complex), BCR (B cell receptor complex), CD4, CD28, CD80, CD86, CD54, CD102, CD50 and ICAMs 1, 2 and 3.

In another embodiment the receptor element is a T cell receptor complex (TCR). TCRs occur as either of two distinct heterodimers, αβ, or γζ both of which are expressed with the non-polymorphic CD3 polypeptides γ, Σ, ε, ζ. The CD3 polypeptides, especially ζ and its variants, are critical for intracellular signaling. The αβ TCR heterodimer expressing cells predominate in most lymphoid compartments and are responsible for the classical helper or cytotoxic T cell responses. In most cases, the αβ TCR ligand is a peptide antigen bound to a class I or a class II MHC molecule (Fundamental Immunology, fourth edition, W. E. Paul, ed., Lippincott-Raven Publishers, 1999, Chapter 10, pp 341-367).

In another embodiment, the activatable element is a member of the large family of G-protein-coupled receptors. It has been reported that a G-protein-coupled receptors are capable of clustering. (Kroeger, et al., J Biol Chem 276:16, 12736-12743, Apr. 20, 2001; Bai, et al., J Biol Chem 273:36, 23605-23610, Sep. 4, 1998; Rocheville, et al., J Biol Chem 275 (11), 7862-7869, Mar. 17, 2000). As used herein G-protein-coupled receptor, and grammatical equivalents thereof, refers to the family of receptors that bind to heterotrimeric "G proteins." Many different G proteins are known to interact with receptors. G protein signaling systems include three components: the receptor itself, a GTP-binding protein (G protein), and an intracellular target protein. The cell membrane acts as a switchboard. Messages arriving through different receptors can produce a single effect if the receptors act on the same type of G protein. On the other hand, signals activating a single receptor can produce more than one effect if the receptor acts on different kinds of G proteins, or if the G proteins can act on different effectors.

In their resting state, the G proteins, which consist of alpha (α), beta (β) and gamma (γ) subunits, are complexed with the nucleotide guanosine diphosphate (GDP) and are in contact with receptors. When a hormone or other first messenger binds to a receptor, the receptor changes conformation and this alters its interaction with the G protein. This spurs a subunit to release GDP, and the more abundant nucleotide guanosine triphosphate (GTP), replaces it, activating the G protein. The G protein then dissociates to separate the α subunit from the still complexed beta and gamma subunits. Either the Gα subunit, or the Gβγ complex, depending on the pathway, interacts with an effector. The effector (which is often an enzyme) in turn converts an inactive precursor molecule into an active "second messenger," which may diffuse through the cytoplasm, triggering a metabolic cascade. After a few seconds, the Gα converts the GTP to GDP, thereby inactivating itself. The inactivated Gα may then reassociate with the Gβγ complex.

Hundreds, if not thousands, of receptors convey messages through heterotrimeric G proteins, of which at least 17 distinct forms have been isolated. Although the greatest variability has been seen in a subunit, several different β and γ structures have been reported. There are, additionally, many different G protein-dependent effectors. Most G protein-coupled receptors are comprised of a single protein chain that passses through the plasma membrane seven times. Such receptors are often referred to as seven-transmembrane receptors (STRs). More than a hundred different STRs have been found, including many distinct receptors that bind the same ligand, and there are likely many more STRs awaiting discovery. In addition, STRs have been identified for which the natural ligands are unknown; these receptors are termed "orphan" G protein-coupled receptors, as described above. Examples include receptors cloned by Neote et al. (1993) Cell 72, 415; Kouba et al. FEBS Lett. (1993)321, 173; and Birkenbach et al. (1993) J. Virol. 67, 2209.

Known ligands for G protein coupled receptors include: purines and nucleotides, such as adenosine, cAMP, ATP, UTP, ADP, melatonin and the like; biogenic amines (and related natural ligands), such as 5-hydroxytryptamine, acetylcholine, dopamine, adrenaline, histamine, noradrenaline, tyramine/octopamine and other related compounds; peptides such as adrenocorticotrophic hormone (acth), melanocyte stimulating hormone (msh), melanocortins, neurotensin (nt), bombesin and related peptides, endothelins, cholecystokinin, gastrin, neurokinin b (nk3), invertebrate tachykinin-like peptides, substance k (nk2), substance p (nk1), neuropeptide y (npy), thyrotropin releasing-factor (trf), bradykinin, angiotensin ii, beta-endorphin, c5a anaphalatoxin, calcitonin, chemokines (also called intercrines), corticotrophic releasing factor (crf), dynorphin, endorphin, fmlp and other formylated peptides, follitropin (fsh), fungal mating pheromones, galanin, gastric inhibitory polypeptide receptor (gip), glucagon-like peptides (glps), glucagon, gonadotropin releasing hormone (gnrh), growth hormone releasing hormone(ghrh), insect diuretic hormone, interleukin-8, leutropin (1 h/hcg), met-enkephalin, opioid peptides, oxytocin, parathyroid hormone (pth) and pthrp, pituitary adenylyl cyclase activating peptide (pacap), secretin, somatostatin, thrombin, thyrotropin (tsh), vasoactive intestinal peptide (vip), vasopressin, vasotocin; eicosanoids such as ip-prostacyclin, pg-prostaglandins, tx-thromboxanes; retinal based compounds such as vertebrate 11-cis retinal, invertebrate 11-cis retinal and other related compounds; lipids and lipid-based compounds such as cannabinoids, anandamide, lysophosphatidic acid, platelet activating factor, leukotrienes and the like; excitatory amino acids and ions such as calcium ions and glutamate.

Examples of G protein coupled receptors include, but are not limited to: α1-adrenergic receptor, α1B-adrenergic receptor, α2-adrenergic receptor, α2B-adrenergic receptor, β1-adrenergic receptor, β2-adrenergic receptor, β3-adrenergic receptor, m1 acetylcholine receptor (AChR), m2 AChR, m3 AChR, m4 AChR, m5 AChR, D1 dopamine receptor, D2 dopamine receptor, D3 dopamine receptor, D4 dopamine receptor, D5 dopamine receptor, A1 adenosine receptor, A2a adenosine receptor, A2b adenosine receptor, A3 adenosine receptor, 5-HT1a receptor, 5-HT1b receptor, 5HT1-like receptor, 5-HT1d receptor, 5HT1d-like receptor, 5HT1d beta receptor, substance K (neurokinin A) receptor, fMLP receptor (FPR), fMLP-like receptor (FPRL-1), angiotensin II type 1 receptor, endothelin ETA receptor, endothelin ETB receptor, thrombin receptor, growth hormone-releasing hormone (GHRH) receptor, vasoactive intestinal peptide receptor, oxytocin receptor, somatostatin SSTR1 and SSTR2, SSTR3, cannabinoid receptor, follicle stimulating hormone (FSH) receptor, leutropin (LH/HCG) receptor, thyroid stimulating hormone (TSH) receptor, thromboxane A2 receptor, platelet-activating factor (PAF) receptor, C5a anaphylatoxin receptor, CXCR1 (IL-8 receptor A), CXCR2 (IL-8 receptor B), Delta Opioid receptor, Kappa Opioid receptor, mip-1alpha/RANTES receptor (CRR1), Rhodopsin, Red opsin, Green opsin, Blue opsin, metabotropic glutamate mGluR1-6, histamine H2 receptor, ATP receptor, neuropeptide Y receptor, amyloid protein precursor receptor, insulin-like growth factor II receptor, bradykinin receptor, gonadotropin-releasing hormone receptor, cholecystokinin receptor, melanocyte stimulating hormone receptor, antidiuretic hormone receptor, glucagon receptor, and adrenocorticotropic hormone II receptor. In addition, there are at least five receptors (CC and CXC receptors) involved in HIV viral attachment to cells. The two major co-receptors for HIV are CXCR4, (fusin receptor, LESTR, SDF-1 a receptor) and CCR5 (m-trophic). Additional examples of receptors include, but are not limited to, the following human receptors: melatonin receptor 1a, galanin receptor 1, neurotensin receptor, adenosine receptor 2a, somatostatin receptor 2 and corticotropin releasing factor receptor 1. Other G protein coupled receptors (GPCRs) are known in the art.

In one embodiment, Lnk is a protein to be measured. Hematopoietic stem cells (HSCs) give rise to variety of hematopoietic cells via pluripotential progenitors. Lineage-committed progenitors are responsible for blood production throughout adult life. Amplification of HSCs or progenitors represents a potentially powerful approach to the treatment of various blood disorders. Animal model studies demonstrated that Lnk acts as a broad inhibitor of signaling pathways in hematopoietic lineages. Lnk is an adaptor protein which belongs to a family of proteins sharing several structural motifs, including a Src homology 2 (SH2) domain which binds phospho-tyrosines in various signal-transducing proteins. The SH2 domain is essential for Lnk-mediated negative regulation of several cytokine receptors (i.e. Mpl, EpoR, c-Kit, I1-3R and IL7R). Therefore, inhibition of the binding of Lnk to cytokine receptors might lead to enhanced downstream signaling of the receptor and thereby to improved hematopoiesis in response to exposure to cytokines (i.e. erythropoietin in anemic patients), (Gueller et al, Adaptor protein Lnk associates with Y568 in c-Kit. 1: Biochem J. 2008 Jun 30.). It has been shown that overexpression of Lnk in Ba/F3-MPLW515L cells inhibits cytokine-independent growth, while suppression of Lnk in UT7-MPLW515L cells enhances proliferation. Lnk blocks the activation of Jak2, Stat3, Erk, and Akt in these cells (Gery et al., Adaptor protein Lnk negatively regulates the mutant MPL, MPLW515L associated with myeloproliferative neoplasms, Blood, 1 November 2007, Vol. 110, No. 9, pp. 3360-3364.).

In one embodiment, the activatable elements are intracellular receptors capable of clustering. Elements of this class are not membrane-bound. Instead, they are free to diffuse through the intracellular matrix where they bind soluble ligands prior to clustering and signal transduction. In contrast to the previously described elements, many members of this class are capable of binding DNA after clustering to directly effect changes in RNA transcription.

In another embodiment the intracellular receptors capable of clustering are perioxisome proliferator-activated receptors (PPAR). PPARs are soluble receptors responsive to lipophillic compounds, and induce various genes involved in fatty acid metabolism. The three PPAR subtypes, PPAR α, β, and γ have been shown to bind to DNA after ligand binding and heterodimerization with retinoid X receptor. (Summanasekera, et al., J Biol Chem, M211261200, Dec. 13, 2002.)

In another embodiment the activatable element is a nucleic acid. Activation and deactivation of nucleic acids can occur in numerous ways including, but not limited to, cleavage of an inactivating leader sequence as well as covalent or non-covalent modifications that induce structural or functional changes. For example, many catalytic RNAs, e.g. hammerhead ribozymes, can be designed to have an inactivating leader sequence that deactivates the catalytic activity of the ribozyme until cleavage occurs. An example of a covalent modification is methylation of DNA. Deactivation by methylation has been shown to be a factor in the silencing of certain genes, e.g. STAT regulating SOCS genes in lymphomas (Chim et al. (2004), Leukemia 18: 356-358).

In another embodiment the activatable element is a small molecule, carbohydrate, lipid or other naturally occurring or synthetic compound capable of having an activated isoform. In addition, as pointed out above, activation of these elements need not include switching from one form to another, but can be detected as the presence or absence of the compound. For example, activation of cAMP (cyclic adenosine mono-phosphate) can be detected as the presence of cAMP rather than the conversion from non-cyclic AMP to cyclic AMP.

Examples of proteins that may include activatable elements include, but are not limited to kinases, phosphatases, lipid signaling molecules, adaptor/scaffold proteins, cytokines, cytokine regulators, ubiquitination enzymes, adhesion molecules, cytoskeletal/contractile proteins, heterotrimeric G proteins, small molecular weight GTPases, guanine nucleotide exchange factors, GTPase activating proteins, caspases, proteins involved in apoptosis, cell cycle regulators, molecular chaperones, metabolic enzymes, vesicular transport proteins, hydroxylases, isomerases, deacetylases, methylases, demethylases, tumor suppressor genes, proteases, ion channels, molecular transporters, transcription factors/DNA binding factors, regulators of transcription, and regulators of translation. Examples of activatable elements, activation states and methods of determining the activation level of activatable elements are described in US Publication Number 20060073474 entitled "Methods and compositions for detecting the activation state of multiple proteins in single cells" and US Publication Number 20050112700 entitled "Methods and compositions for risk stratification" the content of which are incorporate here by reference. See also U.S.S.Nos. 61/048,886; 61/048,920;and Shulzet al., Current Protocols in Immunology 2007, 78:8.17.1-20.

In some embodiments, the protein is selected from the group consisting of HER receptors, PDGF receptors, Kit receptor, FGF receptors, Eph receptors, Trk receptors, IGF receptors, Insulin receptor, Met receptor, Ret, VEGF receptors, TIE1, TIE2, FAK, Jak1, Jak2, Jak3, Tyk2, Src, Lyn, Fyn, Lck, Fgr, Yes, Csk, Abl, Btk, ZAP70, Syk, IRAKs, cRaf, ARaf, BRAF, Mos, Lim kinase, ILK, Tp1, ALK, TNFβ receptors, BMP receptors, MEKKs, ASK, MLKs, DLK, PAKs, Mek 1, Mek 2, MKK3/6, MKK4/7, ASK1,Cot, NIK, Bub, Myt 1, Wee1, Casein kinases, PDK1, SGK1, SGK2, SGK3, Akt1, Akt2, Akt3, p90Rsks, p70S6 Kinase, Prks, PKCs, PKAs, ROCK 1, ROCK 2, Auroras, CaMKs, MNKs, AMPKs, MELK, MARKs, Chk1, Chk2, LKB-1, MAPKAPKs, Pim1, Pim2, Pim3, IKKs, Cdks, Jnks, Erks, IKKs, GSK3α, GSK3β, Cdks, CLKs, PKR, PI3-Kinase class 1, class 2, class 3, mTor, SAPK/JNK1,2,3, p38s, PKR, DNA-PK, ATM, ATR, Receptor protein tyrosine phosphatases (RPTPs), LAR phosphatase, CD45, Non receptor tyrosine phosphatases (NPRTPs), SHPs, MAP kinase phosphatases (MKPs), Dual Specificity phosphatases (DUSPs), CDC25 phosphatases, Low molecular weight tyrosine phosphatase, Eyes absent (EYA) tyrosine phosphatases, Slingshot phosphatases (SSH), serine phosphatases, PP2A, PP2B, PP2C, PP1, PP5, inositol phosphatases, PTEN, SHIPs, myotubularins, phosphoinositide kinases, phopsholipases, prostaglandin synthases, 5-lipoxygenase, sphingosine kinases, sphingomyelinases, adaptor/scaffold proteins, Shc, Grb2, BLNK, LAT, B cell adaptor for PI3-kinase (BCAP), SLAP, Dok, KSR, MyD88, Crk, CrkL, GAD, Nck, Grb2 associated binder (GAB), Fas associated death domain (FADD), TRADD, TRAF2, RIP, T-Cell leukemia family, IL-2, IL-4, IL-8, IL-6, interferon γ, interferon α, suppressors of cytokine signaling (SOCs), Cbl, SCF ubiquitination ligase complex, APC/C, adhesion molecules, integrins, Immunoglobulin-like adhesion molecules, selectins, cadherins, catenins, focal adhesion kinase, p130CAS, fodrin, actin, paxillin, myosin, myosin binding proteins, tubulin, eg5/KSP, CENPs, β-adrenergic receptors, muscarinic receptors, adenylyl cyclase receptors, small molecular weight GTPases, H-Ras, K-Ras, N-Ras, Ran, Rac, Rho, Cdc42, Arfs, RABs, RHEB, Vav, Tiam, Sos, Dbl, PRK, TSC1,2, Ras-GAP, Arf-GAPs, Rho-GAPs, caspases, Caspase 2, Caspase 3, Caspase 6, Caspase 7, Caspase 8, Caspase 9, Bcl-2, Mcl-1, Bcl-XL, Bcl-w, Bcl-B, A1, Bax, Bak, Bok, Bik, Bad, Bid, Bim, Bmf, Hrk, Noxa, Puma, IAPs, XIAP, Smac, Cdk4, Cdk 6, Cdk 2, Cdk1, Cdk 7, Cyclin D, Cyclin E, Cyclin A, Cyclin B, Rb, p16, p14Arf, p27KIP, p21CIP, molecular chaperones, Hsp90s, Hsp70, Hsp27, metabolic enzymes, Acetyl-CoA Carboxylase, ATP citrate lyase, nitric oxide synthase, caveolins, endosomal sorting complex required for transport (ESCRT) proteins, vesicular protein sorting (Vsps), hydroxylases, prolyl-hydroxylases PHD-1, 2 and 3, asparagine hydroxylase FIH transferases, Pin1 prolyl isomerase, topoisomerases, deacetylases, Histone deacetylases, sirtuins, histone acetylases, CBP/P300 family, MYST family, ATF2, DNA methyl transferases, Histone H3K4 demethylases, H3K27, JHDM2A, UTX, VHL, WT-1, p53, Hdm, PTEN, ubiquitin proteases, urokinase-type plasminogen activator (uPA) and uPA receptor (uPAR) system, cathepsins, metalloproteinases, esterases, hydrolases, separase, potassium channels, sodium channels, , multi-drug resistance proteins, P-Gycoprotein, nucleoside transporters, Ets, Elk, SMADs, Rel-A (p65-NFKB), CREB, NFAT, ATF-2, AFT, Myc, Fos, Sp1, Egr-1, T-bet, β-catenin, HIFs, FOXOs, E2Fs, SRFs, TCFs, Egr-1, β-catenin, FOXO STAT1, STAT 3, STAT 4, STAT 5, STAT 6, p53, WT-1, HMGA, pS6, 4EPB-1, eIF4E-binding protein, RNA polymerase, initiation factors, elongation factors.

In some embodiments of the invention, the methods described herein are employed to determine the activation level of an activatable element, e.g., in a cellular pathway. Methods and compositions are provided for the classification of a cell according to the activation level of an activatable element in a cellular pathway. The cell can be a hematopoietic cell. Examples of hematopoietic cells include but are not limited to pluripotent hematopoietic stem cells, granulocyte lineage progenitor or derived cells, monocyte lineage progenitor or derived cells, macrophage lineage progenitor or derived cells, megakaryocyte lineage progenitor or derived cells and erythroid lineage progenitor or derived cells.

### Signaling Pathways

In some embodiments, the methods of the invention are employed to determine the status of an activatable element in a signaling pathway. In some embodiments, a cell is classified, as described herein, according to the activation level of one or more activatable elements in one or more signaling pathways. Signaling pathways and their members have been described. See (Hunter T. Cell Jan. 7, 2000;100(1): 13-27). Exemplary signaling pathways include the following pathways and their members: The MAP kinase pathway including Ras, Raf, MEK, ERK and elk; the PI3K/Akt pathway including PI-3-kinase (PI3K), PDK1, Akt and Bad; the NF-κB pathway including IKKs, IkB; the Wnt pathway including frizzled receptors, beta-catenin, APC and other co-factors and TCF; the T cell receptor (TCR) pathway including Lck and Zap70; and the B cell receptor (BCR) pathway including Lyn, Syk, and PLCγ2 (see Cell Signaling Technology, Inc. 2002 Catalog pages 231-279 and Hunter T., supra.). In some embodiments of the invention, the correlated activatable elements being assayed (or the signaling proteins being examined) are members of the MAP kinase, Akt, NFkB, WNT, RAS/RAF/MEK/ERK, JNK/SAPK, p38 MAPK, Src Family Kinases, JAK/STAT, TCR, BCR, and/or PKC signaling pathways. The functional state of these and other pathways is representative of an ability to carry a signal from one step to another in the relevant transduction cascade. Signal transduction proceeds at a given step when the activatable element of that given step in the process within the cascade is modulated.

In some embodiments, the methods of the invention are employed to determine the status of a signaling protein and/or activatable element in a signaling pathway known in the art including those described herein. Exemplary types of signaling proteins within the scope of the present invention include, but are not limited to kinases, kinase substrates (i.e. phosphorylated substrates), phosphatases, phosphatase substrates, binding proteins (such as 14-3-3), receptor ligands and receptors (cell surface receptor tyrosine kinases and nuclear receptors)). Kinases and protein binding domains, for example, have been well described (see, e.g., Cell Signaling Technology, Inc., 2002 Catalogue "The Human Protein Kinases" and "Protein Interaction Domains" pgs. 254-279).

*Nuclear Factor-kappaB (1VF-κB) Pathway:* Nuclear factor-kappaB (NF-kappaB) transcription factors and the signaling pathways that activate them are central coordinators of innate and adaptive immune responses. In mammalian cells, there are five NF-κB family members, RelA (p65), RelB, c-Rel, p50/p105 (NF-κB1) and p52/p100 (NF-κB2) and different NF-κB complexes are formed from their homo and heterodimers. In most cell types, NF-κB complexes are retained in the cytoplasm by a family of inhibitory proteins known as inhibitors of NF-κB (IκBs). Activation of NF-κB typically involves the phosphorylation of IκB by the IκB kinase (IKK) complex, which results in IκB ubiquitination with subsequent degradation. This releases NF-κB and allows it to translocate freely to the nucleus. The genes regulated by NF-κB include those controlling programmed cell death, cell adhesion, proliferation, the innate- and adaptive-immune responses, inflammation, the cellular-stress response and tissue remodeling. However, the expression of these genes is tightly coordinated with the activity of many other signaling and transcription-factor pathways. Therefore, the outcome of NF-κB activation depends on the nature and the cellular context of its induction. For example, it has become apparent that NF-κB activity can be regulated by both oncogenes and tumor suppressors, resulting in either stimulation or inhibition of apoptosis and proliferation. See Perkins, N. Integrating cell-signaling pathways with NF-κB and IKK function. Reviews: Molecular Cell Biology. Jan, 2007; 8(1): 49-62, hereby fully incorporated by reference in its entirety for all purposes. Hayden, M. Signaling to NF-κB. Genes & Development. 2004; 18: 2195-2224, hereby fully incorporated by reference in its entirety for all purposes. Perkins, N. Good Cop, Bad Cop: The Different Faces of NF-κB. Cell Death and Differentiation. 2006; 13: 759-772, hereby fully incorporated by reference in its entirety for all purposes.

*Phosphatidylinositol 3-kinase (PI3-K)lAKT Pathway:* PI3-Ks are activated by a wide range of cell surface receptors to generate the lipid second messengers phosphatidylinositol 3,4-biphosphate (PIP₂) and phosphatidylinositol 3,4,5-trisphosphate (PIP₃). Examples of receptor tyrosine kinases include but are not limited to FLT3 LIGAND, EGFR, IGF-1R, HER2/neu, VEGFR, and PDGFR. The lipid second messengers generated by PI3Ks regulate a diverse array of cellular functions. The specific binding of PI3,4P₂ and PI3,4,5P₃ to target proteins is mediated through the pleckstrin homology (PH) domain present in these target proteins. One key downstream effector of PI3-K is Akt, a serine/threonine kinase, which is activated when its PH domain interacts with PI3,4P₂ and PI3,4,5P₃ resulting in recruitment of Akt to the plasma membrane. Once there, in order to be fully activated, Akt is phosphorylated at threonine 308 by 3-phosphoinositide-dependent protein kinase-1 (PDK-1) and at serine 473 by several PDK2 kinases. Akt then acts downstream of PI3K to regulate the phosphorylation of a number of substrates, including but not limited to forkhead box O transcription factors, Bad, GSK-3β, I-κB, mTOR, MDM-2, and S6 ribosomal subunit. These phosphorylation events in turn mediate cell survival, cell proliferation, membrane trafficking, glucose homeostasis, metabolism and cell motility. Deregulation of the PI3K pathway occurs by activating mutations in growth factor receptors, activating mutations in a PI3-K gene (e.g. PIK3CA), loss of function mutations in a lipid phosphatase (e.g. PTEN), up-regulation of Akt, or the impairment of the tuberous sclerosis complex (TSC1/2). All these events are linked to increased survival and proliferation. See Vivanco, I. The Phosphatidylinositol 3-Kinase-AKT Pathway in Human Cancer. Nature Reviews: Cancer. Jul, 2002; 2: 489-501 and Shaw, R. Ras, PI(3)K and mTOR signaling controls tumor cell growth. Nature. May, 2006; 441: 424-430, Marone et al., Biochimica et Biophysica Acta, 2008; 1784, p159- 185 hereby fully incorporated by reference in their entirety for all purposes.

*Protein Kinase C (PKC) Signaling:* The PKC family of serine/threonine kinases mediate signaling pathways following activation of receptor tyrosine kinases, G-protein coupled receptors and cytoplasmic tyrosine kinases. Activation of PKC family members is associated with cell proliferation, differentiation, survival, immune function, invasion, migration and angiogenesis. Disruption of PKC signaling has been implicated in tumorigenesis and drug resistance. PKC isoforms have distinct and overlapping roles in cellular functions. PKC was originally identified as a phospholipid and calcium-dependent protein kinase. The mammalian PKC superfamily consists of 13 different isoforms that are divided into four subgroups on the basis of their structural differences and related cofactor requirements cPKC (classical PKC) isoforms (α, *β*I, *β*II and y), which respond both to Ca2+ and DAG (diacylglycerol), nPKC (novel PKC) isoforms (*δ*, *ε, θ* and *η*), which are insensitive to Ca2+, but dependent on DAG, atypical PKCs (aPKCs, *i*/λ, ξ, which are responsive to neither co-factor, but may be activated by other lipids and through protein-protein interactions, and the related PKN (protein kinase N) family (e.g. PKN1, PKN2 and PKN3), members of which are subject to regulation by small GTPases. Consistent with their different biological functions, PKC isoforms differ in their structure, tissue distribution, subcellular localization, mode of activation and substrate specificity. Before maximal activation of its kinase, PKC requires a priming phosphorylation which is provided constitutively by phosphoinositide-dependent kinase 1 (PDK-1). The phospholipid DAG has a central role in the activation of PKC by causing an increase in the affinity of classical PKCs for cell membranes accompanied by PKC activation and the release of an inhibitory substrate (a pseudo-substrate) to which the inactive enzyme binds. Activated PKC then phosphorylates and activates a range of kinases. The downstream events following PKC activation are poorly understood, although the MEK-ERK (mitogen activated protein kinase kinase-extracellular signal-regulated kinase) pathway is thought to have an important role. There is also evidence to support the involvement of PKC in the PI3K-Akt pathway. PKC isoforms probably form part of the multi-protein complexes that facilitate cellular signal transduction. Many reports describe dysregulation of several family members.

*Mitogen Activated Protein (MAP) Kinase Pathways:* MAP kinases transduce signals that are involved in a multitude of cellular pathways and functions in response to a variety of ligands and cell stimuli. (Lawrence et al., Cell Research (2008) 18: 436-442). Signaling by MAPKs affects specific events such as the activity or localization of individual proteins, transcription of genes, and increased cell cycle entry, and promotes changes that orchestrate complex processes such as embryogenesis and differentiation. Aberrant or inappropriate functions of MAPKs have now been identified in diseases ranging from cancer to inflammatory disease to obesity and diabetes. MAPKs are activated by protein kinase cascades consisting of three or more protein kinases in series: MAPK kinase kinases (MAP3Ks) activate MAPK kinases (MAP2Ks) by dual phosphorylation on S/T residues; MAP2Ks then activate MAPKs by dual phosphorylation on Y and T residues MAPKs then phosphorylate target substrates on select S/T residues typically followed by a proline residue. In the ERK1/2 cascade the MAP3K is usually a member of the Raf family. Many diverse MAP3Ks reside upstream of the p38 and the c-Jun N-terminal kinase/stress-activated protein kinase (JNK/ SAPK) MAPK groups, which have generally been associated with responses to cellular stress. Downstream of the activating stimuli, the kinase cascades may themselves be stimulated by combinations of small G proteins, MAP4Ks, scaffolds, or oligomerization of the MAP3K in a pathway. In the ERK1/2 pathway, Ras family members usually bind to Raf proteins leading to their activation as well as to the subsequent activation of other downstream members of the pathway.

*Ras*/*RAF*/*MEK*/*ERK Pathway:* Classic activation of the RAS/Raf/MAPK cascade occurs following ligand binding to a receptor tyrosine kinase at the cell surface, but a vast array of other receptors have the ability to activate the cascade as well, such as integrins, serpentine receptors, heterotrimeric G-proteins, and cytokine receptors. Although conceptually linear, considerable cross talk occurs between the Ras/Raf/MAPK/Erk kinase (MEK)/Erk MAPK pathway and other MAPK pathways as well as many other signaling cascades. The pivotal role of the Ras/Raf/MEK/Erk MAPK pathway in multiple cellular functions underlies the importance of the cascade in oncogenesis and growth of transformed cells. As such, the MAPK pathway has been a focus of intense investigation for therapeutic targeting. Many receptor tyrosine kinases are capable of initiating MAPK signaling. They do so after activating phosphorylation events within their cytoplasmic domains provide docking sites for src-homology 2 (SH2) domain-containing signaling molecules. Of these, adaptor proteins such as Grb2 recruit guanine nucleotide exchange factors such as SOS-1 or CDC25 to the cell membrane. The guanine nucleotide exchange factor is now capable of interacting with Ras proteins at the cell membrane to promote a conformational change and the exchange of GDP for GTP bound to Ras. Multiple Ras isoforms have been described, including K-Ras, N-Ras, and H-Ras. Termination of Ras activation occurs upon hydrolysis of RasGTP to RasGDP. Ras proteins have intrinsically low GTPase activity. Thus, the GTPase activity is stimulated by GTPase-activating proteins such as NF-1 GTPase-activating protein/neurofibromin and p120 GTPase activating protein thereby preventing prolonged Ras stimulated signaling. Ras activation is the first step in activation of the MAPK cascade. Following Ras activation, Raf (A-Raf, B-Raf, or Raf-1) is recruited to the cell membrane through binding to Ras and activated in a complex process involving phosphorylation and multiple cofactors that is not completely understood. Raf proteins directly activate MEK1 and MEK2 via phosphorylation of multiple serine residues. MEK1 and MEK2 are themselves tyrosine and threonine/ serine dual-specificity kinases that subsequently phosphorylate threonine and tyrosine residues in Erk1 and Erk2 resulting in activation. Although MEK1/2 have no known targets besides Erk proteins, Erk has multiple targets including Elk-1, c-Ets1, c-Ets2, p90RSK1, MNK1, MNK2, and TOB .The cellular functions of Erk are diverse and include regulation of cell proliferation, survival, mitosis, and migration. McCubrey, J. Roles of the RaflMEKlERK pathway in cell growth, malignant transformation and drug resistance. Biochimica et Biophysica Acta. 2007; 1773: 1263-1284, hereby fully incorporated by reference in its entirety for all purposes, Friday and Adjei, Clinical Cancer Research (2008) 14, p342-346.

*c-Jun N-terminal kinase (JNK)*/*stress-activated protein kinase (SAPK) Pathway:* The c-Jun N-terminal kinases (JNKs) were initially described as a family of serine/threonine protein kinases, activated by a range of stress stimuli and able to phosphorylate the N-terminal transactivation domain of the c- Jun transcription factor. This phosphorylation enhances c-Jun dependent transcriptional events in mammalian cells. Further research has revealed three JNK genes (JNK1, JNK2 and JNK3) and their splice-forms as well as the range of external stimuli that lead to JNK activation. JNK1 and JNK2 are ubiquitous, whereas JNK3 is relatively restricted to brain. The predominant MAP2Ks upstream of JNK are MEK4 (MKK4) and MEK7 (MKK7). MAP3Ks with the capacity to activate JNK/SAPKs include MEKKs (MEKK1, -2, - 3 and -4), mixed lineage kinases (MLKs, including MLK1-3 and DLK), Tp12, ASKs, TAOs and TALK1. Knockout studies in several organisms indicate that different MAP3Ks predominate in JNK/SAPK activation in response to different upstream stimuli. The wiring may be comparable to, but perhaps even more complex than, MAP3K selection and control of the ERK1/2 pathway. JNK/SAPKs are activated in response to inflammatory cytokines; environmental stresses, such as heat shock, ionizing radiation, oxidant stress and DNA damage; DNA and protein synthesis inhibition; and growth factors. JNKs phosphorylate transcription factors c-Jun, ATF-2, p53, Elk-1, and nuclear factor of activated T cells (NFAT), which in turn regulate the expression of specific sets of genes to mediate cell proliferation, differentiation or apoptosis. JNK proteins are involved in cytokine production, the inflammatory response, stress-induced and developmentally programmed apoptosis, actin reorganization, cell transformation and metabolism. Raman, M. Differential regulation and properties of MAPKs. Oncogene. 2007; 26: 3100-3112, hereby fully incorporated by reference in its entirety for all purposes.

*p38 MAPK Pathway:* Several independent groups identified the p38 Map kinases, and four p38 family members have been described (α, β, γ, δ). Although the p38 isoforms share about 40% sequence identity with other MAPKs, they share only about 60% identity among themselves, suggesting highly diverse functions. p38 MAPKs respond to a wide range of extracellular cues particularly cellular stressors such as UV radiation, osmotic shock, hypoxia, pro-inflammatory cytokines and less often growth factors. Responding to osmotic shock might be viewed as one of the oldest functions of this pathway, because yeast p38 activates both short and long-term homeostatic mechanisms to osmotic stress. p38 is activated via dual phosphorylation on the TGY motif within its activation loop by its upstream protein kinases MEK3 and MEK6. MEK3/6 are activated by numerous MAP3Ks including MEKK1-4, TAOs, TAK and ASK. p38 MAPK is generally considered to be the most promising MAPK therapeutic target for rheumatoid arthritis as p38 MAPK isoforms have been implicated in the regulation of many of the processes, such as migration and accumulation of leucocytes, production of cytokines and pro-inflammatory mediators and angiogenesis, that promote disease pathogenesis. Further, the p38 MAPK pathway plays a role in cancer, heart and neurodegenerative diseases and may serve as promising therapeutic target. Cuenda, A. *p38 MAP-Kinases pathway regulation, function, and role in human diseases.* Biochimica et Biophysica Acta. 2007; 1773: 1358-1375; Thalhamer et al., Rheumatology 2008;47:409-414; Roux, P. ERK and p38 MAPK-Activated Protein Kinases: a Family of Protein Kinases with Diverse Biological Functions. Microbiology and Molecular Biology Reviews. Jun, 2004; 320-344 hereby fully incorporated by reference in its entirety for all purposes.

*Src Family Kinases:* Src is the most widely studied member of the largest family of nonreceptor protein tyrosine kinases, known as the Src family kinases (SFKs). Other SFK members include Lyn, Fyn, Lck, Hck, Fgr, Blk, Yrk, and Yes. The Src kinases can be grouped into two sub-categories, those that are ubiquitously expressed (Src, Fyn, and Yes), and those which are found primarily in hematopoietic cells (Lyn, Lck, Hck, Blk, Fgr). (Benati, D. Src Family Kinases as Potential Therapeutic Targets for Malignancies and Immunological Disorders. Current Medicinal Chemistry. 2008; 15: 1154-1165) SFKs are key messengers in many cellular pathways, including those involved in regulating proliferation, differentiation, survival, motility, and angiogenesis. The activity of SFKs is highly regulated intramolecularly by interactions between the SH2 and SH3 domains and intermolecularly by association with cytoplasmic molecules. This latter activation may be mediated by focal adhesion kinase (FAK) or its molecular partner Crk-associated substrate (CAS), which play a prominent role in integrin signaling, and by ligand activation of cell surface receptors, e.g. epidermal growth factor receptor (EGFR). These interactions disrupt intramolecular interactions within Src, leading to an open conformation that enables the protein to interact with potential substrates and downstream signaling molecules. Src can also be activated by dephosphorylation of tyrosine residue Y530. Maximal Src activation requires the autophosphorylation of tyrosine residue Y419 (in the human protein) present within the catalytic domain. Elevated Src activity may be caused by increased transcription or by deregulation due to overexpression of upstream growth factor receptors such as EGFR, HER2, platelet-derived growth factor receptor (PDGFR), fibroblast growth factor receptor (FGFR), vascular endothelial growth factor receptor, ephrins, integrin, or FAK. Alternatively, some human tumors show reduced expression of the negative Src regulator, Csk. Increased levels, increased activity, and genetic abnormalities of Src kinases have been implicated in both solid tumor development and leukemias. Ingley, E. Src family kinases: Regulation of their activities, levels and identification of new pathways. Biochimica et Biophysica Acta. 2008; 1784 56-65, hereby fully incorporated by reference in its entirety for all purposes. Benati and Baldari., Curr Med Chem. 2008;15(12):1154-65, Finn (2008) Ann Oncol. May 16, hereby fully incorporated by reference in its entirety for all purposes.

*Janus kinase (JAK)*/*Signal transducers and activators of transcription (STAT) pathway:* The JAK/STAT pathway plays a crucial role in mediating the signals from a diverse spectrum of cytokine receptors, growth factor receptors, and G-protein-coupled receptors. Signal transducers and activators of transcription (STAT) proteins play a crucial role in mediating the signals from a diverse spectrum of cytokine receptors growth factor receptors, and G-protein-coupled receptors. STAT directly links cytokine receptor stimulation to gene transcription by acting as both a cytosolic messenger and nuclear transcription factor. In the Janus Kinase (JAK)-STAT pathway, receptor dimerization by ligand binding results in JAK family kinase (JFK) activation and subsequent tyrosine phosphorylation of the receptor, which leads to the recruitment of STAT through the SH2 domain, and the phosphorylation of conserved tyrosine residue. Tyrosine phosphorylated STAT forms a dimer, translocates to the nucleus, and binds to specific DNA elements to activate target gene transcription, which leads to the regulation of cellular proliferation, differentiation, and apoptosis. The entire process is tightly regulated at multiple levels by protein tyrosine phosphatases, suppressors of cytokine signaling and protein inhibitors of activated STAT. In mammals seven members of the STAT family (STAT1, STAT2, STAT3, STAT4, STAT5a, STAT5b and STAT6) have been identified. JAKs contain two symmetrical kinase-like domains; the C-terminal JAK homology 1 (JH1) domain possesses tyrosine kinase function while the immediately adjacent JH2 domain is enzymatically inert but is believed to regulate the activity of JH1. There are four JAK family members: JAK1, JAK2, JAK3 and tyrosine kinase 2 (Tyk2). Expression is ubiquitous for JAK1, JAK2 and TYK2 but restricted to hematopoietic cells for JAK3. Mutations in JAK proteins have been described for several myeloid malignancies. Specific examples include but are not limited to: Somatic JAK3 (e.g. JAK3A572V, JAK3V722I, JAK3P132T) and fusion JAK2 (e.g. ETV6-JAK2, PCM1- JAK2, BCR-JAK2) mutations have respectively been described in acute megakaryocytic leukemia and acute leukemia/chronic myeloid malignancies, JAK2 (V617F, JAK2 exon 12 mutations) and MPL MPLWS15L/K/S, MPLS505N) mutations associated with myeloproliferative disorders and myeloproliferative neoplasms. JAK2 mutations, primarily JAK2V617F, are invariably associated with polycythemia vera (PV). This mutation also occurs in the majority of patients with essential thrombocythemia (ET) or primary myelofibrosis (PMF) (Tefferi n., Leukemia & Lymphoma, March 2008; 49(3): 388 - 397). STATs can be activated in a JAK-independent manner by src family kinase members and by oncogenic FLt3 ligand-ITD (Hayakawa and Naoe, Ann N Y Acad Sci. 2006 Nov;1086:213-22; Choudhary et al. Activation mechanisms of STAT5 by oncogenic FLt3 ligand-ITD. Blood (2007) vol. 110 (1) pp. 370-4). Although mutations of STATs have not been described in human tumors, the activity of several members of the family, such as STAT1, STAT3 and STAT5, is dysregulated in a variety of human tumors and leukemias. STAT3 and STAT5 acquire oncogenic potential through constitutive phosphorylation on tyrosine, and their activity has been shown to be required to sustain a transformed phenotype. This was shown in lung cancer where tyrosine phosphorylation of STAT3 was JAK-independent and mediated by EGF receptor activated through mutation and Src. (Alvarez et al., Cancer Research, Cancer Res 2006; 66) STAT5 phosphorylation was also shown to be required for the long-term maintenance of leukemic stem cells. (Schepers et al. STAT5 is required for long-term maintenance of normal and leukemic human stem/progenitor cells. Blood (2007) vol. 110 (8) pp. 2880-2888) In contrast to STAT3 and STAT5, STAT1 negatively regulates cell proliferation and angiogenesis and thereby inhibits tumor formation. Consistent with its tumor suppressive properties, STAT1 and its downstream targets have been shown to be reduced in a variety of human tumors (Rawlings, J. The JAK/STAT signaling pathway. J of Cell Science. 2004; 117 (8):1281-1283, hereby fully incorporated by reference in its entirety for all purposes).

### Binding Elements

In some embodiments of the invention, the activation level of an activatable element is determined. One embodiment makes this determination by contacting a cell from a cell population with a binding element that is specific for an activation state of the activatable element. The term "Binding element" includes any molecule, e.g., peptide, nucleic acid, small organic molecule which is capable of detecting an activation state of an activatable element over another activation state of the activatable element. Binding elements and labels for binding elements are shown in U.S.S.N. /048,886; 61/048,920 and 61/048,657.

In some embodiments, the binding element is a peptide, polypeptide, oligopeptide or a protein. The peptide, polypeptide, oligopeptide or protein can be made up of naturally occurring amino acids and peptide bonds, or synthetic peptidomimetic structures. Thus "amino acid", or "peptide residue", as used herein include both naturally occurring and synthetic amino acids. For example, homo-phenylalanine, citrulline and noreleucine are considered amino acids for the purposes of the invention. The side chains can be in either the (R) or the (S) configuration. In some embodiments, the amino acids are in the (S) or L-configuration. If non-naturally occurring side chains are used, non-amino acid substituents can be used, for example to prevent or retard in vivo degradation. Proteins including non-naturally occurring amino acids can be synthesized or in some cases, made recombinantly; see van Hest et al., FEBS Lett 428:(1-2) 68-70 May 22, 1998 and Tang et al., Abstr. Pap Am. Chem. S218: U138 Part 2 Aug. 22, 1999, both of which are expressly incorporated by reference herein.

Methods of the present invention can be used to detect any particular activatable element in a sample that is antigenically detectable and antigenically distinguishable from other activatable elements which are present in the sample. For example, the activation state-specific antibodies of the present invention can be used in the present methods to identify distinct signaling cascades of a subset or subpopulation of complex cell populations; and the ordering of protein activation (e.g., kinase activation) in potential signaling hierarchies. Hence, in some embodiments the expression and phosphorylation of one or more polypeptides are detected and quantified using methods of the present invention. In some embodiments, the expression and phosphorylation of one or more polypeptides that are cellular components of a cellular pathway are detected and quantified using methods of the present invention. As used herein, the term "activation state-specific antibody" or "activation state antibody" or grammatical equivalents thereof, refer to an antibody that specifically binds to a corresponding and specific antigen. Preferably, the corresponding and specific antigen is a specific form of an activatable element. Also preferably, the binding of the activation state-specific antibody is indicative of a specific activation state of a specific activatable element.

In some embodiments, the binding element is an antibody. In some embodiment, the binding element is an activation state-specific antibody.

The term "antibody" includes full length antibodies and antibody fragments, and can refer to a natural antibody from any organism, an engineered antibody, or an antibody generated recombinantly for experimental, therapeutic, or other purposes as further defined below. Examples of antibody fragments, as are known in the art, such as Fab, Fab', F(ab')2, Fv, scFv, or other antigen-binding subsequences of antibodies, either produced by the modification of whole antibodies or those synthesized de novo using recombinant DNA technologies. The term "antibody" comprises monoclonal and polyclonal antibodies. Antibodies can be antagonists, agonists, neutralizing, inhibitory, or stimulatory. They can be humanized, glycosylated, bound to solid supports, and posses other variations. See U.S.S.Nos. 61/048,886; 61/048,920 and 61/048,657 for more information about antibodies as binding elements.

Activation state specific antibodies can be used to detect kinase activity, however additional means for determining kinase activation are provided by the present invention. For example, substrates that are specifically recognized by protein kinases and phosphorylated thereby are known. Antibodies that specifically bind to such phosphorylated substrates but do not bind to such non-phosphorylated substrates (phospho-substrate antibodies) can be used to determine the presence of activated kinase in a sample.

The antigenicity of an activated isoform of an activatable element is distinguishable from the antigenicity of non-activated isoform of an activatable element or from the antigenicity of an isoform of a different activation state. In some embodiments, an activated isoform of an element possesses an epitope that is absent in a non-activated isoform of an element, or vice versa. In some embodiments, this difference is due to covalent addition of moieties to an element, such as phosphate moieties, or due to a structural change in an element, as through protein cleavage, or due to an otherwise induced conformational change in an element which causes the element to present the same sequence in an antigenically distinguishable way. In some embodiments, such a conformational change causes an activated isoform of an element to present at least one epitope that is not present in a non-activated isoform, or to not present at least one epitope that is presented by a non-activated isoform of the element. In some embodiments, the epitopes for the distinguishing antibodies are centered around the active site of the element, although as is known in the art, conformational changes in one area of an element may cause alterations in different areas of the element as well.

Many antibodies, many of which are commercially available (for example, see Cell Signaling Technology, www.cellsignal.com or Becton Dickinson, www.bd.com) have been produced which specifically bind to the phosphorylated isoform of a protein but do not specifically bind to a non-phosphorylated isoform of a protein. Many such antibodies have been produced for the study of signal transducing proteins which are reversibly phosphorylated. Particularly, many such antibodies have been produced which specifically bind to phosphorylated, activated isoforms of protein. Examples of proteins that can be analyzed with the methods described herein include, but are not limited to, kinases, HER receptors, PDGF receptors, FLT3 receptor, Kit receptor, FGF receptors, Eph receptors, Trk receptors, IGF receptors, Insulin receptor, Met receptor, Ret, VEGF receptors, TIE1, TIE2, erythropoetin receptor, thromobopoetin receptor, CD114, CD116, FAK, Jak1, Jak2, Jak3, Tyk2, Src, Lyn, Fyn, Lck, Fgr, Yes, Csk, Abl, Btk, ZAP70, Syk, IRAKs, cRaf, ARaf, BRAF, Mos, Lim kinase, ILK, Tp1, ALK, TGFβ receptors, BMP receptors, MEKKs, ASK, MLKs, DLK, PAKs, Mek 1, Mek 2, MKK3/6, MKK4/7, ASK1,Cot, NIK, Bub, Myt 1, Weel, Casein kinases, PDK1, SGK1, SGK2, SGK3, Akt1, Akt2, Akt3, p90Rsks, p70S6Kinase,Prks, PKCs, PKAs, ROCK 1, ROCK 2, Auroras, CaMKs, MNKs, AMPKs, MELK, MARKs, Chk1, Chk2, LKB-1, MAPKAPKs, Pim1, Pim2, Pim3, IKKs, Cdks, Jnks, Erks, IKKs, GSK3α, GSK3β, Cdks, CLKs, PKR, PI3-Kinase class 1, class 2, class 3, mTor, SAPK/JNK1,2,3, p38s, PKR, DNA-PK, ATM, ATR, phosphatases, Receptor protein tyrosine phosphatases (RPTPs), LAR phosphatase, CD45, Non receptor tyrosine phosphatases (NPRTPs), SHPs, MAP kinase phosphatases (MKPs), Dual Specificity phosphatases (DUSPs), CDC25 phosphatases, Low molecular weight tyrosine phosphatase, Eyes absent (EYA) tyrosine phosphatases, Slingshot phosphatases (SSH), serine phosphatases, PP2A, PP2B, PP2C, PP1, PPS, inositol phosphatases, PTEN, SHIPs, myotubularins, lipid signaling, phosphoinositide kinases, phopsholipases, prostaglandin synthases, 5-lipoxygenase, sphingosine kinases, sphingomyelinases, adaptor/scaffold proteins, Shc, Grb2, BLNK, LAT, B cell adaptor for PI3-kinase (BCAP), SLAP, Dok, KSR, MyD88, Crk, CrkL, GAD, Nck, Grb2 associated binder (GAB), Fas associated death domain (FADD), TRADD, TRAF2, RIP, T-Cell leukemia family, cytokines, IL-2, IL-4, IL-8, IL-6, interferon γ, interferon α, cytokine regulators, suppressors of cytokine signaling (SOCs), ubiquitination enzymes, Cbl, SCF ubiquitination ligase complex, APC/C, adhesion molecules, integrins, Immunoglobulin-like adhesion molecules, selectins, cadherins, catenins, focal adhesion kinase, p130CAS, cytoskeletal/contractile proteins, fodrin, actin, paxillin, myosin, myosin binding proteins, tubulin, eg5/KSP, CENPs, heterotrimeric G proteins, β-adrenergic receptors, muscarinic receptors, adenylyl cyclase receptors, small molecular weight GTPases, H-Ras, K- Ras, N-Ras, Ran, Rac, Rho, Cdc42, Arfs, RABs, RHEB, guanine nucleotide exchange factors, Vav, Tiam, Sos, Dbl, PRK, TSC1,2, GTPase activating proteins, Ras-GAP, Arf-GAPs, Rho-GAPs, caspases, Caspase 2, Caspase 3, Caspase 6, Caspase 7, Caspase 8, Caspase 9, proteins involved in apoptosis, Bc1-2, Mcl-1, Bc1-XL, Bcl-w, Bcl-B, Al, Bax, Bak, Bok, Bik, Bad, Bid, Bim, Bmf, Hrk, Noxa, Puma, IAPs, XIAP, Smac, cell cycle regulators, Cdk4, Cdk 6, Cdk 2, Cdk1, Cdk 7, Cyclin D, Cyclin E, Cyclin A, Cyclin B, Rb, p16, p14Arf, p27KIP, p21CIP, molecular chaperones, Hsp90s, Hsp70, Hsp27, metabolic enzymes, Acetyl-CoAa Carboxylase, ATP citrate lyase, nitric oxide synthase, vesicular transport proteins, caveolins, endosomal sorting complex required for transport (ESCRT) proteins, vesicular protein sorting (Vsps), hydroxylases, prolyl-hydroxylases PHD-1, 2 and 3, asparagine hydroxylase FIH transferases, isomerases, Pin1 prolyl isomerase, topoisomerases, deacetylases, Histone deacetylases, sirtuins, acetylases, histone acetylases, CBP/P300 family, MYST family, ATF2, methylases, DNA methyl transferases, demethylases, Histone H3K4 demethylases, H3K27, JHDM2A, UTX, tumor suppressor genes, VHL, WT-1, p53, Hdm, PTEN, proteases, ubiquitin proteases, urokinase-type plasminogen activator (uPA) and uPA receptor (uPAR) system, cathepsins, metalloproteinases, esterases, hydrolases, separase, ion channels, potassium channels, sodium channels, molecular transporters, multi-drug resistance proteins, P-Gycoprotein, nucleoside transporters, transcription factors/ DNA binding proteins, Ets, Elk, SMADs, Rel-A (p65-NFKB), CREB, NFAT, ATF-2, AFT, Myc, Fos, Spl, Egr-1, T-bet, β-catenin, HIFs, FOXOs, E2Fs, SRFs, TCFs, Egr-1, β-FOXO STAT1, STAT 3, STAT 4, STAT 5, STAT 6, p53, WT-1, HMGA, regulators of translation, pS6, 4EPB-1, eIF4E-binding protein, regulators of transcription, RNA polymerase, initiation factors, elongation factors. In some embodiments, the protein is S6.

In some embodiments, an epitope-recognizing fragment of an activation state antibody rather than the whole antibody is used. In some embodiments, the epitope-recognizing fragment is immobilized. In some embodiments, the antibody light chain that recognizes an epitope is used. A recombinant nucleic acid encoding a light chain gene product that recognizes an epitope can be used to produce such an antibody fragment by recombinant means well known in the art.

In some embodiments, assessment of the activation state of the activatable element is made using mass spectrometry. The activation state of the activatable element can be determined using quantitative mass spectrometry. One type of quantitative mass spectrometry is stable isotope labeling by amino acids in cell culture (SILAC). To enable quantitative assessment of activation using SILAC, cells are grown in either light medium (e.g. containing the radio-neutral form of the natural amino acids lysine and arginine) or in heavy medium (e.g. containing lysine and arginine having naturally-occurring carbon-12 completely substituted with the carbon-13 isotope). SILAC methods are further described in U.S.S.N. 11/368,996 and 11/314,323, and U.S. Patent Nos. 7,300,753 and 6,906,320. Following culture of cells for greater than 12, 14, 16, 18, 20, 22, 24, 30, 36, 48, or 72 hours, the appropriate carbon isotope is incorporated into cellular proteins from the growth medium. Cells cultured thus can be treated to isolate and query an activatable element using any of the methods described herein. For example, antibodies can be used to immunoprecipitate a target protein. Isolated proteins can be identified, quantified, and/or measured for one or more modifications using quantitative mass spectrometry. Pooling of samples obtained from heavy- and light-labeled cells can be used to detect heavy and light peptides simultaneously using mass spectrometry. This simultaneous detection allows direct quantitative comparison of heavy and light peptides. In some embodiments, one population of cells (e.g. heavy-labeled) is treated with a modulator, while the other population of cells (e.g. light-labeled) does not receive contact with the modulator. Cell populations that are differentially labeled and treated can be quantitatively compared using SILAC.

In some embodiments, no enrichment step is performed, and SILAC analysis is performed directly on whole cell lysates. To ensure that any measured changes are robust, SILAC procedures can be repeated with the labeling reversed.

In some embodiments, assessment of the activation state of the activatable element is made using microfluidic image cytometry (MIC). Microscale technologies such as microfluidics offer intrinsic advantages of minimal sample/reagent usage, operational fidelity, high throughput, cost efficiency, and precise control over reagent and sample delivery to microscale environments. In some embodiments, microfluidic image cytometry involves a cell array chip comprising a plurality of microfluidic cell culture chambers, wherein each chamber has a volume of about 20, 30, 40, 50, 60, 70, 80, 90, 100, 120, 140, 160, 180, 200, 220, 240, 260, 280, 300, 350, 400, 450, or 500 nL. Microchannels can be etched on the chips using lithography methods known in the art in order to control contact of cells within the microfluidic cell culture chambers with various regeants and culture media.

Cells can be placed within the microfluidic cell culture chambers, and treated using microscale versions of the methods described herein. For example, the activation state of one or more activatable elements can be assessed for cells within the microfluidic cell culture chambers using immunocytochemistry. In other embodiments, the cells are analyzed using immunohistochemistry. Following the immunolabeling of these methods, the activation state of the activatable elements within the cells can be visualized using known microscopy-based image acquisition methods.

In alternative embodiments of the instant invention, aromatic amino acids of protein binding elements can be replaced with other molecules. See U.S. S. Nos. 61/048,886; 61/048,920 and 61/048,657.

In some embodiments, the activation state-specific binding element is a peptide comprising a recognition structure that binds to a target structure on an activatable protein. A variety of recognition structures are well known in the art and can be made using methods known in the art, including by phage display libraries (see e.g., Gururaja et al. Chem. Biol. (2000) 7:515-27; Houimel et al., Eur. J. Immunol. (2001) 31:3535-45; Cochran et al. J. Am. Chem. Soc. (2001) 123:625-32; Houimel et al. Int. J. Cancer (2001) 92:748-55, each incorporated herein by reference). Further, fluorophores can be attached to such antibodies for use in the methods of the present invention.

A variety of recognition structures are known in the art (e.g., Cochran et al., J. Am. Chem. Soc. (2001) 123:625-32; Boer et al., Blood (2002) 100:467-73, each expressly incorporated herein by reference)) and can be produced using methods known in the art (see e.g., Boer et al., Blood (2002) 100:467-73; Gualillo et al., Mol. Cell Endocrinol. (2002) 190:83-9, each expressly incorporated herein by reference)), including for example combinatorial chemistry methods for producing recognition structures such as polymers with affinity for a target structure on an activatable protein (see e.g., Barn et al., J. Comb. Chem. (2001) 3:534-41; Ju et al., Biotechnol. (1999) 64:232-9, each expressly incorporated herein by reference). In another embodiment, the activation state-specific antibody is a protein that only binds to an isoform of a specific activatable protein that is phosphorylated and does not bind to the isoform of this activatable protein when it is not phosphorylated or nonphosphorylated. In another embodiment the activation state-specific antibody is a protein that only binds to an isoform of an activatable protein that is intracellular and not extracellular, or vice versa. In a some embodiment, the recognition structure is an anti-laminin single-chain antibody fragment (scFv) (see e.g., Sanz et al., Gene Therapy (2002) 9:1049-53; Tse et al., J. Mol. Biol. (2002) 317:85-94, each expressly incorporated herein by reference).

In some embodiments the binding element is a nucleic acid. The term "nucleic acid" include nucleic acid analogs, for example, phosphoramide (Beaucage et al., Tetrahedron 49(10):1925 (1993) and references therein; Letsinger, J. Org. Chem. 35:3800 (1970); Sprinzl et al., Eur. J. Biochem. 81:579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Pat. No. 5,644,048), phosphorodithioate (Briu et al., J. Am. Chem. Soc. 111:2321 (1989), O-methylphophoroamidite linkages (see Eckstein, Oligonucleotides and Analogues: A Practical Approach, Oxford University Press), and peptide nucleic acid backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996), all of which are incorporated by reference). Other analog nucleic acids include those with positive backbones (Denpcy et al., Proc. Natl. Acad. Sci. USA 92:6097 (1995); non-ionic backbones (U.S. Pat. Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y. S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Pat. Nos. 5,235,033 and 5,034,506, and Chapters 6 and 7, ASC Symposium Series 580, "Carbohydrate Modifications in Antisense Research", Ed. Y. S. Sanghui and P. Dan Cook. Nucleic acids containing one or more carbocyclic sugars are also included within the definition of nucleic acids (see Jenkins et al., Chem. Soc. Rev. (1995) pp169- 176). Several nucleic acid analogs are described in Rawls, C & E News Jun. 2, 1997 page 35. All of these references are hereby expressly incorporated by reference. These modifications of the ribose-phosphate backbone can be done to facilitate the addition of additional moieties such as labels, or to increase the stability and half-life of such molecules in physiological environments.

In some embodiment the binding element is a small organic compound. Binding elements can be synthesized from a series of substrates that can be chemically modified. "Chemically modified" herein includes traditional chemical reactions as well as enzymatic reactions. These substrates generally include, but are not limited to, alkyl groups (including alkanes, alkenes, alkynes and heteroalkyl), aryl groups (including arenes and heteroaryl), alcohols, ethers, amines, aldehydes, ketones, acids, esters, amides, cyclic compounds, heterocyclic compounds (including purines, pyrimidines, benzodiazepins, beta-lactams, tetracylines, cephalosporins, and carbohydrates), steroids (including estrogens, androgens, cortisone, ecodysone, etc.), alkaloids (including ergots, vinca, curare, pyrollizdine, and mitomycines), organometallic compounds, hetero-atom bearing compounds, amino acids, and nucleosides. Chemical (including enzymatic) reactions can be done on the moieties to form new substrates or binding elements that can then be used in the present invention.

In some embodiments the binding element is a carbohydrate. As used herein the term carbohydrate is meant to include any compound with the general formula (CH₂O)ₙ. Examples of carbohydrates are di-, tri- and oligosaccharides, as well polysaccharides such as glycogen, cellulose, and starches.

In some embodiments the binding element is a lipid. As used herein the term lipid herein is meant to include any water insoluble organic molecule that is soluble in nonpolar organic solvents. Examples of lipids are steroids, such as cholesterol, and phospholipids such as sphingomeylin.

Examples of activatable elements, activation states and methods of determining the activation level of activatable elements are described in US publication number 20060073474 entitled "Methods and compositions for detecting the activation state of multiple proteins in single cells" and US publication number 20050112700 entitled "Methods and compositions for risk stratification" the content of which are incorporate here by reference.

### Labels

The methods and compositions of the instant invention provide binding elements comprising a label, labeling element, or tag. By label or labeling element is meant a molecule that can be directly (i.e., a primary label) or indirectly (i.e., a secondary label) detected; for example a label can be visualized and/or measured or otherwise identified so that its presence or absence can be known. Binding elements and labels for binding elements are shown in U.S.S.N. /048,886; 61/048,920 and 61/048,657.

A compound can be directly or indirectly conjugated to a label which provides a detectable signal, e.g. radioisotopes, fluorophores, enzymes, antibodies, particles such as magnetic particles, chemiluminescent molecules, molecules that can be detected by mass spec, or specific binding molecules, etc. Specific binding molecules include pairs, such as biotin and streptavidin, digoxin and antidigoxin etc. Examples of labels include, but are not limited to, optical fluorescent and chromogenic dyes including labels, label enzymes and radioisotopes. In some embodiments of the invention, these labels can be conjugated to the binding elements.

In some embodiments, one or more binding elements are uniquely labeled. Using the example of two activation state specific antibodies, by "uniquely labeled" is meant that a first activation state antibody recognizing a first activated element comprises a first label, and second activation state antibody recognizing a second activated element comprises a second label, wherein the first and second labels are detectable and distinguishable, making the first antibody and the second antibody uniquely labeled.

In general, labels fall into four classes: a) isotopic labels, which can be radioactive or heavy isotopes; b) magnetic, electrical, thermal labels; c) colored, optical labels including luminescent, phosphorous and fluorescent dyes or moieties; and d) binding partners. Labels can also include enzymes (horseradish peroxidase, luciferase, beta-galactosidase, etc.) and magnetic particles. In some embodiments, the detection label is a primary label. A primary label is one that can be directly detected, such as a fluorophore.

Labels include optical labels such as fluorescent dyes or moieties. Fluorophores can be either "small molecule" fluors, or proteinaceous fluors (e.g. green fluorescent proteins and all variants thereof).

In some embodiments, activation state-specific antibodies are labeled with quantum dots as disclosed by Chattopadhyay, P.K. et al. Quantum dot semiconductor nanocrystals for immunophenotyping by polychromatic flow cytometry. Nat. Med. 12, 972-977 (2006). Quantum dot labels are commercially available through Invitrogen, http://probes.invitrogen.com/products/qdot/.

Quantum dot labeled antibodies can be used alone or they can be employed in conjunction with organic fluorochrome-conjugated antibodies to increase the total number of labels available. As the number of labeled antibodies increase so does the ability for subtyping known cell populations. Additionally, activation state-specific antibodies can be labeled using chelated or caged lanthanides as disclosed by Erkki, J. et al. Lanthanide chelates as new fluorochrome labels for cytochemistry. J. Histochemistry Cytochemistry, 36:1449-1451, 1988, and U.S. Patent No. 7,018850, entitled Salicylamide-Lanthanide Complexes for Use as Luminescent Markers. Other methods of detecting fluorescence can also be used, e.g., Quantum dot methods (see, e.g., Goldman et al., J. Am. Chem. Soc. (2002) 124:6378-82; Pathak et al. J. Am. Chem. Soc. (2001) 123:4103-4; and Remade et al., Proc. Natl. Sci. USA (2000) 18:553-8, each expressly incorporated herein by reference) as well as confocal microscopy.

In some embodiments, the activatable elements are labeled with tags suitable for Inductively Coupled Plasma Mass Spectrometer (ICP-MS) as disclosed in Tanner et al. Spectrochimica Acta Part B: Atomic Spectroscopy, 2007 Mar;62(3):188-195.

Alternatively, detection systems based on FRET, discussed in detail below, can be used. FRET finds use in the instant invention, for example, in detecting activation states that involve clustering or multimerization wherein the proximity of two FRET labels is altered due to activation. In some embodiments, at least two fluorescent labels are used which are members of a fluorescence resonance energy transfer (FRET) pair.

The methods and composition of the present invention can also make use of label enzymes. By label enzyme is meant an enzyme that may be reacted in the presence of a label enzyme substrate that produces a detectable product. Suitable label enzymes for use in the present invention include but are not limited to, horseradish peroxidase, alkaline phosphatase and glucose oxidase. Methods for the use of such substrates are well known in the art. The presence of the label enzyme is generally revealed through the enzyme's catalysis of a reaction with a label enzyme substrate, producing an identifiable product. Such products can be opaque, such as the reaction of horseradish peroxidase with tetramethyl benzedine, and can have a variety of colors. Other label enzyme substrates, such as Luminol (available from Pierce Chemical Co.), have been developed that produce fluorescent reaction products. Methods for identifying label enzymes with label enzyme substrates are well known in the art and many commercial kits are available. Examples and methods for the use of various label enzymes are described in Savage et al., Previews 247:6-9 (1998), Young, J. Virol. Methods 24:227-236 (1989), which are each hereby incorporated by reference in their entirety.

By radioisotope is meant any radioactive molecule. Suitable radioisotopes for use in the invention include, but are not limited to ¹⁴C, ³H, ³²P, .³³P, ³⁵S, ¹²⁵I and ¹³I. The use of radioisotopes as labels is well known in the art.

As mentioned, labels can be indirectly detected, that is, the tag is a partner of a binding pair. By "partner of a binding pair" is meant one of a first and a second moiety, wherein the first and the second moiety have a specific binding affinity for each other. Suitable binding pairs for use in the invention include, but are not limited to, antigens/antibodies (for example, digoxigenin/anti-digoxigenin, dinitrophenyl (DNP)/anti-DNP, dansyl-X-anti-dansyl, Fluorescein/anti-fluorescein, lucifer yellow/anti-lucifer yellow, and rhodamine anti-rhodamine), biotin/avidin (or biotin/streptavidin) and calmodulin binding protein (CBP)/calmodulin. Other suitable binding pairs include polypeptides such as the FLAG-peptide [Hopp et al., BioTechnology, 6:1204-1210 (1988)]; the KT3 epitope peptide [Martin et al., Science, 255: 192-194 (1992)]; tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)] and the antibodies each thereto. As will be appreciated by those in the art, binding pair partners can be used in applications other than for labeling, as is described herein.

As will be appreciated by those in the art, a partner of one binding pair can also be a partner of another binding pair. For example, an antigen (first moiety) can bind to a first antibody (second moiety) that can, in turn, be an antigen for a second antibody (third moiety). It will be further appreciated that such a circumstance allows indirect binding of a first moiety and a third moiety via an intermediary second moiety that is a binding pair partner to each.

As will be appreciated by those in the art, a partner of a binding pair can comprise a label, as described above. It will further be appreciated that this allows for a tag to be indirectly labeled upon the binding of a binding partner comprising a label. Attaching a label to a tag that is a partner of a binding pair, as just described, is referred to herein as "indirect labeling".

By "surface substrate binding molecule" or "attachment tag" and grammatical equivalents thereof is meant a molecule have binding affinity for a specific surface substrate, which substrate is generally a member of a binding pair applied, incorporated or otherwise attached to a surface. Suitable surface substrate binding molecules and their surface substrates include, but are not limited to poly-histidine (poly-his) or poly-histidine-glycine (poly-his-gly) tags and Nickel substrate; the Glutathione-S Transferase tag and its antibody substrate (available from Pierce Chemical); the flu HA tag polypeptide and its antibody 12CA5 substrate [Field et al., Mol. Cell. Biol., 8:2159-2165 (1988)]; the c-myc tag and the 8F9, 3C7, 6E10, G4, B7 and 9E10 antibody substrates thereto [Evan et al., Molecular and Cellular Biology, 5:3610-3616 (1985)]; and the Herpes Simplex virus glycoprotein D (gD) tag and its antibody substrate [Paborsky et al., Protein Engineering, 3(6):547-553 (1990)]. In general, surface binding substrate molecules useful in the present invention include, but are not limited to, polyhistidine structures (His-tags) that bind nickel substrates, antigens that bind to surface substrates comprising antibody, haptens that bind to avidin substrate (e.g., biotin) and CBP that binds to surface substrate comprising calmodulin.

An alternative activation state indicator useful with the instant invention is one that allows for the detection of activation by indicating the result of such activation. For example, phosphorylation of a substrate can be used to detect the activation of the kinase responsible for phosphorylating that substrate. Similarly, cleavage of a substrate can be used as an indicator of the activation of a protease responsible for such cleavage. Methods are well known in the art that allow coupling of such indications to detectable signals, such as the labels and tags described above in connection with binding elements. For example, cleavage of a substrate can result in the removal of a quenching moiety and thus allowing for a detectable signal being produced from a previously quenched label.

### Detection

In practicing the methods of this invention, the detection of the status of the one or more activatable elements can be carried out by a person, such as a technician in the laboratory. Alternatively, the detection of the status of the one or more activatable elements can be carried out using automated systems. In either case, the detection of the status of the one or more activatable elements for use according to the methods of this invention is performed according to standard techniques and protocols well-established in the art.

One or more activatable elements can be detected and/or quantified by any method that detects and/or quantitates the presence of the activatable element of interest. Such methods can include radioimmunoassay (RIA) or enzyme linked immunoabsorbance assay (ELISA), immunohistochemistry, immunofluorescent histochemistry with or without confocal microscopy, reversed phase assays, homogeneous enzyme immunoassays, and related non-enzymatic techniques, Western blots, whole cell staining , immunoelectronmicroscopy, nucleic acid amplification, gene array, protein array, mass spectrometry, patch clamp, 2-dimensional gel electrophoresis, differential display gel electrophoresis, microsphere-based multiplex protein assays, label-free cellular assays and flow cytometry, etc. U. S. Pat. No. 4,568,649 describes ligand detection systems, which employ scintillation counting. These techniques are particularly useful for modified protein parameters. Cell readouts for proteins and other cell determinants can be obtained using fluorescent or otherwise tagged reporter molecules. Flow cytometry methods are useful for measuring intracellular parameters. See the above patents and applications for example methods.

In some embodiments, the present invention provides methods for determining an activatable element's activation profile for a single cell. The methods can comprise analyzing cells by flow cytometry on the basis of the activation level of at least two activatable elements. Binding elements (e.g. activation state-specific antibodies) are used to analyze cells on the basis of activatable element activation level, and can be detected as described below. Alternatively, non- binding elements systems as described above can be used in any system described herein.

Detection of cell signaling states can be accomplished using binding elements and labels. Cell signaling states can be detected by a variety of methods known in the art. They generally involve a binding element, such as an antibody, and a label, such as a fluorchrome to form a detection element. Detection elements do not need to have both of the above agents, but can be one unit that possesses both qualities. These and other methods are well described in U.S. Patent No. 7,381535 and 7,393,656 and U.S.S.Nos. 10/193,462; 11/655,785; 11/655,789; 11/655,821; 11/338,957, 61/048,886; 61/048,920; and 61/048,657 which are all incorporated by reference in their entireties.

In one embodiment of the invention, it is advantageous to increase the signal to noise ratio by contacting the cells with the antibody and label for a time greater than 5, 6, 7, 8, 9, 10, 11, 12, 13, 14, 15, 16, 17, 18, 19, 20, 24 or up to 48 or more hours.

When using fluorescent labeled components in the methods and compositions of the present invention, it will recognized that different types of fluorescent monitoring systems, e.g., cytometric measurement device systems, can be used to practice the invention. In some embodiments, flow cytometric systems are used or systems dedicated to high throughput screening, e.g. 96 well or greater microtiter plates. Methods of performing assays on fluorescent materials are well known in the art and are described in, e.g., Lakowicz, J. R., Principles of Fluorescence Spectroscopy, New York: Plenum Press (1983); Herman, B., Resonance energy transfer microscopy, in: Fluorescence Microscopy of Living Cells in Culture, Part B, Methods in Cell Biology, vol. 30, ed. Taylor, D. L. & Wang, Y.-L., San Diego: Academic Press (1989), pp. 219-243; Turro, N. J., Modern Molecular Photochemistry, Menlo Park: Benjamin/Cummings Publishing Col, Inc. (1978), pp. 296-361.

Fluorescence in a sample can be measured using a fluorometer. In general, excitation radiation, from an excitation source having a first wavelength, passes through excitation optics. The excitation optics cause the excitation radiation to excite the sample. In response, fluorescent proteins in the sample emit radiation that has a wavelength that is different from the excitation wavelength. Collection optics then collect the emission from the sample. The device can include a temperature controller to maintain the sample at a specific temperature while it is being scanned. According to one embodiment, a multi-axis translation stage moves a microtiter plate holding a plurality of samples in order to position different wells to be exposed. The multi-axis translation stage, temperature controller, auto-focusing feature, and electronics associated with imaging and data collection can be managed by an appropriately programmed digital computer. The computer also can transform the data collected during the assay into another format for presentation. In general, known robotic systems and components can be used.

Other methods of detecting fluorescence can also be used, e.g., Quantum dot methods (see, e.g., Goldman et al., J. Am. Chem. Soc. (2002) 124:6378-82; Pathak et al. J. Am. Chem. Soc. (2001) 123:4103-4; and Remade et al., Proc. Natl. Sci. USA (2000) 18:553-8, each expressly incorporated herein by reference) as well as confocal microscopy. In general, flow cytometry involves the passage of individual cells through the path of a laser beam. The scattering the beam and excitation of any fluorescent molecules attached to, or found within, the cell is detected by photomultiplier tubes to create a readable output, e.g. size, granularity, or fluorescent intensity.

The detecting, sorting, or isolating step of the methods of the present invention can entail fluorescence-activated cell sorting (FACS) techniques, where FACS is used to select cells from the population containing a particular surface marker, or the selection step can entail the use of magnetically responsive particles as retrievable supports for target cell capture and/or background removal. A variety of FACS systems are known in the art and can be used in the methods of the invention (see e.g., WO99/54494, filed Apr. 16, 1999; U.S. Ser. No. 20010006787, filed Jul. 5, 2001, each expressly incorporated herein by reference).

In some embodiments, a FACS cell sorter (e.g. a FACSVantage™ Cell Sorter, Becton Dickinson Immunocytometry Systems, San Jose, Calif.) is used to sort and collect cells based on their activation profile (positive cells) in the presence or absence of an increase in activation level in an activatable element in response to a modulator. Other flow cytometers that are commercially available include the LSR II and the Canto II both available from Becton Dickinson. See Shapiro, Howard M., Practical Flow Cytometry, 4th Ed., John Wiley & Sons, Inc., 2003 for additional information on flow cytometers.

In some embodiments, the cells are first contacted with fluorescent-labeled activation state-specific binding elements (e.g. antibodies) directed against specific activation state of specific activatable elements. In such an embodiment, the amount of bound binding element on each cell can be measured by passing droplets containing the cells through the cell sorter. By imparting an electromagnetic charge to droplets containing the positive cells, the cells can be separated from other cells. The positively selected cells can then be harvested in sterile collection vessels. These cell-sorting procedures are described in detail, for example, in the FACSVantage™. Training Manual, with particular reference to sections 3-11 to 3-28 and 10-1 to 10-17, which is hereby incorporated by reference in its entirety. See the patents, applications and articles referred to, and incorporated above for detection systems.

Fluorescent compounds such as Daunorubicin and Enzastaurin are problematic for flow cytometry based biological assays due to their broad fluorescence emission spectra. These compounds get trapped inside cells after fixation with agents like paraformaldehyde, and are excited by one or more of the lasers found on flow cytometers. The fluorescence emission of these compounds is often detected in multiple PMT detectors which complicates their use in multiparametric flow cytometry. A way to get around this problem is to compensate out the fluorescence emission of the compound from the PMT detectors used to measure the relevant biological markers. This is achieved using a PMT detector with a bandpass filter near the emission maximum of the fluorescent compound, and cells incubated with the compound as the compensation control when calculating a compensation matrix. The cells incubated with the fluorescent compound are fixed with paraformaldehyde, then washed and permeabilized with 100% methanol. The methanol is washed out and the cells are mixed with unlabeled fixed/permed cells to yield a compensation control consisting of a mixture of fluorescent and negative cell populations.

In another embodiment, positive cells can be sorted using magnetic separation of cells based on the presence of an isoform of an activatable element. In such separation techniques, cells to be positively selected are first contacted with specific binding element (e.g., an antibody or reagent that binds an isoform of an activatable element). The cells are then contacted with retrievable particles (e.g., magnetically responsive particles) that are coupled with a reagent that binds the specific binding element. The cell-binding element-particle complex can then be physically separated from non-positive or non-labeled cells, for example, using a magnetic field. When using magnetically responsive particles, the positive or labeled cells can be retained in a container using a magnetic field while the negative cells are removed. These and similar separation procedures are described, for example, in the Baxter Immunotherapy Isolex training manual which is hereby incorporated in its entirety.

In some embodiments, methods for the determination of a receptor element activation state profile for a single cell are provided. The methods comprise providing a population of cells and analyze the population of cells by flow cytometry. Preferably, cells are analyzed on the basis of the activation level of at least two activatable elements. In some embodiments, a multiplicity of activatable element activation-state antibodies is used to simultaneously determine the activation level of a multiplicity of elements.

In some embodiment, cell analysis by flow cytometry on the basis of the activation level of at least two elements is combined with a determination of other flow cytometry readable outputs, such as the presence of surface markers, granularity and cell size to provide a correlation between the activation level of a multiplicity of elements and other cell qualities measurable by flow cytometry for single cells.

As will be appreciated, the present invention also provides for the ordering of element clustering events in signal transduction. Particularly, the present invention allows the artisan to construct an element clustering and activation hierarchy based on the correlation of levels of clustering and activation of a multiplicity of elements within single cells. Ordering can be accomplished by comparing the activation level of a cell or cell population with a control at a single time point, or by comparing cells at multiple time points to observe subpopulations arising out of the others.

The present invention provides a valuable method of determining the presence of cellular subsets within cellular populations. Ideally, signal transduction pathways are evaluated in homogeneous cell populations to ensure that variances in signaling between cells do not qualitatively nor quantitatively mask signal transduction events and alterations therein. As the ultimate homogeneous system is the single cell, the present invention allows the individual evaluation of cells to allow true differences to be identified in a significant way.

Thus, the invention provides methods of distinguishing cellular subsets within a larger cellular population. As outlined herein, these cellular subsets often exhibit altered biological characteristics (e.g. activation levels, altered response to modulators) as compared to other subsets within the population. For example, as outlined herein, the methods of the invention allow the identification of subsets of cells from a population such as primary cell populations, e.g. peripheral blood mononuclear cells that exhibit altered responses (e.g. response associated with presence of a condition) as compared to other subsets. In addition, this type of evaluation distinguishes between different activation states, altered responses to modulators, cell lineages, cell differentiation states, etc.

As will be appreciated, these methods provide for the identification of distinct signaling cascades for both artificial and stimulatory conditions in complex cell populations, such as peripheral blood mononuclear cells, or naive and memory lymphocytes.

When necessary cells are dispersed into a single cell suspension, e.g. by enzymatic digestion with a suitable protease, e.g. collagenase, dispase, etc; and the like. An appropriate solution is used for dispersion or suspension. Such solution will generally be a balanced salt solution, e.g. normal saline, PBS, Hanks balanced salt solution, etc., conveniently supplemented with fetal calf serum or other naturally occurring factors, in conjunction with an acceptable buffer at low concentration, generally from 5-25 mM. Convenient buffers include HEPES1 phosphate buffers, lactate buffers, etc. The cells can be fixed, e.g. with 3% paraformaldehyde, and are usually permeabilized, e.g. with ice cold methanol; HEPES-buffered PBS containing 0.1% saponin, 3% BSA; covering for 2 min in acetone at -200C; and the like as known in the art and according to the methods described herein.

In some embodiments, one or more cells are contained in a well of a 96 well plate or other commercially available multiwell plate. In an alternate embodiment, the reaction mixture or cells are in a cytometric measurement device. Other multiwell plates useful in the present invention include, but are not limited to 384 well plates and 1536 well plates. Still other vessels for containing the reaction mixture or cells and useful in the present invention will be apparent to the skilled artisan.

The addition of the components of the assay for detecting the activation level or activity of an activatable element, or modulation of such activation level or activity, can be sequential or in a predetermined order or grouping under conditions appropriate for the activity that is assayed for. Such conditions are described here and known in the art. Moreover, further guidance is provided below (see, e.g., in the Examples).

In some embodiments, the activation level of an activatable element is measured using Inductively Coupled Plasma Mass Spectrometer (ICP-MS). A binding element that has been labeled with a specific element binds to the activatable. When the cell is introduced into the ICP, it is atomized and ionized. The elemental composition of the cell, including the labeled binding element that is bound to the activatable element, is measured. The presence and intensity of the signals corresponding to the labels on the binding element indicates the level of the activatable element on that cell (Tanner et al. Spectrochimica Acta Part B: Atomic Spectroscopy, 2007 Mar;62(3):188-195.).

As will be appreciated by one of skill in the art, the instant methods and compositions find use in a variety of other assay formats in addition to flow cytometry analysis. For example, DNA microarrays are commercially available through a variety of sources (Affymetrix, Santa Clara CA) or they can be custom made in the lab using arrayers which are also know (Perkin Elmer). In addition, protein chips and methods for synthesis are known. These methods and materials can be adapted for the purpose of affixing activation state binding elements to a chip in a prefigured array. In some embodiments, such a chip comprises a multiplicity of element activation state binding elements, and is used to determine an element activation state profile for elements present on the surface of a cell.

In some embodiments, a chip comprises a multiplicity of the "second set binding elements," in this case generally unlabeled. Such a chip is contacted with sample, preferably cell extract, and a second multiplicity of binding elements comprising element activation state specific binding elements is used in the sandwich assay to simultaneously determine the presence of a multiplicity of activated elements in sample. Preferably, each of the multiplicity of activation state-specific binding elements is uniquely labeled to facilitate detection.

In some embodiments, confocal microscopy can be used to detect activation profiles for individual cells. Confocal microscopy relies on the serial collection of light from spatially filtered individual specimen points, which is then electronically processed to render a magnified image of the specimen. The signal processing involved confocal microscopy has the additional capability of detecting labeled binding elements within single cells, accordingly in this embodiment the cells can be labeled with one or more binding elements. In some embodiments the binding elements used in connection with confocal microscopy are antibodies conjugated to fluorescent labels, however other binding elements, such as other proteins or nucleic acids are also possible.

In some embodiments, the methods and compositions of the instant invention can be used in conjunction with an "In-Cell Western Assay." In such an assay, cells are initially grown in standard tissue culture flasks using standard tissue culture techniques. Once grown to optimum confluency, the growth media is removed and cells are washed and trypsinized. The cells can then be counted and volumes sufficient to transfer the appropriate number of cells are aliquoted into microwell plates (e.g., Nunc ™ 96 Microwell ™ plates). The individual wells are then grown to optimum confluency in complete media whereupon the media is replaced with serum-free media. At this point controls are untouched, but experimental wells are incubated with a modulator, e.g. EGF. After incubation with the modulator cells are fixed and stained with labeled antibodies to the activation elements being investigated. Once the cells are labeled, the plates can be scanned using an imager such as the Odyssey Imager (LiCor, Lincoln Nebr.) using techniques described in the Odyssey Operator's Manual v1.2., which is hereby incorporated in its entirety. Data obtained by scanning of the multiwell plate can be analyzed and activation profiles determined as described below.

In some embodiments, the detecting is by high pressure liquid chromatography (HPLC), for example, reverse phase HPLC, and in a further aspect, the detecting is by mass spectrometry.

Flow cytometry or capillary electrophoresis formats can be used for individual capture of magnetic and other beads, particles, cells, and organisms.

Flexible hardware and software allow instrument adaptability for multiple applications. The software program modules allow creation, modification, and running of methods. The system diagnostic modules allow instrument alignment, correct connections, and motor operations. Customized tools, labware, and liquid, particle, cell and organism transfer patterns allow different applications to be performed. Databases allow method and parameter storage. Robotic and computer interfaces allow communication between instruments.

In some embodiment, the methods of the invention include the use of liquid handling components. The liquid handling systems can include robotic systems comprising any number of components. In addition, any or all of the steps outlined herein can be automated; thus, for example, the systems can be completely or partially automated. See USSN 61/048,657.

As will be appreciated by those in the art, there are a wide variety of components which can be used, including, but not limited to, one or more robotic arms; plate handlers for the positioning of microplates; automated lid or cap handlers to remove and replace lids for wells on non-cross contamination plates; tip assemblies for sample distribution with disposable tips; washable tip assemblies for sample distribution; 96 well loading blocks; cooled reagent racks; microtiter plate pipette positions (optionally cooled); stacking towers for plates and tips; and computer systems.

Fully robotic or microfluidic systems include automated liquid-, particle-, cell- and organism-handling including high throughput pipetting to perform all steps of screening applications. This includes liquid, particle, cell, and organism manipulations such as aspiration, dispensing, mixing, diluting, washing, accurate volumetric transfers; retrieving, and discarding of pipet tips; and repetitive pipetting of identical volumes for multiple deliveries from a single sample aspiration. These manipulations are cross-contamination-free liquid, particle, cell, and organism transfers. This instrument performs automated replication of microplate samples to filters, membranes, and/or daughter plates, high-density transfers, full-plate serial dilutions, and high capacity operation.

In some embodiments, chemically derivatized particles, plates, cartridges, tubes, magnetic particles, or other solid phase matrix with specificity to the assay components are used. The binding surfaces of microplates, tubes or any solid phase matrices include non-polar surfaces, highly polar surfaces, modified dextran coating to promote covalent binding, antibody coating, affinity media to bind fusion proteins or peptides, surface-fixed proteins such as recombinant protein A or G, nucleotide resins or coatings, and other affinity matrix are useful in this invention.

In some embodiments, platforms for multi-well plates, multi-tubes, holders, cartridges, minitubes, deep-well plates, microfuge tubes, cryovials, square well plates, filters, chips, optic fibers, beads, and other solid-phase matrices or platform with various volumes are accommodated on an upgradable modular platform for additional capacity. This modular platform includes a variable speed orbital shaker, and multi-position work decks for source samples, sample and reagent dilution, assay plates, sample and reagent reservoirs, pipette tips, and an active wash station. In some embodiments, the methods of the invention include the use of a plate reader.

In some embodiments, thermocycler and thermoregulating systems are used for stabilizing the temperature of heat exchangers such as controlled blocks or platforms to provide accurate temperature control of incubating samples from 0° C to 100° C.

In some embodiments, interchangeable pipet heads (single or multi-channel) with single or multiple magnetic probes, affinity probes, or pipetters robotically manipulate the liquid, particles, cells, and organisms. Multi-well or multi-tube magnetic separators or platforms manipulate liquid, particles, cells, and organisms in single or multiple sample formats.

In some embodiments, the instrumentation will include a detector, which can be a wide variety of different detectors, depending on the labels and assay. In some embodiments, useful detectors include a microscope(s) with multiple channels of fluorescence; plate readers to provide fluorescent, ultraviolet and visible spectrophotometric detection with single and dual wavelength endpoint and kinetics capability, fluorescence resonance energy transfer (FRET), luminescence, quenching, two-photon excitation, and intensity redistribution; CCD cameras to capture and transform data and images into quantifiable formats; and a computer workstation.

In some embodiments, the robotic apparatus includes a central processing unit which communicates with a memory and a set of input/output devices (e.g., keyboard, mouse, monitor, printer, etc.) through a bus. Again, as outlined below, this can be in addition to or in place of the CPU for the multiplexing devices of the invention. The general interaction between a central processing unit, a memory, input/output devices, and a bus is known in the art. Thus, a variety of different procedures, depending on the experiments to be run, are stored in the CPU memory.

These robotic fluid handling systems can utilize any number of different reagents, including buffers, reagents, samples, washes, assay components such as label probes, etc.

### Gating and Analysis

In some embodiments of the invention, different gating strategies can be used in order to analyze a specific cell population (e.g., only blasts) in a sample of mixed population after treatment with the modulator. These gating strategies can be based on the presence of one or more specific surface markers expressed on each cell type. In some embodiments, the first gate eliminates cell doublets so that the user can analyze singlets. The following gate can differentiate between dead cells and live cells and the subsequent gating of live cells classifies them into, e.g. myeloid blasts, monocytes and lymphocytes. A clear comparison can be carried out to study the effect of potential modulators, such as G-CSF on activable elements in: ungated samples, myeloid blasts, monocytes, granulocytes, lymphocytes, and/or other cell types by using two-dimensional contour plot representations, two-dimensional dot plot representations, and/or histograms. For example, a comparison can be carried out to study the effect of a modulator of the Jak/Stat signaling pathway in different cell populations within a patient sample by using two-dimensional contour plot representations of Stat5 and Stat3 phosphorylation (downstream intracellular readouts for Jak kinases) (X and Y axis). The level of basal phosphorylation and the change in phosphorylation in both Stat3 and Stat5 in response to a modulator such as G-CSF can be compared. G-CSF mediates increases in both Stat3 and Stat5 phosphorylation and this signaling can occur concurrently (subpopulations with increases in both p-Stat 3 and p-Stat5) or individually (subpopulations with either an increase in p-Stat3 or pStat5 alone). The advantage of gating is to get a clearer picture and more precise results of the effect of various activable elements on a specific cell sub-population such as blasts within a complex human sample.

In some embodiments, the present invention provides methods for classification, diagnosis, theranosis, prognosis of a condition and/or prediction of outcome after administering a therapeutic agent to treat the condition by characterizing one or more pathways in a population of cells. The characterization of one or more pathways can be performed by exposing a cell population to one or more modulators and determining the activation level of an activatable element of at least one cell in the cell population. The data can be analyzed using various metrics. Examples of metrics include: 1) measuring the difference in the log of the median fluorescence value between an unstimulated fluorochrome-antibody stained sample and a sample that has not been treated with a stimulant or stained (log (MFI_{Unstimulated Stained}) - log (MFI_{Gated Unstained})); 2) measuring the difference in the log of the median fluorescence value between a stimulated fluorochrome-antibody stained sample and a sample that has not been treated with a stimulant or stained (log (MFI_{Stimulated Stained}) - log(MFI_{Gated Unstained})); 3) measuring the change between the stimulated fluorochrome-antibody stained sample and the unstimulated fluorochrome-antibody stained sample log (MFI_{Stimulated Stained}) - log (MFI_{Unstimulated Stained}), also called "fold change in median fluorescence intensity;" 4) measuring the percentage of cells in a Quadrant Gate of a contour plot which measures multiple populations in one or more dimensions; 5) measuring MFI of phosphor positive population to obtain percentage positivity above the background; and 6) use of multimodality and spread metrics for large sample population and for subpopulation analysis. Other possible metrics include third-color analysis (3D plots); percentage positive and relative expression of various markers; clinical analysis on an individual patient basis for various parameters, including, but not limited to age, race, cytogenetics, mutational status, blast percentage, CD34+ percentage, time of relapse, survival, etc. In alternative embodiments, there are other ways of analyzing data, such as third color analysis (3D plots), which can be similar to Cytobank 2D, plus third D in color. In another embodiment, a user can analyze the signaling in subpopulations based on surface markers. For example, the user could look at: "stem cell populations" by CD34+ CD38- or CD34+ CD33- expressing cells; or drug transporter positive cells or cells identified based on their expression of the receptor for Flt3, or multiple leukemic subclones based on CD33, CD45, HLA-DR, CD11b and analyzing signaling in each subpopulation. In another alternative embodiment, a user can analyze the data based on intracellular markers, such as transcription factors or other intracellular proteins, based on a functional assay, or based on other fluorescent markers.

In some embodiments where flow cytometry is used, prior to analyzing data the populations of interest and the method for characterizing these populations are determined. For instance, there are at least two general ways of identifying populations for data analysis: (i) "Outside-in" comparison of Parameter sets for individual samples or subset (e.g., patients in a trial). In this more common case, cell populations are homogenous or lineage gated in such a way as to create distinct sets considered to be homogenous for targets of interest. An example of sample-level comparison would be the identification of signaling profiles in immune cells of a patient and correlation of these profiles with non-random distribution of clinical responses. This is considered an outside-in approach because the population of interest is pre-defined prior to the mapping and comparison of its profile to other populations. (ii) "Inside-out" comparison of parameters at the level of individual cells in a heterogeneous population. An example of this would be the signal transduction state mapping of mixed hematopoietic cells under certain conditions and subsequent comparison of computationally identified cell clusters with lineage specific markers. This could be considered an inside-out approach to single cell studies as it does not presume the existence of specific populations prior to classification. A possible drawback of this approach is that it creates populations which, at least initially, may require multiple transient markers to enumerate and may never be accessible with a single cell surface epitope. As a result, the biological significance of such populations can be difficult to determine. One advantage of this unconventional approach is the unbiased tracking of cell populations without drawing potentially arbitrary distinctions between lineages or cell types.

Each of these techniques capitalizes on the ability of flow cytometry to deliver large amounts of multiparameter data at the single cell level. For cells associated with a condition (e.g. an autoimmune disease), a third "meta-level" of data exists because cells associated with a condition (e.g. immune cells) are generally treated as a single entity and classified according to historical techniques. These techniques have included organ or tissue of origin, degree of differentiation, proliferation index, metastatic spread, and genetic or metabolic data regarding the patient.

In some embodiments, the present invention uses variance mapping techniques for mapping condition signaling space. These methods represent a significant advance in the study of condition biology because it enables comparison of conditions independent of a putative normal control. Traditional differential state analysis methods (e.g., DNA microarrays, subtractive Northern blotting) generally rely on the comparison of cells associated with a condition from each patient sample with a normal control, generally adjacent and theoretically untransformed tissue. Alternatively, they rely on multiple clusterings and reclusterings to group and then further stratify patient samples according to phenotype. In contrast, variance mapping of condition states compares condition samples first with themselves and then against the parent condition population. As a result, activation states with the most diversity among conditions provide the core parameters in the differential state analysis. Given a pool of diverse conditions, this technique allows a researcher to identify the molecular events that underlie differential condition pathology (e.g., autoimmune disease responses to therapeutic agents), as opposed to differences between conditions and a proposed normal control.

In some embodiments, when variance mapping is used to profile the signaling space of patient samples, conditions whose signaling response to modulators is similar are grouped together, regardless of tissue or cell type of origin. Similarly, two conditions that are thought to be relatively alike based on lineage markers or tissue of origin could have vastly different abilities to interpret environmental stimuli and would be profiled in two different groups.

When groups of signaling profiles have been identified it is frequently useful to determine whether other factors, such as clinical responses, presence of gene mutations, and protein expression levels, are non-randomly distributed within the groups. If experiments or literature suggest such a hypothesis in an arrayed flow cytometry experiment, it can be judged with simple statistical tests, such as the Student's t-test and the X² test. Similarly, if two variable factors within the experiment are thought to be related, the r² correlation coefficient from a linear regression is used to represent the degree of this relationship.

Examples of analysis for activatable elements are described in US publication number 20060073474 entitled "Methods and compositions for detecting the activation state of multiple proteins in single cells" and US publication number 20050112700 entitled "Methods and compositions for risk stratification" the content of which are incorporate here by reference.

Advances in flow cytometry have enabled the individual cell enumeration of up to thirteen simultaneous parameters (De Rosa et al., 2001) and are moving towards the study of genomic and proteomic data subsets (Krutzik and Nolan, 2003; Perez and Nolan, 2002). Likewise, advances in other techniques (e.g. microarrays) allow for the identification of multiple activatable elements. As the number of parameters, epitopes, and samples have increased, the complexity of experiments and the challenges of data analysis have grown rapidly. An additional layer of data complexity has been added by the development of stimulation panels which enable the study of activatable elements under a growing set of experimental conditions. See Krutzik et al, Nature Chemical Biology Feb. 2008. Methods for the analysis of multiple parameters are well known in the art. See U.S.S.No. 61/079,579 for gating analysis.

In some embodiments where flow cytometry is used, flow cytometry experiments are performed and the results are expressed as fold changes using graphical tools and analyses, including, but not limited to a heat map or a histogram to facilitate evaluation. One common way of comparing changes in a set of flow cytometry samples is to overlay histograms of one parameter on the same plot. Flow cytometry experiments ideally include a reference sample against which experimental samples are compared. Reference samples can include normal and/or cells associated with a condition. See also U.S.S.No. 61/079,537 for visualization tools.

The patients are stratified based on nodes that inform the clinical question using a variety of metrics. To stratify the patients between those patients with No Response (NR) versus a Complete Response (CR), a prioritization of the nodes can be made according to statistical significance (such as p-value or area under the curve) or their biological relevance. In some embodiments, stratification is achieved through the use of the corners classifier. This classifier is an algorithm that can utilize p variables where X1,Xp are the activation level and fold change in signaling node values. Stratification of N patients into two classes, Class 0 and Class 1, is performed by defining a region R in the space of X variables that defines the boundaries between the two classes. The principle objectives for use of the classifier are that each subject falls into exactly one class, such as active disease or well-controlled disease, of sizes n0 and n1, and that the two classes are separated in space by one or more of the p variables. The corner classifier has similarities to the Mann-Whitney-Wilcoxon test, using only rank-order information in the X variables to test if two independent samples are of the same probability distribution. In some embodiments, thresholds are set for one variable and expecting Class 0 and Class 1 subjects to fall on opposite sides of the threshold. By combining multiple variables, an X-dimensional space is created that delineates Class 0 from Class 1.

### Nodes

In some embodiments, nodes are used in the classification, diagnosis, prognosis, theranosis, and/or prediction of an outcome of an autoimmune disease in a subject. As used herein, the term "node" describes a modulator and a molecule used to measure the activation level of an activatable element. For example, a node can be expressed in terms of [activatable element, modulator]. In some embodiments, a node can also incorporate marker and/or cell-type data, such as [activatable element, modulator, cell type]. In further embodiments, a node can describe the basal level of an activatable element measured in a cell type in the absence of a modulator, for example [response measured, basal, cell type]. In some of the embodiments discussed herein, a node comprises a modulator and a labeled antibody that binds to a state-specific epitope associated with an activatable element. "Node state data," as used herein, refers to quantitative data corresponding to the signal of a molecule used to measure the response of an activatable element in one or more cells (i.e. a "node state", "activation level"). Node state data can be raw signal data or metrics ("node state metrics") quantifying any characteristic of the raw signal data. Node state metrics can express raw signal data as a relative value to a signal data generated from other cells (e.g. cells untreated with a modulator). A node can be any combination of an activatable element and a modulator. A node can also be any combination of an activatable element, modulator, and a cell type, wherein a cell type is determined by any of the preceding methods and can be expressed in terms of one or more markers.

Nodes useful in the classification, diagnosis, prognosis, theranosis, and/or prediction of an outcome of an autoimmune disease include any having a defined correlation to the presence, absence, stage, sub-type, activity level, drug responsiveness, progression, and/or changes in activity level of an autoimmune disease. Correlations can be higher than 50%, 60%, 70%, 80%, 85%, 90%, 91%, 92%, 93%, 94%, 95%, 96%, 97%, 98%, 99%, or higher. Nodes can be used alone or in combination. Combinations of nodes can include any combination of 2, 3, 4, 5, 6, 7, 8, 9, 10, 15, 20, 25 or more nodes. In some embodiments, combinations of nodes improve their utility in the classification, diagnosis, prognosis, theranosis, and/or prediction of an outcome of an autoimmune disease. In some embodiments, combinations of nodes have higher correlations to the presence, absence, stage, sub-type, activity level, drug responsiveness, progression, and/or changes in activity level of an autoimmune disease than an individual node of the combination. Correlations for node combinations can be higher than 50%, 60%, 70%, 80%, 85%, 90%, 91%, 92%, 93%, 94%, 95%, 96%, 97%, 98%, 99%, or higher. Correlations can include levels of one or more activatable elements in the active or inactive state. Correlations can also include the total protein level of an activatable element, combining both active and inactive isoforms.

Examples of nodes useful in the classification, diagnosis, prognosis, theranosis, and/or prediction of an outcome of an autoimmune disease include, but are not limited to, [Stat3, IL4, B cells], [Stat1, IL10, CD4-CD45RA+ cells], [Stat3, IL6, B cells], [Stat3, IL4, B cells], [Stat1, IFNα, CD4-CD45RA+ cells], [Stat1, IL6, CD4-CD45RA+ cells], [Stat6, IL15, CD4-CD45RA+ cells], [Stat3, IFNγ, B cells], [Stat5, IFNα, CD4-CD45RA+ cells], [Stat5, IFNα, B cells], [Stat5, IL21, CD4-CD45RA+ cells], [Stat1, IFNα, CD4-CD45RA- cells], [Stat6, IFNα, CD4-CD45RA+ cells], [Stat6, IL6, CD4+CD45RA+ cells], [Stat1, IL10, monocytes], [Stat5, IL10, monocytes], [Stat3, IFNα, CD4-CD45RA+ cells], [Stat6, IL10, B cells], [Stat1, basal, CD4-CD45RA+ cells], [Stat6, basal, B cells], [CD69, basal, CD19+ B cells], [Stat5, IL21, regulatory T cells], [Stat1, IL6, CD4+ T cells], [Lck, TCR conjugating antibodies, CD8+ T cells], [p38, basal, memory and effector CD4+ T cells], [Lck, total protein, CD8+ T cells], [Stat5, IL15, CD4+ T cells], [PLCγ2, basal, CD4+ T cells], [PLCγ2, TCR conjugating antibodies, CD4+ T cells], [Stat3, basal, B cells], [PLCγ2, basal, CD8+ T cells], [Stat3, IFNα, CD4+ memory and effector T cells], [Stat3, IL10, CD8+ memory and effector T cells], [Stat3, IL10, monocytes], [Stat3, IFNα, regulatory T cells], [Lck, TCR conjugating antibodies, CD4+ memory/effector T cells], [Stat5, IL2, CD4+ memory and effector T cells], [Stat1, IL6, CD4+CD45RA+ cells], and combination thereof.

### Methods

Embodiments of the invention may be used to diagnose, predict, or to provide therapeutic decisions for disease treatment, such as rheumatoid arthritis and SLE. One aspect of the invention involves contacting an immune cell with a modulator or no modulator; determining the activation state of one or more activatable elements in the cell; and classifying the cell based on said activation state.

In some embodiments, this invention is directed to methods and compositions, and kits for analysis, drug screening, diagnosis, prognosis, for methods of disease treatment, prediction and identification of a new druggable target.

In some embodiments, the present invention involves methods of analyzing experimental data. In some embodiments, the physiological status of cells present in a sample (e.g. clinical sample) is used, for example, in diagnosis or prognosis of a condition, patient selection for therapy using some of the agents described above, to monitor treatment, modify therapeutic regimens, to further optimize the selection of therapeutic agents which may be administered as one or a combination of agents and to identify new druggable targets. Hence, therapeutic regimens can be individualized and tailored according to the data obtained prior to, and at different times over the course of treatment, thereby providing a regimen that is individually appropriate. In some embodiments, a compound is contacted with cells to analyze the response to the compound.

In some embodiments, the present invention is directed to methods for classifying a sample derived from an individual having or suspected of having an autoimmune condition. The invention allows for identification of prognostically and therapeutically relevant subgroups of conditions and prediction of the clinical course of an individual. The methods of the invention provide tools useful in the treatment of an individual afflicted with an autoimmune condition, including but not limited to methods for assigning a risk group, methods of predicting an increased risk of relapse, methods of predicting an increased risk of increased disease activity (e.g. flares), methods of predicting an increased risk of developing secondary complications, methods of choosing a therapy for an individual, methods of predicting duration of response, response to a therapy for an individual, methods of determining the efficacy of a therapy in an individual, and methods of determining the prognosis for an individual. The present invention provides methods that can serve as a prognostic indicator to predict the course of an autoimmune condition, e.g. whether an individual will have a high or low disease activity. In another embodiment, the present invention provides information to a physician to aid in the clinical management of a patient so that the information may be translated into action, including treatment, prognosis or prediction.

In some embodiments, the invention provides methods for classification, diagnosis, prognosis, theranosis, and/or prediction of an outcome of an autoimmune disease in a subject by analyzing different discrete immune cell populations in the subject. In some embodiments, the classification, diagnosis, prognosis, theranosis, and/or prediction of an outcome of an autoimmune disease in a subject is determined by a method comprising contacting a first immune cell from a first discrete immune cell population from the subject with at least a first modulator or no modulator, contacting a second immune cell from a second discrete immune cell population from the subject with at least a second modulator or no modulator, determining an activation level of at least one activatable element in the first immune cell and the second immune cell, creating a response panel for the subject comprising the determined activation levels of the activatable elements, and making a decision regarding the classification, diagnosis, prognosis, theranosis, and/or prediction of an outcome of the autoimmune disease in the subject, wherein the decision is based on the response panel. In some embodiments, the combination of activatable elements, immune cell types and modulators are selected from the combination of activatable elements, immune cell types and modulators listed in Tables 1, 2, 3, and 4.

The activation of an activatable element in the cell upon treatment with one or more modulators can reveal operative pathways in a condition that can then be used, e.g., as an indicator to predict course of the condition, to identify risk group, to predict an increased risk of developing secondary complications, to choose a therapy for an individual, to predict response to a therapy for an individual, to determine the efficacy of a therapy in an individual, and to determine the prognosis for an individual. In some embodiments, the invention is directed to methods of characterizing a plurality of pathways in single immune cells and/or a plurality of different discrete immune cell populations. Exemplary pathways include JAK/STAT MAPK and PI3K-Akt pathways. In some embodiments, the characterization of the pathways is correlated with diagnosing, prognosing or determining condition progression in an individual. In some embodiments, the characterization of the pathways is correlated with predicting response to treatment or choosing a treatment in an individual. In some embodiments, the characterization of the pathways is correlated with finding a new druggable target.

In some embodiments, the invention is directed to methods of determining a phenotypic profile of a population of cells by exposing the population of cells to a plurality of modulators in separate cultures, determining the presence or absence of an increase in activation level of an activatable element in the cell population from each of the separate culture and classifying the cell population based on the presence or absence of the increase in the activation of the activatable element from each of the separate culture. In some embodiments, the presence or absence of an increase in activation level of a plurality of activatable elements is determined. In some embodiments, each of the activatable elements belongs to a particular pathway and the activation level of the activatable elements is used to characterize each of the particular pathways. In some embodiments, a plurality of pathways are characterized by exposing a population of cells to a plurality of modulators in separate cultures, determining the presence or absence of an increase in activation levels of a plurality of activatable elements in the cell population from each of the separate culture, wherein the activatable elements are within the pathways being characterized and classifying the cell population based on the characterizations of said multiple pathways. In some embodiment, a plurality of activatable elements is analyzed in different discrete immune cell populations.

In some embodiments the invention provides methods of classification, diagnosis, prognosis, theranosis, and/or prediction of an outcome of an autoimmune disease in a subject, the method comprising: a) contacting a first cell from a first cell population from the subject with: (i) at least a first modulator or a fragment thereof, or (ii) a presence of no modulator; b) contacting a second cell from a second cell population from the individual with : (i) at least a second modulator or a fragment thereof, or (ii) a presence of no modulator; c) determining an activation level of at least one activatable element in the first cell and the second cell; and c) classifying, diagnosing, prognosing, theranosing, and/or predicting an outcome of the autoimmune disease in the subject based on the activation level of the at least one activatable element. In some embodiments, the methods further comprise creating a response panel for the subject comprising the determined activation levels of the activatable elements. In some embodiments, the first cell population and the second cell population are immune cells. In some embodiments, the methods further comprise contacting a third cell from a third cell population from the subject with: (i) at least a second modulator or a fragment thereof, or (ii) a presence of no modulator; and determining an activation level of at least one activatable element in the third cell. In some embodiments, the first and/or second modulator is a cytokine. In some embodiments, the first and/or second modulator is a STAT pathway modulator.

In some embodiments the invention provides methods of classification, diagnosis, prognosis, theranosis, and/or prediction of an outcome of an autoimmune disease in a subject, the method comprising: a) contacting a B cell from a B cell population from the subject with: (i) at least a first modulator or a fragment thereof, or (ii) a presence of no modulator; b) contacting a T cell from a T cell population from the individual with : (i) at least a second modulator or a fragment thereof, or (ii) a presence of no modulator; c) determining an activation level of at least one activatable element in the B cell and the T cell; and c) classifying, diagnosing, prognosing, theranosing, and/or predicting an outcome of the autoimmune disease in the subject based on the activation level of the at least one activatable element. In some embodiments, the B cell is contacted with a presence of no modulator. In some embodiments, at least one activatable element in the B cell type is selected from the group consisting of: CD69, Stat3, Lck, p-Lck, pZap70/Syk, pI3K, Stat5, and phospho-Stat3. In some embodiments, the T cell is a naive CD4 T cell. In some embodiments, at least one activatable element in the naive CD4 T cell is p-Stat1 and the naive CD4 T cell is contacted with IL6

In some embodiments, the methods further comprise contacting a CD8 T cell from a CD8 T cell population from the subject with: (i) at least a second modulator or a fragment thereof, or (ii) a presence of no modulator; and determining an activation level of at least one activatable element in the CD8 T cell. In some embodiments, the CD8 T cell is a memory /effector T cell. In some embodiments, at least one activatable element is p-Lck and the CD8 memory /effector T cell is contacted with a T cell receptor stimulation. In some embodiments, the T cell is a regulatory T cell. In some embodiments, the at least one activatable element is p-Stat5 and the regulatory T cell is contacted with IL-21.

In some embodiments the invention provides methods of classification, diagnosis, prognosis, theranosis, and/or prediction of an outcome of an autoimmune disease in a subject, the method comprising: a) contacting a CD4 T cell from a CD4 Tcell population from the subject with: (i) at least a first modulator or a fragment thereof, or (ii) a presence of no modulator; b) contacting a CD8 T cell from a CD8 T cell population from the individual with : (i) at least a second modulator or a fragment thereof, or (ii) a presence of no modulator; c) determining an activation level of at least one activatable element in the CD4 T cell and the CD8 T cell; and c) classifying, diagnosing, prognosing, theranosing, and/or predicting an outcome of the autoimmune disease in the subject based on the activation level of the at least one activatable element. In some embodiments, at least one activatable element in said CD4 T cell and/or CD8 T cell is selected from the group consisting of: p-p38, Lck, p-Lck, p-Stat5, and PLCγ2.

In some embodiments, the invention provides methods for categorizing disease activity in an autoimmune disease, comprising: (a) determining an activation level of at least one activatable element in one or more cells in a plurality of immune cell populations in response to: (i) at least a modulator or a fragment thereof, or (ii) a presence of no modulator; and (b) categorizing the disease activity, based on the activation level of at least one activatable element in the one or more cells. In some embodiments, the disease is rheumatoid arthritis, and the at least one activatable element is selected from the group consisting of: CD69, p-Stat5, p-Stat1, p-Lck, p-p38, Lck, p-Stat1, p-Stat3, p-p38, PLCγ2, and p-PLCγ2. In some embodiments, the combination of activatable elements, immune cell types and modulators are selected from the combination of activatable elements, immune cell types and modulators listed in Tables 1, 2, 3, and 4. In some embodiments, the disease is systemic lupus erythematosis and the plurality of immune cell populations is a T cell population and a B cell population.

In some embodiments, the activation level of p-Stat 3 is determined in a Naïve CD8 T cell population in response to IFNα, the activation of p-Stat6 is determined in a B cell population in response to a presence of no modulator, and the activation level of the activation of p-Stat6 is determined in a B cell population in response to IL-10.

In some embodiments, the activation level of p-Stat 3 is determined in a Naïve CD8 T cell population in response to IFNα, the activation of p-Stat1 is determined in a Naïve CD8 T cell population in response to a presence of no modulator, and the activation level of the activation of p-Stat1 is determined in a Naïve CD4 T cell population in response to IL-6.

In some embodiments, the invention provides methods of diagnosing, prognosing, determining progression, predicting a response to a treatment or choosing a treatment for an autoimmune disease in an individual, said methods comprising the steps of: (1) classifying one or more immune cells associated with rheumatoid arthritis or systemic lupus erythematosis in said individual by a method comprising: a) determining an activation level of at least three activatable elements in the presence of a modulator or no modulator; b) classifying said one or more immune cells based on said activation levels of said activatable elements; and (2) making a decision regarding a diagnosis, prognosis, progression, response to a treatment or a selection of treatment for rheumatoid arthritis or systemic lupus erythematosis in said individual based on said classification of said one or more immune cells. In some embodiments, at least one of the activatable elements is an activatable element from a STAT pathway. In some embodiments, at least one of the activatable elements is an activatable element from a cytokine pathway.

In some embodiments, the invention provides a method of categorizing disease activity, comprising a) determining an activation level of at least three activatable elements in immune cells b) classifying said one or more immune cells based on said activation levels of said activatable elements; and c) categorizing said disease activity, based on said classification of immune cells. In some embodiments, the disease is rheumatoid arthritis, and one or more of the at least three activatable elements is selected from the group consisting of: CD69, p-Stat5, p-Stat1, p-Lck, p-p38, Lck, p-Stat1, p-Stat3, p-p38, PLCγ2, p-PLCγ2. In some embodiments, the disease is systemic lupus erythematosis, and one or more of the at least three activatable elements are selected from the group consisting of: Stat 1, Stat3, Stat5, and Stat6, and the modulators are selected from the group consisting ofIL4, IL6, IL10, IL15, IFNγ, and IFNα.

In some embodiments, the invention provides methods of predicting treatment responsiveness of a subject with rheumatoid arthritis, comprising: a) contacting a B cell from a B cell population from the subject with: (i) at least a first modulator or a fragment thereof, or (ii) a presence of no modulator; b) contacting a CD8 T cell from a CD8 T cell population from the subject with : (i) at least a second modulator or a fragment thereof, or (ii) a presence of no modulator; c) determining an activation level of at least one activatable element in the B cell and the CD8 T cell; and d) predicting treatment responsiveness of the subject based on the activation level of the at least one activatable element. In some embodiments, the activation level of an activatable element selected from the group consisting of CD69, Stat3, Lck, p-Lck, pZap70/Syk, pI3K, Stat5, and phospho-Stat3 is determined in the B cell. In some embodiments, the activation level of an activatable element selected from the group consisting of Lck, and p-PLCγ2 is determined in the CD8 T cell. In some embodiments, the treatment is Orencia.

In some embodiments, the invention provides methods of predicting changes in disease activity in a subject with systemic lupus erythematosis, comprising: a)contacting a Naïve CD8 cell from a CD8 cell population from the subject with: (i) at least a first modulator or a fragment thereof, or (ii) a presence of no modulator; b) contacting a B cell from a B cell population from the subject with: (i) at least a first modulator or a fragment thereof, or (ii) a presence of no modulator; c)determining an activation level of at least one activatable element in the B cell and the Naïve CD8 T cell; and d) predicting changes in disease activity in the subject based on the activation level of the at least one activatable element. In some embodiments, the activation level of p-Stat3 is determined in the B cell in response to a modulator selected from the group consisting IL4, IL6, and IFNγ. In some embodiments, the activation level of p-Stat5 is determined in the B cell in response to IFNα. In some embodiments, the activation level of p-Stat6 is determined in the B cell in response to IL10, or a presence of no modulator. In some embodiments, the activation level of p-Stat1 is determined in the Naïve CD8 T cell in response to a modulator selected from the group consisting of IL-10, IFNα, and IL-6. In some embodiments, the activation level of p-Stat6 is determined in the Naïve CD8 T cell in response to a modulator selected from the group consisting of IL-15, and IFNα. In some embodiments, the activation level of p-Stat5 is determined in the Naïve CD8 T cell in response to IL-21.

In some embodiments, the activatable elements and/or nodes are selected from the activatable elements, modulators and cell types listed in Tables 1, 2, 3, or 4. In some embodiments, the combination of activatable element, modulator and cell type is selected from those listed in Table 1. In some embodiments, the combination of activatable element, modulator and cell type is selected from those listed in Table 2. In some embodiments, the treatment is Orencia, and the combination of activatable element, modulator and cell type is e selected from those listed in Table 3. In some embodiments, the treatment is prednisone, and the combination of activatable element, modulator and cell type is selected from those listed in Table 4.

In some embodiments, the invention provides methods for predicting response to a treatment for an autoimmune disease, wherein the positive predictive value (PPV) is higher than 60, 70, 80, 90, 95, or 99.9 %. In some embodiments, the invention provides methods for predicting response to a treatment for an autoimmune disease, wherein the PPV is equal or higher than 70%. In some embodiments, the invention provides methods for predicting response to a treatment for an autoimmune disease, wherein the PPV is equal or higher than 85%. In some embodiments, the invention provides methods for predicting response to a treatment for an autoimmune disease, wherein the PPV is equal or higher than 95%. In some embodiments, the invention provides methods for predicting response to a treatment for an autoimmune disease, wherein the negative predictive value (NPV) is higher than 60, 70, 80, 90, 95, or 99.9 %. In some embodiments, the invention provides methods for predicting response to a treatment for an autoimmune disease, wherein the NPV is higher than 70 %. In some embodiments, the invention provides methods for predicting response to a treatment for an autoimmune disease, wherein the NPV is higher than 85 %. In some embodiments, the invention provides methods for predicting response to a treatment for an autoimmune disease, wherein the NPV is higher than 95 %.

In some embodiments, the invention provides methods for diagnosing, prognosing, determining progression or predicting response for treatment of an autoimmune disease wherein the AUC value is higher than 0.5, 0.6, 07, 0.8 or 0.9. In some embodiments, the invention provides methods for diagnosing, prognosing, determining progression or predicting response for treatment an autoimmune disease wherein the AUC value is higher than 0.7. In some embodiments, the invention provides methods for diagnosing, prognosing, determining progression or predicting response for treatment of an autoimmune disease wherein the AUC value is higher than 0.8. In some embodiments, the invention provides methods for diagnosing, prognosing, determining progression or predicting response for treatment of an autoimmune disease wherein the AUC value is higher than 0.9.

In some embodiments, the invention is directed to methods for classifying a cell by determining the presence or absence of an increase in activation level of an activatable element in the cell, in combination with additional expression markers. In some embodiments, expression markers such as CD3 CD4, CD19, Foxp3, CD25, CD33, CD45RA, CD69, andphosphoproteins pStat1 (pY701), pStat3 (pY705), pStat5 (pY694), pLck (pY505), and pZap70 (pY319)/pSyk (pY352), phospho-PLCγ2, and others noted herein can also be used for stratifying responders and non-responders. The expression markers may be detected using many different techniques, for example using nodes from flow cytometry data (see the articles and patent applications referred to above). Other common techniques employ expression arrays (commercially available from Affymetrix, Santa Clara CA), taqman (commercially available from ABI, Foster City CA), SAGE (commercially available from Genzyme, Cambridge MA), sequencing techniques (see the commercial products from Helicos, 454, US Genomics, Pacific Bio, Ion Torrent, and Life Technologies) and other commonly know assays. See Golub et al., Science 286: 531-537 (1999). Expression markers are measured in unstimulated cells to know whether they have an impact on functional apoptosis. This provides implications for treatment and prognosis for the disease. Under this hypothesis, the amount of drug transporters correlates with the response of the patient and non-responders may have more levels of drug transporters (to move a drug out of a cell) as compared to responders.

In some embodiments, the invention is directed to methods of classifying a cell population by contacting the cell population with at least one modulator that affects signaling mediated by receptors selected from the group comprising of growth factors, mitogens and cytokines. In some embodiments, the invention is directed to methods of classifying a cell population by contacting the cell population with at least one modulator that affects signaling mediated by receptors selected from the group comprising SDF-1α, IFN-α, IFN-γ, IL-10, IL-6, IL-27, G-CSF, FLT-3L, IGF-1, M-CSF, SCF, PMA, and Thapsigargin; determining the activation states of a plurality of activatable elements in the cell comprising; and classifying the cell based on said activation states and expression levels. In some embodiments, the cell population is also exposed in a separate culture to at least one modulator that slows or stops the growth of cells and/or induces apoptosis of cells.

In some embodiments, the cell population is also exposed to at least one modulator that interferes with immune signaling. In some embodiments, the modulator that interferes with immune signaling is selected from the group consisting of Non-steroidal Anti-inflammatory Agents (NSAIDs), steroid medications, and immune disease-modifying agents.

NSAIDs include, but are not limited to, ibuprofen (Advil®, Motrin®, Nuprin®) and naproxen (Alleve®) and many others are available by prescription including meloxicam (Mobic®), etodolac (Lodine®), nabumetone (Relafen®), sulindac (Clinoril®), tolementin (Tolectin®), choline magnesium salicylate (Trilasate®), diclofenac (Cataflam®, Voltaren®, Arthrotec®), Diflusinal (Dolobid®), indomethicin (Indocin®), Ketoprofen (Orudis®, Oruvail®), Oxaprozin (Daypro®), and piroxicam (Feldene®). NSAIDs also includes drugs known as COX-2 inhibitors that are also effective in controlling inflammation. COX-2 inhibitors include, but are not limited to celecoxib, Celebrex®; etoricoxib, Arcoxia®; and lumiracoxib, Prexige®.

Steroid medications include, but are not limited to, prednisone, and bisphosphonates such as alendronate (Fosamax®), risedronate (Actonel®), and ibandronate (Boniva®).

Immune disease-modifying agents include, but are not limited to methotrexate (Rheumatrex®, Trexall®), hydroxychloroquine (Plaquenil ®), sulfasalazine (Azulfidine®), leflunomide (Arava®),

etanercept (Enbrel®), adalimumab (Humira ®), and infliximab (Remicade®), abatacept (Orencia®), rituximab (Rituxan®), anakinra (Kineret®), azathioprine (Imuran®), cyclophosphamide, and cyclosporine A (Neoral®, Sandimmune®).

Another embodiment of the invention further includes using the modulators IL-3, IL-4, GM-CSF, EPO, LPS, TNF-α, and CD40L. In some embodiments, the cell population is an immune cell population.

In some embodiments, cells other than the cells associated with a condition or a combination of cells are used, e.g., in assigning a risk group, predicting an increased risk of increased disease activity, predicting an increased risk of developing secondary complications, choosing a therapy for an individual, predicting response to a therapy for an individual, determining the efficacy of a therapy in an individual, and/or determining the prognosis for an individual. That is that cells other than cells associated with a condition are in fact reflective of the condition process.

In some embodiments, the invention provides methods to carry out multiparameter flow cytometry for monitoring phospho-protein responses to various factors in autoimmune disease at the single cell level. Phospho-protein members of signaling cascades and the kinases and phosphatases that interact with them are required to initiate and regulate proliferative signals in cells. Apart from the basal level of protein phosphorylation alone, the effect of potential drug molecules on these network pathways was studied to discern unique autoimmune disease network profiles, which correlate with the genetics and disease outcome. Single cell measurements of phospho-protein responses reveal shifts in the signaling potential of a phospho-protein network, enabling categorization of cell network phenotypes by multidimensional molecular profiles of signaling. See U.S. Patent No. 7,393,656. See also Irish et. al., Single cell profiling of potentiated phospho-protein networks in cancer cells. Cell. 2004, vol. 118, p. 1-20.

Flow cytometry is useful in a clinical setting, since relatively small sample sizes, as few as 10,000 cells, can produce a considerable amount of statistically tractable multidimensional signaling data and reveal key cell subsets that are responsible for a phenotype. See U.S. Patent Nos. 7,381,535 and 7,393,656. See also Krutzik et al, 2004).

Thus, in some embodiments, the invention provides methods of diagnosing, prognosing, determining progression, predicting a response to a treatment or choosing a treatment for an autoimmune disease in an individual, said methods comprising the steps of: In some embodiments the invention provides methods of classification, diagnosis, prognosis, theranosis, and/or prediction of an outcome of an autoimmune disease in a subject, the method comprising: a) contacting a first cell from a first cell population from the subject with: (i) at least a first modulator or a fragment thereof, or (ii) a presence of no modulator; b) contacting a second cell from a second cell population from the individual with : (i) at least a second modulator or a fragment thereof, or (ii) a presence of no modulator; c) determining using flow cytometry an activation level of at least one activatable element in the first cell and the second cell; and c) classifying, diagnosing, prognosing, theranosing, and/or predicting an outcome of the autoimmune disease in the subject based on the activation level of the at least one activatable element. In some embodiments, the first cell population and the second cell population are immune cells. In some embodiments, the methods further comprise contacting a third cell from a third cell population from the subject with: (i) at least a second modulator or a fragment thereof, or (ii) a presence of no modulator; and determining by flow cytometry an activation level of at least one activatable element in the third cell.

### Kits

In some embodiments, the invention provides kits for the classification, diagnosis, prognosis, theranosis, and/or prediction of an outcome of an autoimmune disease in a subject. In some embodiments, the invention provides kits for the classification, diagnosis, prognosis, theranosis, and/or prediction of an outcome of an autoimmune disease in a subject after administering a therapeutic agent to treat the autoimmune disease. In some embodiments, the kit comprises one or more modulators, inhibitors, specific binding elements for signaling molecules, and may additionally comprise one or more therapeutic agents. The kit may further comprise a software package for data analysis of the cellular state and its physiological status, which may include reference profiles for comparison with the test profile and comparisons to other analyses as referred to above. The kit may also include instructions for use for any of the above applications.

Kits provided by the invention may comprise one or more of the state-specific binding elements described herein, such as phospho-specific antibodies. A kit may also include other reagents that are useful in the invention, such as modulators, fixatives, containers, plates, buffers, therapeutic agents, instructions, and the like.

In some embodiments, the kit comprises one or more antibodies which recognize dynamic state changes, protein modification, phosphorylation, methylation, acetylation, ubiquitination, SUMOylation, or cleavage of activatable elements. Examples of activatable elements recognized by antibodies and other binding elements provided by kits of the present invention include, but are not limited to proteins, kinases, HER receptors, PDGF receptors, FLT3 receptor, Kit receptor, FGF receptors, Eph receptors, Trk receptors, IGF receptors, Insulin receptor, Met receptor, Ret, VEGF receptors, TIE1, TIE2, erythropoetin receptor, thromobopoetin receptor, CD114, CD116, FAK, Jak1, Jak2, Jak3, Tyk2, Src, Lyn, Fyn, Lck, Fgr, Yes, Csk, Abl, Btk, ZAP70, Syk, IRAKs, cRaf, ARaf, BRAF, Mos, Lim kinase, ILK, Tpl, ALK, TGFβ receptors, BMP receptors, MEKKs, ASK, MLKs, DLK, PAKs, Mek 1, Mek 2, MKK3/6, MKK4/7, ASK1,Cot, NIK, Bub, Myt 1, Weel, Casein kinases, PDK1, SGK1, SGK2, SGK3, Akt1, Akt2, Akt3, p90Rsks, p70S6Kinase,Prks, PKCs, PKAs, ROCK 1, ROCK 2, Auroras, CaMKs, MNKs, AMPKs, MELK, MARKs, Chk1, Chk2, LKB-1, MAPKAPKs, Pim1, Pim2, Pim3, IKKs, Cdks, Jnks, Erks, IKKs, GSK3α, GSK3β, Cdks, CLKs, PKR, PI3-Kinase class 1, class 2, class 3, mTor, SAPK/JNK1,2,3, p38s, PKR, DNA-PK, ATM, ATR, phosphatases, Receptor protein tyrosine phosphatases (RPTPs), LAR phosphatase, CD45, Non receptor tyrosine phosphatases (NPRTPs), SHPs, MAP kinase phosphatases (MKPs), Dual Specificity phosphatases (DUSPs), CDC25 phosphatases, Low molecular weight tyrosine phosphatase, Eyes absent (EYA) tyrosine phosphatases, Slingshot phosphatases (SSH), serine phosphatases, PP2A, PP2B, PP2C, PP1, PPS, inositol phosphatases, PTEN, SHIPs, myotubularins, lipid signaling, phosphoinositide kinases, phopsholipases, prostaglandin synthases, 5-lipoxygenase, sphingosine kinases, sphingomyelinases, adaptor/scaffold proteins, Shc, Grb2, BLNK, LAT, B cell adaptor for PI3-kinase (BCAP), SLAP, Dok, KSR, MyD88, Crk, CrkL, GAD, Nck, Grb2 associated binder (GAB), Fas associated death domain (FADD), TRADD, TRAF2, RIP, T-Cell leukemia family, cytokines, IL-2, IL-4, IL-8, IL-6, interferon γ, interferon α, cytokine regulators, suppressors of cytokine signaling (SOCs), ubiquitination enzymes, Cbl, SCF ubiquitination ligase complex, APC/C, adhesion molecules, integrins, Immunoglobulin-like adhesion molecules, selectins, cadherins, catenins, focal adhesion kinase, p130CAS, cytoskeletal/contractile proteins, fodrin, actin, paxillin, myosin, myosin binding proteins, tubulin, eg5/KSP, CENPs, heterotrimeric G proteins, β-adrenergic receptors, muscarinic receptors, adenylyl cyclase receptors, small molecular weight GTPases, H-Ras, K- Ras, N-Ras, Ran, Rac, Rho, Cdc42, Arfs, RABs, RHEB, guanine nucleotide exchange factors, Vav, Tiam, Sos, Dbl, PRK, TSC1,2, GTPase activating proteins, Ras-GAP, Arf-GAPs, Rho-GAPs, caspases, Caspase 2, Caspase 3, Caspase 6, Caspase 7, Caspase 8, Caspase 9, proteins involved in apoptosis, Bc1-2, Mcl-1, Bc1-XL, Bcl-w, Bcl-B, A1, Bax, Bak, Bok, Bik, Bad, Bid, Bim, Bmf, Hrk, Noxa, Puma, IAPs, XIAP, Smac, cell cycle regulators, Cdk4, Cdk 6, Cdk 2, Cdk1, Cdk 7, Cyclin D, Cyclin E, Cyclin A, Cyclin B, Rb, p16, p14Arf, p27KIP, p21CIP, molecular chaperones, Hsp90s, Hsp70, Hsp27, metabolic enzymes, Acetyl-CoAa Carboxylase, ATP citrate lyase, nitric oxide synthase, vesicular transport proteins, caveolins, endosomal sorting complex required for transport (ESCRT) proteins, vesicular protein sorting (Vsps), hydroxylases, prolyl-hydroxylases PHD-1, 2 and 3, asparagine hydroxylase FIH transferases, isomerases, Pin1 prolyl isomerase, topoisomerases, deacetylases, Histone deacetylases, sirtuins, acetylases, histone acetylases, CBP/P300 family, MYST family, ATF2, methylases, DNA methyl transferases, demethylases, Histone H3K4 demethylases, H3K27, JHDM2A, UTX, tumor suppressor genes, VHL, WT-1, p53, Hdm, PTEN, proteases, ubiquitin proteases, urokinase-type plasminogen activator (uPA) and uPA receptor (uPAR) system, cathepsins, metalloproteinases, esterases, hydrolases, separase, ion channels, potassium channels, sodium channels, molecular transporters, multi-drug resistance proteins, P-Gycoprotein, nucleoside transporters, transcription factors/ DNA binding proteins, Ets, Elk, SMADs, Rel-A (p65-NFKB), CREB, NFAT, ATF-2, AFT, Myc, Fos, Spl, Egr-1, T-bet, β-catenin, HIFs, FOXOs, E2Fs, SRFs, TCFs, Egr-1, β-FOXO STAT1, STAT 3, STAT 4, STAT 5, STAT 6, p53, WT-1, HMGA, regulators of translation, S6, pS6, 4EPB-1, eIF4E-binding protein, regulators of transcription, RNA polymerase, initiation factors, elongation factors.

Kits provided by the invention may comprise one or more of the modulators described herein. Non-limiting examples of modulators include IL-1, IL-2, IL-3, IL-4, IL-6, IL-7, IL-10, IL-12, IL-15, IL-21, IL-27, GM-CSF, G-CSF, IFNα, IFNγ, T cell receptor (TCR) cross-linking antibodies, B cell receptor (BCR) cross-linking antibodies SDF-1α, FLT-3L, IGF-1, M-CSF, SCF, PMA, Thapsigargin, H₂O₂, etoposide, AraC, daunorubicin, staurosporine, benzyloxycarbonyl-Val-Ala-Asp (OMe) fluoromethylketone (ZVAD), lenalidomide, EPO, azacitadine, decitabine, LPS, TNF-α, and CD40L. Examples of TCR crosslinking antibodies include, but are not limited to, anti-CD3 and anti CD28 antibodies. Examples of BCR crosslinking antibodies include, but are not limited to, anti-IgG, anti-IgM, anti-kappa, and anti-lambda antibodies.

Kits provided by the invention can comprise one or more labeling elements. Non-limiting examples of labeling elements include small molecule fluorophores, proteinaceous fluorophores, radioisotopes, enzymes, antibodies, chemiluminescent molecules, biotin, streptavidin, digoxigenin, chromogenic dyes, luminescent dyes, phosphorous dyes, luciferase, magnetic particles, beta-galactosidase, amino groups, carboxy groups, maleimide groups, oxo groups and thiol groups, quantum dots , chelated or caged lanthanides, isotope tags, radiodense tags, electron-dense tags, radioactive isotopes, paramagnetic particles, agarose particles, mass tags, e-tags, nanoparticles, and vesicle tags.

The state-specific binding element of the invention can be conjugated to a solid support and/or to detectable groups directly or indirectly. The reagents may also include ancillary agents such as buffering agents and stabilizing agents, e.g., polysaccharides and the like. The kit may further include, where necessary, other members of the signal-producing system of which system the detectable group is a member (e.g., enzyme substrates), agents for reducing background interference in a test, control reagents, apparatus for conducting a test, and the like. The kit may be packaged in any suitable manner, such as with all elements in a single container along with a sheet of printed instructions for carrying out the test.

In some embodiments, the kits of the invention enable the detection of activatable elements by sensitive cellular assay methods, such as IHC and flow cytometry, which are suitable for the clinical detection, classification, diagnosis, prognosis, theranosis, outcome prediction, and screening of cells and tissue from subjects having an autoimmune disease involving altered pathway signaling.

Such kits may additionally comprise one or more therapeutic agents. The kit may further comprise a software package for data analysis of the physiological status, which may include reference profiles for comparison with the test profile.

Such kits may also include information, such as scientific literature references, package insert materials, clinical trial results, and/or summaries of these and the like, which indicate or establish the activities and/or advantages of the composition, and/or which describe dosing, administration, side effects, drug interactions, or other information useful to the health care provider. Such information may be based on the results of various studies, for example, studies using experimental animals involving in vivo models and studies based on human clinical trials. Kits described herein can be provided, marketed and/or promoted to health providers, including physicians, nurses, pharmacists, formulary officials, and the like. Kits may also, in some embodiments, be marketed directly to the consumer.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

### Example 1: Monitoring disease activity in rheumatoid arthritis

A blood draw of 5mL to 10mL was collected from a subject. Within four hours, the blood was ficoll-prepared to isolate the leukocytes (usually 5 to 10 million cells) from the erythrocytes and other blood cells. The leukocytes were cryopreserved in 90% fetal calf serum (FCS), 10% dimethyl sulfoxide (DMSO). A subject's sample was later thawed at 37°C and washed twice with media (RPMI with 5% FCS). Alternatively, the cells can be used fresh without freezing. The cells were allowed to recover at 37°C for 1 hour. The cells were aliquoted at 0.5 to 1 million cells and exposed to modulator (i.e. stimulated) for 5 to 15 minutes at 37°C. Exposure to modulator can include the use of any of the described modulators, and were performed at saturating dosages (eg. 50ng/mL). Stimulation of B cell receptor (BCR) was performed by the addition of anti-IgG, anti-IgM, anti-kappa, and anti-lambda (BD Biosciences) at 10ug/mL for 15 minutes. T cell receptor (TCR) stimulation was performed by resting the cells on ice for 15 minutes and then adding 10ug/mL of mouse anti-CD3 and anti-CD28 (BD Biosciences) for 10 minutes. The cells were washed with cold PBS to remove free antibody and the cells were transferred to a prewarmed tube of media containing 10ug/mL of anti-mouse antibody (Cruz Biotechnology) where the TCR stimulation was performed for 5 to 15 minutes.

Following exposure to modulator, the cells were fixed and permeabilized with eBioscience Foxp3 Staining Buffer Set for 30 minutes, preserving a cells activation state. The cells were stained for the surface markers CD3 CD4, CD 19 (BD Biosciences), and the T regulatory transcription factor Foxp3 (eBioscience). The samples exposed to cytokines were permeabilized a second time with 400uL of ice-cold methanol for 1 hour at -20°C. Cells were then given a unique barcode according to the one or more modulators to which they were exposed. For example, using the two succinimidyl ester-conjugated fluorophores Pacific Orange and Alexa 750 (Invitrogen), more than 20 combinations can be used to label each of the different cytokine or TCR and BCR stimulation cell populations. For more information on barcoding, see Krutzik et al. (2006), Nature Methods 3: 361-368.

After washing, the cells were stained in 100uL staining volume with antibodies to detect the surface proteins CD25, CD33, CD45RA, and CD69 (BD Biosciences), in addition to antibodies that recognize the phospho-proteins pStat1 (pY701) (Cell Signaling), pStat3 (pY705), pStat5 (pY694), pLck (pY505), and pZap70 (pY319)/pSyk (pY352) (BD Biosciences). The fixed, permeabilized, and stained cells were then analyzed on a BD LSRII flow cytometer. Data analysis can be performed using FlowJo 8.8.6 (Tree Star). Each sample was gated on cells based on forward and side scatter. The monocytes were gated based on CD33 staining leaving a population of lymphocytes. The lymphocytes and monocytes were then gated according to their barcode which represents the one or more modulators to which the population was exposed. The lymphocytes were further divided into CD3+CD4+CD45RA+, CD3+CD4+CD45RA-, CD3+CD4-CD45RA+, CD3+CD4-CD45RA-, CD3+CD4+CD25hi Foxp3+ regulatory T cells, and CD19+ B cells. For each cell subset, the median fluorescent intensity (MFI) of the phospho-proteins was determined. Additionally, the fold change (or activation) of stimulated (i.e. exposed to modulator) to basal (i.e. not exposed to modulator) was calculated for each stimulation, phospho¬protein, cell subset combination, referred to as a signaling node. With the data expressed as values of MFI and fold change, the subjects were stratified using the corners classifier, as described above.

The above procedure was followed using samples collected from subjects with rheumatoid arthritis. Disease activity was accurately categorized using the two nodes [CD69 MFI, basal, CD19+ B cells] and [phospho-Stat5 MFI, IL21, regulatory T cells]. In this example, subjects with a high disease score lay in a space in the lower right of the 2D classifier, while subjects with low disease lay outside of this space. In a sample of 12 subjects with active RA, defined as having a Health Assessment Questionnaire (HAQ) score of 0.75 or greater, this node combination correctly stratified 11 subjects. In addition, only 1 of 31 subjects having low disease activity was captured using this node combination. This node combination stratifier, or classifier, was also effective as determined using a bootstrapping/bagging approach for internal cross-validation. The validation was performed by resampling (selecting a random sample with replacement from the original data set). As a result, some samples were included more than once whereas other samples were excluded. Bagging refers to fitting the corners classifier to the bootstrap resample and then using the classifier to test those samples excluded in the random sampling for the accuracy in predicting the disease activity of these samples. After 1000 bootstrap resamples, each subject had a list of predictions for disease activity and the majority vote was taken. Classifiers that maintained their predictive power after resampling are likely to be influenced by the disease influences on each variable rather than affected by an artifact.

The addition of one of either [phospho-Stat1 MFI, IL6, naive CD4+ T cells] or [phospho-Lck, 5 minute TCR stimulation, CD8+ memory/effector T cells] nodes to the above classifier improved the accuracy of determination of disease activity. Other useful classifiers demonstrating accuracy in determining disease activity, as determined by Disease Activity Score (DAS28-4), included the following node combinations: [phospho-p38 MFI, basal, CD4+ memory/effector T cells], [Lck MFI, total protein, CD8+ memory and effector T cells], [phospho-Stat5, IL15, CD4+ memory and effector T cells]; and [PLCγ2, basal, CD4+ T cells], [Lck MFI, total protein, CD8+ memory and effector T cells], [phospho-Stat5, IL15, CD4+ memory and effector T cells]. Additional nodes evaluated for prediction of DAS28 and HAQ scores appear in Table 1 and Table 2, respectively, and includes values for the area under the curve (AUC) for receiver-operator characteristic (ROC) curves, as described below. These nodes and classifiers can be applied to other subjects to assess disease activity, and/or classify an autoimmune disease.

**Table 1: Nodes and the number of times used in classifiers predictive of DAS28**

| Nodes in DAS Classifiers (83 classifiers) | # of Classifiers | Combined AUC | Average AUC |
|---|---|---|---|
| IL15, CD4+CD45RA-, pStat5 | 26 | 21.1 | 0.81 |
| Unstim, CD4-CD45RA+, pPI3K | 23 | 19.23 | 0.84 |
| total, CD4-CD45RA-, tLck | 17 | 14.03 | 0.83 |
| total, CD4-CD45RA+, tLck | 18 | 13.77 | 0.77 |
| Unstim, CD4+CD45RA-, pp38 | 15 | 11.38 | 0.76 |
| TCR 5', CD4+CD45RA+, pPLCgII | 14 | 10.94 | 0.78 |
| total, CD19, tStat3 | 11 | 8.77 | 0.8 |
| IL2 0.2ng, Tregs, pStat5 | 7 | 5.82 | 0.83 |
| IL27, CD4-CD45RA+, pStat1 | 6 | 4.83 | 0.81 |
| TCR 15', CD4-CD45RA+, pZap70/pSyk | 5 | 3.7 | 0.74 |
| IL2, CD4+CD45RA-, pStat5 | 4 | 3.09 | 0.77 |
| IL21, Tregs, pStat5 | 3 | 2.53 | 0.84 |
| TCR 5', CD4+CD45RA+, pLck | 3 | 2.41 | 0.8 |
| TCR 5', CD4+CD45RA+, pPI3K | 3 | 2.38 | 0.79 |
| IL15, CD4-CD45RA-, pStat5 | 3 | 2.26 | 0.75 |
| IFNa, CD4-CD45RA-, pStat1 | 3 | 2.23 | 0.74 |
| IL21, CD4-CD45RA+, pStat5 | 3 | 2.18 | 0.73 |
| total, CD4+CD45RA-, tLck | 3 | 2.17 | 0.72 |
| IFNa, CD4+CD45RA-, pStat3 | 2 | 1.66 | 0.83 |
| TCR 5', CD4+CD45RA-, pPLCgII | 2 | 1.65 | 0.83 |
| total, CD19, tLck | 2 | 1.62 | 0.81 |
| IFNa, CD19+, pStat1 | 2 | 1.54 | 0.77 |
| IL27, CD4-CD45RA+, pStat3 | 2 | 1.54 | 0.77 |
| IL15, Tregs, pStat5 | 2 | 1.5 | 0.75 |
| TCR 5', CD4+CD45RA-, pLck | 2 | 1.43 | 0.71 |
| IL10, CD4-CD45RA-, pStat3 | 2 | 1.3 | 0.65 |
| IL10, CD4+CD45RA-, pStat3 | 1 | 0.92 | 0.92 |
| IFNa, CD4-CD45RA-, pStat5 | 1 | 0.9 | 0.9 |
| IFNa, CD4-CD45RA-, pStat3 | 1 | 0.89 | 0.89 |
| IL7, CD4-CD45RA+, pStat5 | 1 | 0.88 | 0.88 |
| TCR 5', CD4+CD45RA-, pp38 | 1 | 0.88 | 0.88 |
| Unstim, CD19+, pZap70/pSyk | 1 | 0.88 | 0.88 |
| IL7, Tregs, pStat5 | 1 | 0.87 | 0.87 |
| IL21, CD4+CD45RA-, pStat3 | 1 | 0.87 | 0.87 |
| total, CD4-CD45RA-, CD69 | 1 | 0.85 | 0.85 |
| Unstim, CD19+, pP13K | 1 | 0.84 | 0.84 |
| IL6, Monocytes, pStat3 | 1 | 0.84 | 0.84 |
| Unstim, CD19+, pLck | 1 | 0.84 | 0.84 |
| Unstim, CD4+CD45RA+, pP13K | 1 | 0.83 | 0.83 |
| TCR 5', CD4+CD45RA+, pp38 | 1 | 0.83 | 0.83 |
| IL6, CD4+CD45RA-, pStat3 | 1 | 0.83 | 0.83 |
| Unstim, Monocytes, pStat3 | 1 | 0.83 | 0.83 |
| IL27, Monocytes, pStat3 | 1 | 0.83 | 0.83 |
| IL21, CD4+CD45RA+, pStat5 | 1 | 0.82 | 0.82 |
| total, CD4+CD45RA-, CD69 | 1 | 0.78 | 0.78 |
| TCR 15', CD4+CD45RA+, pZap70/pSyk | 1 | 0.77 | 0.77 |
| TCR 5', Tregs, pPI3K | 1 | 0.76 | 0.76 |
| total, CD4-CD45RA+, CD69 | 1 | 0.75 | 0.75 |
| TCR 5', CD4+CD45RA-, pPI3K | 1 | 0.74 | 0.74 |
| IL21, CD4+CD45RA+, pStat3 | 1 | 0.72 | 0.72 |

**Table 2: Nodes and the number of times used in classifiers predictive of HAQ score**

| Nodes in HAQ Classifiers (72 classifiers) | # of Classifiers | Combined AUC | Average AUC |
|---|---|---|---|
| IL21, Tregs, pStat5 | 24 | 20.92 | 0.87 |
| total, CD19, CD69 | 21 | 18.33 | 0.87 |
| Unstim, Tregs, pZap70/pSyk | 10 | 8.6 | 0.86 |
| IL27, CD4+CD45RA-, pStat1 | 10 | 8.51 | 0.85 |
| IL21, CD4+CD45RA-, pStat3 | 9 | 8.29 | 0.92 |
| IL21, CD4+CD45RA+, pStat5 | 6 | 5.11 | 0.85 |
| total, CD4-CD45RA-, CD69 | 6 | 5.07 | 0.84 |
| IL6, CD4+CD45RA+, pStat1 | 6 | 4.99 | 0.83 |
| IL21, CD4-CD45RA+, pStat5 | 5 | 4.61 | 0.92 |
| IL27, CD4+CD45RA+, pStat1 | 5 | 4.12 | 0.82 |
| TCR 5', CD4-CD45RA+, pLck | 4 | 3.61 | 0.9 |
| IL2 0.2ng, Tregs, pStat5 | 4 | 3.45 | 0.86 |
| IL27, CD4-CD45RA-, pStat1 | 4 | 3.12 | 0.78 |
| TCR 5', CD4+CD45RA+, pLck | 3 | 2.63 | 0.88 |
| IL2-GMCSF, CD4-CD45RA+, pStat5 | 3 | 2.55 | 0.85 |
| IL27, Tregs, pStat1 | 3 | 2.48 | 0.83 |
| IL10, CD4+CD45RA+, pStat3 | 3 | 2.45 | 0.82 |
| total, CD19, tLck | 3 | 2.45 | 0.82 |
| IL27, CD4+CD45RA+, pStat3 | 3 | 2.44 | 0.81 |
| IL21, CD4+CD45RA-, pStat5 | 2 | 1.84 | 0.92 |
| TCR 5', Tregs, pPLCgII | 2 | 1.72 | 0.86 |
| TCR 5', Tregs, pZap70/Syk | 2 | 1.69 | 0.84 |
| BCR, CD19+, pZap70/Syk | 2 | 1.69 | 0.84 |
| TCR 5', CD4+CD45RA+, pZap70/pSyk | 2 | 1.67 | 0.84 |
| TCR 15', Tregs, pZap70/Syk | 2 | 1.65 | 0.83 |
| TCR 5', Treqs, pLck | 2 | 1.65 | 0.82 |
| IFNa, CD4+CD45RA+, pStat5 | 2 | 1.59 | 0.79 |
| TCR 5', CD4-CD45RA-, pp38 | 2 | 1.48 | 0.74 |
| Unstim, CD4+CD45RA-, pZap70/pSyk | 1 | 0.93 | 0.93 |
| IFNa, CD4-CD45RA+, pStat5 | 1 | 0.93 | 0.93 |
| TCR 5', CD4-CD45RA+, pp38 | 1 | 0.93 | 0.93 |
| TCR 5', CD4-CD45RA-, pLck | 1 | 0.9 | 0.9 |
| Unstim, CD4-CD45RA+, pp38 | 1 | 0.9 | 0.9 |
| IL6, Tregs, pStat1 | 1 | 0.9 | 0.9 |
| Unstim, Tregs, pPLCgII | 1 | 0.9 | 0.9 |
| TCR 5', CD4+CD45RA+, pPLCgII | 1 | 0.9 | 0.9 |
| GM-CSF, Monocytes, pStat5 | 1 | 0.89 | 0.89 |
| IL7, CD4+CD45RA+, pStat5 | 1 | 0.89 | 0.89 |
| TCR 5', CD4+CD45RA-, pPLCgII | 1 | 0.89 | 0.89 |
| IFNa, Tregs, pStat5 | 1 | 0.87 | 0.87 |
| total, CD4+CD45RA-, CD69 | 1 | 0.87 | 0.87 |
| IL2, CD4+CD45RA+, pStat5 | 1 | 0.87 | 0.87 |
| TCR 5', CD4+CD45RA+, pP13K | 1 | 0.86 | 0.86 |
| IFNa, CD4+CD45RA-, pStat5 | 1 | 0.85 | 0.85 |
| IFNa, CD19+, pStat3 | 1 | 0.84 | 0.84 |
| total, CD4+CD45RA+, CD69 | 1 | 0.84 | 0.84 |
| TCR 5', CD4-CD45RA+, pP13K | 1 | 0.83 | 0.83 |
| Unstim, CD4-CD45RA-, pStat1 | 1 | 0.82 | 0.82 |
| Unstim, CD4+CD45RA-, pstat1 | 1 | 0.81 | 0.81 |
| IFNa, CD19+, pStat1 | 1 | 0.8 | 0.8 |
| Unstim, Tregs, pLck | 1 | 0.8 | 0.8 |
| TCR 5', CD4+CD45RA-, pZap70/pSyk | 1 | 0.79 | 0.79 |
| BCR, CD19+, pLck | 1 | 0.78 | 0.78 |
| Unstim, Tregs, pStat5 | 1 | 0.78 | 0.78 |
| IFNa, CD4+CD45RA+, pStat3 | 1 | 0.75 | 0.75 |
| TCR 5', CD4+CD45RA-, pPI3K | 1 | 0.72 | 0.72 |
| IL15, CD4-CD45RA+, pStat5 | 1 | 0.72 | 0.72 |
| IL10, CD4-CD45RA-, pStat3 | 1 | 0.7 | 0.7 |
| IL7, Tregs, pStat5 | 1 | 0.66 | 0.66 |

### Example 2: Classifying subjects with rheumatoid arthritis by therapeutic regimen

The invention can be used as a tool by drug development companies to better understand the molecular mechanisms, or mode of action, of a specific drug. In this example, the invention was used to investigate the drugs prednisone and Orencia (Abatacept). Samples were collected and processed similarly as in Example 1. A study of 39 female RA patients, roughly half taking Orencia, demonstrated that specific cellular features were enriched in the treated group. The classifier Orencia-1 was defined by the combination of nodes [phospho-Stat3 MFI, basal, B cells], [Lck MFI, total protein, CD8+ naive T cells], [phospho-PLCy2 MFI, basal, CD8+ T cells]. The model correctly predicted 20 of 21 treated subjects with only one false positive. A bagging/bootstrapping cross-validation confirmed the predictive power of this classifier. In effect, the model shows that Orencia predisposes patients to having lower basal activated PLCy2 and Stat3 and higher protein levels of Lck in specific cell types.

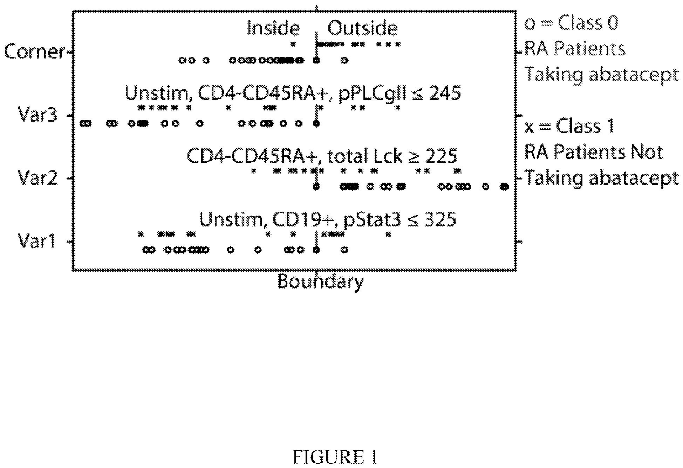

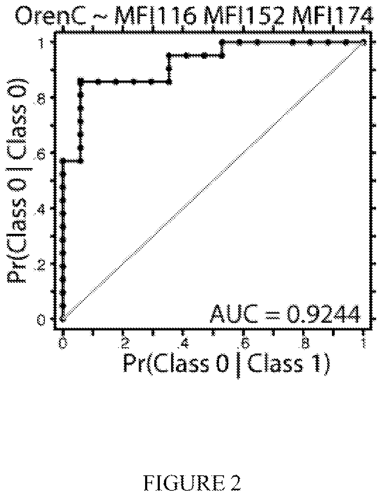

Over the course of development of the invention many models formed from the signaling data were evaluated for their predictive power. Taking the model from Figure 1, one can plot the true positive rate on the y-axis and the false positive rate on the x-axis using a function known as a receiver-operator characteristic (ROC) curve (Figure 2). As mentioned, cross-validation was performed by randomly sampling the data such that not all samples are sampled, and then predicting the class of excluded samples based on the model. After 1000 resamples, the majority prediction (eg. p1 > 1/2, where p1 is the fraction of Class 1 assertions in that subject's list) for each subject was the output prediction. The ROC curve works by stepping through the possible values to base a prediction (eg. p1 > 0.00, p1 > 0.12, p1 > 1.00). By setting a low threshold the true positive rate can be 1.00 but the false positive rate can be unacceptably high. The ROC curve provides a way to visualize this threshold over a range of values and identifying optimum performance for a model. This analysis is useful in identifying optimal models from subpar models.

Figure 2 demonstrates that the corners classifier developed from patients taking Orencia (Figure 1) is a model of high predictive power, as suggested by the area under the curve (AUC). A model based on randomness would have an appearance of a diagonal line (shown in Figure 2) with an AUC of 0.5. The Orencia-1 classifier model has an AUC of 0.9244 indicative of a successful model. An additional classifier for Orencia usage that performed well under internal cross validation with an AUC of 0.8627 includes the nodes [phospho-Lck MFI, TCR stimulated, CD8+ T cells], [Lck MFI, total protein, CD8+ T cells], [log fold phospho Stat3, IFNα, regulatory T cells]. Additional nodes evaluated for the classification of subjects treated with Orencia and prednisone appear in Table 3 and Table 4, respectively. These methods can be applied to subjects receiving other medications to identify possible modes of action and additional drug targets.

**Table 3: Nodes and the number of times used in classifiers predictive of Orencia usage in female donors**

| Nodes in Orencia Female (31 classifiers) | # of Classifiers | Combined AUC | Average AUC |
|---|---|---|---|
| total, CD4-CD45RA+, tLck | 20 | 17.25 | 0.86 |
| IFNa, CD4+CD45RA-, pStat3 | 14 | 12.18 | 0.87 |
| TCR 5', CD4-CD45RA+, pLck | 14 | 12.08 | 0.86 |
| IL21, CD4-CD45RA+, pStat3 | 5 | 4.5 | 0.9 |
| Unstim, CD4-CD45RA+, pPLCgII | 5 | 4.47 | 0.89 |
| IL15, CD4-CD45RA-, pStat5 | 4 | 3.54 | 0.88 |
| IL21, CD19+, pStat3 | 4 | 3.47 | 0.87 |
| Unstim, CD4+CD45RA-, pPLCgII | 2 | 1.83 | 0.92 |
| Unstim,CD19+, pStat3 | 2 | 1.79 | 0.89 |
| IFNa, CD4-CD45RA+, pStat3 | 2 | 1.73 | 0.86 |
| Unstim, CD4-CD45RA-, pStat3 | 2 | 1.71 | 0.85 |
| TCR 5', CD4+CD45RA+, pPI3K | 2 | 1.71 | 0.85 |
| IFNa, CD19+, pStat3 | 2 | 1.69 | 0.84 |
| IL27, Monocytes, pStat3 | 1 | 0.9 | 0.9 |
| IL6, Monocytes, pStat3 | 1 | 0.9 | 0.9 |
| IFNa, CD4+CD45RA-, pStat1 | 1 | 0.89 | 0.89 |
| IFNa, CD4-CD45RA+, pStat1 | 1 | 0.89 | 0.89 |
| BCR, CD19+, pZap70/pSyk | 1 | 0.88 | 0.88 |
| TCR 5', CD4+CD45RA+, pLck | 1 | 0.82 | 0.82 |

**Table 4: Nodes and the number of times used in classifiers predictive of prednisone usage in female donors**

| Nodes in prednisone female (28 classifiers) | # of Classifiers | Combined AUC | Average AUC |
|---|---|---|---|
| IL10, CD4-CD45RA-, pStat3 | 18 | 14.47 | 0.8 |
| IL10, CD4+CD45RA+, pStat3 | 8 | 6.6 | 0.82 |
| TCR 15', CD4+CD45RA-, pLck | 7 | 6.07 | 0.87 |
| IFNa, Monocytes, pStat5 | 6 | 4.98 | 0.83 |
| IFNa, Treqs, pStat3 | 5 | 3.84 | 0.77 |
| IL10, Monocytes, pStat3 | 4 | 3.19 | 0.8 |
| IL2, CD4+CD45RA-, pStat5 | 2 | 1.76 | 0.88 |
| Unstim, CD4-CD45RA-, pPLCgII | 2 | 1.63 | 0.81 |
| IFNa, CD4+CD45RA+, pStat5 | 2 | 1.63 | 0.81 |
| IFNa, CD19+, pStat5 | 2 | 1.56 | 0.78 |
| BCR, CD19+, pZap70/pSyk | 1 | 0.9 | 0.9 |
| IL21, CD4-CD45RA+, pStat5 | 1 | 0.89 | 0.89 |
| IFNa, CD19+, pStat3 | 1 | 0.87 | 0.87 |
| BCR, CD19+, pLck | 1 | 0.86 | 0.86 |
| IL15, Treqs, pStat5 | 1 | 0.86 | 0.86 |
| Unstim, CD4-CD45RA+, pZap70/pSyk | 1 | 0.85 | 0.85 |
| IL10, CD4-CD45RA+, pStat3 | 1 | 0.85 | 0.85 |
| TCR 15', CD4-CD45RA+, pLck | 1 | 0.83 | 0.83 |
| Unstim, CD4-CD45RA+, pLck | 1 | 0.82 | 0.82 |
| IL10, Treqs, pStat3 | 1 | 0.79 | 0.79 |
| Unstim, Treqs, pLck | 1 | 0.78 | 0.78 |
| IL6, CD4+CD45RA+, pStat1 | 1 | 0.74 | 0.74 |

### Example 3: Predicting changes in disease activity

Disease activity in systemic lupus erythematosis (SLE) is often expressed in terms of the Systemic Lupus Erythematosus Disease Activity Index (SLEDAI) and/or the Physician Global Assessment (PGA). Using these measures, and samples collected from subjects at multiple times, it was demonstrated that methods of the invention can identify nodes that objectively predict levels of increased disease activity (e.g. flares) at least 90 days in advance. Blood samples were collected from patients at two times, separated by 90 days, and disease activity was scored at each time point. Samples were processed similarly as in Example 1. Modulation, activation level determination, and analysis were performed as described herein. For some of the signaling nodes, signaling responses to certain stimulations in certain cell types was higher in patients that exhibited an increase in disease activity, and included [Stat3, IL4, B cells], [Stat1, IL10, CD4-CD45RA+ cells], [Stat3, IL6, B cells], [Stat3, IL4, B cells], [Stat1, IFNα, CD4-CD45RA+ cells], [Stat1, IL6, CD4-CD45RA+ cells], [Stat6, IL15, CD4-CD45RA+ cells] (SLEDAI > 3 at day 90), and [Stat3, IFNγ, B cells] (SLEDAI > 3 at day 90). For other signaling nodes, signaling responses to certain stiumlations in certain cell types were lower in subjects that exhibited an increase in disease activity, and included [Stat5, IFNα, CD4-CD45RA+ cells], [Stat5, IFNα, B cells], [Stat5, IL21, CD4-CD45RA+ cells], [Stat1, IFNα, CD4-CD45RA- cells], [Stat6, IFNα, CD4-CD45RA+ cells], [Stat6, IL6, CD4+CD45RA+ cells] (SLEDAI > 3 at day 90), [Stat1, IL10, monocytes], and [Stat5, IL10, monocytes] (SLEDAI > 3 at day 90). In addition, the method identified nodes correlated with the absence of measurable disease at both time points, including increased signaling response in the node [Stat3, IFNα, CD4-CD4RA+], and reduced signaling responses in the two nodes [Stat6, IL10, B cells] and [Stat6, basal, B cells].

Pairwise use of these signaling nodes increased the strength of their correlation to the desired clinical correlate, for example, greatly increasing the ability to predict flare. Combining the following three nodes with the indicated MFI thresholds defines a region in three dimensional signaling space that encompasses all subjects who flared by day 90, but none of the subjects who did not flare by day 90: [Stat1, IL6, CD4+CD45RA+ cells] (MFI≥ 1207), [Stat1, basal, CD4-CD45RA+ cells] (MFI ≥ 83.9), and [Stat6, IFNα, CD4-CD45RA+ cells] (MFI ≤ 108). Likewise, a two variable corner classifier captured 11 out of 12 subjects who had a SLEDAI score of zero at the time of first blood draw and no increase in SLEDAI within 90 days, while only capturing one of 29 patients who had a SLEDAI score greater than zero for at least one of the two times: [Stat6, basal, B cells] (MFI ≤ 83.1) and [Stat3, IFNα, CD4-CD45RA+ cells] (MFI ≥ 337). These classifiers can be applied to data collected from other subjects to provide a basis for the prediction of disease stability or flare at least 90 days in advance.

## Claims

1. A method for classifying rheumatoid arthritis in a subject, said method comprising:
contacting a first leukocyte from the subject, wherein the first leukocyte is a regulatory T cell, with at least one modulator, wherein the modulator is IL-21;
contacting a second leukocyte from the subject, wherein the second leukocyte is a CD19+ B cell, with a presence of no modulator;
determining the activation level of at least one activatable element in the first leukocyte and at least one activatable element in the second leukocyte following the contacting, wherein the at least one activatable element in the first leukocyte is phospho-Stat5 and the at least one activatable element in the second leukocyte is CD69; and
classifying, a rheumatoid arthritis in the subject based on the determination of the activation level of CD69 in the CD19+ B cell and the activation level of phospho-Stat5 in the regulatory T cell.

2. The method according to claim 1, wherein the determining the activation level of the at least one activatable element comprises detecting the binding of a binding element selected from an antibody, a recombinant protein, and a fluorescent dye to the at least one activatable element.

3. The method according to claim 2, wherein the binding element detects a particular activation state of a particular activatable element.

4. The method according to claim 1, further comprising detecting in the first leukocyte and second leukocyte the presence or absence of a cellular marker selected from a cell surface marker and an intracellular marker.

5. The method according to claim 1, wherein determining the activation level of the at least one activatable element comprises the use of flow cytometry, immunofluorescence, confocal microscopy, immunohistochemistry, immunoelectronmicroscopy, nucleic acid amplification, gene array, protein array, mass spectrometry, patch clamp, 2-dimensional gel electrophoresis, differential display gel electrophoresis, microsphere-based multiplex protein assays, ELISA, and label-free cellular assays.

6. The method according to claim 1, further comprising:
contacting a third leukocyte from the subject to: i) at least a third modulator or (ii) a presence of no modulator;
determining an activation level of at least one activatable element in the third leukocyte; and
classifying, diagnosing, prognosing, theranosing and/or predicting an outcome of an rheumatoid arthritis in the subject based on the activation level of the at least one activatable element in the first leukocyte, the activation level of the at least one activatable element in the second leukocyte, and the activation level of the at least one activatable element in the third leukocyte.

## Patentansprüche

1. Verfahren zum Klassifizieren rheumatoider Arthritis bei einem Individuum, wobei das Verfahren Folgendes umfasst:
Kontaktieren eines ersten Leukozyten des Individuums, worin der erste Leukozyt eine regulatorische T-Zelle ist, mit zumindest einem Modulator, worin der Modulator IL-21 ist;
Kontaktieren eines zweiten Leukozyten des Individuums, worin der zweite Leukozyt eine CD19+-B-Zelle ist, mit einer Gegenwart keines Modulators;
Bestimmen des Aktivierungsniveaus von zumindest einem aktivierbaren Element im ersten Leukozyten und zumindest einem aktivierbaren Element im zweiten Leukozyten nach dem Kontaktieren, worin das zumindest eine aktivierbare Element im ersten Leukozyten Phospho-Stat5 ist und das zumindest eine aktivierbare Element im zweiten Leukozyten CD69 ist; und
Klassifizieren einer rheumatoiden Arthritis bei dem Individuum auf Basis der Bestimmung des Aktivierungsniveaus von CD69 in der CD19+-B-Zelle und des Aktivierungsniveaus von Phospho-Stat5 in der regulatorischen T-Zelle.

2. Verfahren nach Anspruch 1, worin die Bestimmung des Aktivierungsniveaus des zumindest einen aktivierbaren Elements das Detektieren einer Bindung eines aus einem Antikörper, einem rekombinanten Protein und einem Fluoreszenzfarbstoff ausgewählten Bindungselements an das zumindest eine aktivierbare Element umfasst.

3. Verfahren nach Anspruch 2, worin das Bindungselement einen speziellen Aktivierungszustand eines speziellen aktivierbaren Elements detektiert.

4. Verfahren nach Anspruch 1, das weiters das Detektieren einer Anwesenheit oder Abwesenheit eines aus einem Zelloberflächenmarker und einem intrazellulären Marker ausgewählten zellulären Markers in dem ersten Leukozyten und dem zweiten Leukozyten umfasst.

5. Verfahren nach Anspruch 1, worin die Bestimmung des Aktivierungsniveaus des zumindest einen aktivierbaren Elements die Verwendung von Durchflusszytometrie, Immunfluoreszenz, konfokaler Mikroskopie, Immunhistochemie, Immunelektronenmikroskopie, Nucleinsäure-Amplifikation, Gen-Array, Protein-Array, Massenspektrometrie, Patch-Clamp-Technik, 2-dimensionaler Gelelektrophorese, Differential-Display-Gelelektrophorese, mikrokügelchenbasierten Multiplex-Proteintests, ELISA und markierungsfreien zellbasierten Tests umfasst.

6. Verfahren nach Anspruch 1, das weiters das Folgende umfasst:
Kontaktieren eines dritten Leukozyten des Individuums mit: i) zumindest einem dritten Modulator oder (ii) einer Gegenwart keines Modulators;
Bestimmen eines Aktivierungsniveaus von zumindest einem aktivierbaren Element in dem dritten Leukozyten; und
Klassifizieren, Diagnostizieren, Prognostizieren, Theranosieren und/oder Vorhersagen eines Ausgangs einer rheumatoiden Arthritis bei dem Individuum auf Basis des Aktivierungsniveaus des zumindest einen aktivierbaren Elements im ersten Leukozyten, des Aktivierungsniveaus des zumindest einen aktivierbaren Elements im zweiten Leukozyten und des Aktivierungsniveaus des zumindest einen aktivierbaren Elements im dritten Leukozyten.

## Revendications

1. Procédé pour classifier la polyarthrite rhumatoïde chez un sujet, ledit procédé comprenant :
la mise en contact d'un premier leucocyte du sujet, où le premier leucocyte est une cellule T régulatrice, avec au moins un modulateur, où le modulateur est l'IL-21 ;
la mise en contact d'un deuxième leucocyte du sujet, où le deuxième leucocyte est une cellule B CD19+, sans présence de modulateur ;
la détermination du niveau d'activation d'au moins un élément activable dans le premier leucocyte et d'au moins un élément activable dans le deuxième leucocyte suite à la mise en contact, où le au moins un élément activable dans le premier leucocyte est la phospho-Stat5 et le au moins un élément activable dans le deuxième leucocyte est CD69 ; et
la classification de la polyarthrite rhumatoïde chez le sujet en se basant sur la détermination du niveau d'activation de CD69 dans la cellule B CD19+ et du niveau d'activation de la phospho-Stat5 dans la cellule T régulatrice.

2. Procédé selon la revendication 1, dans lequel la détermination du niveau d'activation du au moins un élément activable comprend la détection de la liaison d'un élément de liaison sélectionné parmi un anticorps, une protéine recombinante, et un colorant fluorescent, à le au moins un élément activable.

3. Procédé selon la revendication 2, dans lequel l'élément de liaison détecte un état d'activation particulier d'un élément activable particulier.

4. Procédé selon la revendication 1, comprenant en outre la détection, dans le premier leucocyte et le deuxième leucocyte, de la présence ou l'absence d'un marqueur cellulaire sélectionné parmi un marqueur de surface cellulaire et un marqueur intracellulaire.

5. Procédé selon la revendication 1, dans lequel la détermination du niveau d'activation du au moins un élément activable comprend l'utilisation de la cytométrie en flux, l'immunofluorescence, la microscopie confocale, l'immunohistochimie, la microscopie immunoélectronique, une amplification d'acide nucléique, un réseau de gènes, un réseau de protéines, la spectrométrie de masse, un patch-clamp, une électrophorèse bidimensionnelle sur gel, une électrophorèse différentielle sur gel, des dosages des protéines multiplexes basés sur des microsphères, et des dosages cellulaires sans marqueur.

6. Procédé selon la revendication 1, comprenant en outre :
la mise en contact d'un troisième leucocyte du sujet avec : i) au moins un troisième modulateur ou (ii) sans présence de modulateur ;
la détermination d'un niveau d'activation d'au moins un élément activable dans le troisième leucocyte ; et
la classification, le diagnostic, le pronostic, le théranostic et/ou la prédiction de l'issue de la polyarthrite rhumatoïde chez le sujet en se basant sur le niveau d'activation du au moins un élément activable dans le premier leucocyte, le niveau d'activation du au moins un élément activable dans le deuxième leucocyte, et le niveau d'activation du au moins un élément activable dans le troisième leucocyte.
